# EUROPEAN PATENT SPECIFICATION

(11) **EP 1 686 999 B1**
(45) Date of publication and mention of the grant of the patent: **01.07.2009**
(21) Application number: 04798505.6
(22) Date of filing: 15.11.2004
(51) Int. Cl.: A61K 31/506, C07D 403/12, C07D 409/14, C07D 495/04, C07D 487/04, C07D 471/04, C07D 401/14, C07D 417/04, C07D 403/14, A61P 35/00

(54) **PYRAZOLE DERIVATIVES AS INHIBITORS OF RECEPTOR TYROSINE KINASES**
PYRAZOL-DERIVATE ALS HEMMER DER REZEPTOR-TYROSIN-KINASEN
DERIVES DE PYRAZOLE EN TANT QU'INHIBITEURS DE RECEPTEUR TYROSINE KINASES

(30) Priority: 17.11.2003 US 520581 P; 25.03.2004 US 556213 P
(43) Date of publication of application: 09.08.2006
(73) Proprietor: AstraZeneca AB, 151 85 Södertälje (SE)
(72) Inventor: BLOCK, Michael Howard, AstraZeneca R & D Boston, Waltham, Massachusetts 02451 (US); HAN, Yongxin, Array BioPharma Inc, Boulder, Colorado 80301 (US); JOSEY, John Anthony, Array BioPharma Inc, Boulder, Colorado 80301 (US); LEE, John W, AstraZeneca R & D Boston, Waltham, Massachusetts 02451 (US); SCOTT, David, AstraZeneca R & D Boston, Waltham, Massachusetts 02451 (US); WANG, Bin, Array BioPharma Inc, Boulder, Colorado 80301 (US); WANG, Haixia, AstraZeneca R & D Boston, Waltham, Massachusetts 02451 (US); WANG, Tao, AstraZeneca R & D Boston, Waltham, Massachusetts 02451 (US); YU, Dingwei, AstraZeneca R & D Boston, Waltham, Massachusetts 02451 (US)
(86) International application number: PCT/GB2004/004784
(87) International publication number: WO 2005/049033

(56) References cited:
- WO-A-01/60816
- WO-A-03/048133

## Description

The present invention relates to novel pyrazole derivatives, their pharmaceutical compositions and methods of use. In addition, the present invention relates to therapeutic methods for the treatment and prevention of cancers and to the use of these pyrazole derivatives in the manufacture of medicaments for use in the treatment and prevention of cancers.

Receptor tyrosine kinases (RTK's) are a sub-family of protein kinases that play a critical role in cell signalling and are involved in a variety of cancer related processes including cell proliferation, survival, angiogenesis and metastasis. Currently up to 100 different RTK's including tropomyosin-related kinases (Trk's) have been identified.

Trk's are the high affinity receptors activated by a group of soluble growth factors called neurotrophins (NT). The Trk receptor family has three members - TrkA, TrkB and TrkC. Among the NTs there are (i) nerve growth factor (NGF) which activates TrkA, (ii) brain-derived growth factor (BDNF) and NT-4/5 which activate TrkB and (iii) NT3 which activates TrkC. Each Trk receptor contains an extra-cellular domain (ligand binding), a trans-membrane region and an intra-cellular domain (including kinase domain). Upon binding of the ligand, the kinase catalyzes auto-phosphorylation and triggers downstream signal transduction pathways.

Trk's are widely expressed in neuronal tissue during its development where Trk's are critical for the maintenance and survival of these cells. A post-embryonic role for the Trk/neurotrophin axis (or pathway), however, remains in question. There are reports showing that Trk's play important role in both development and function of the nervous system (Patapoutian, A. et al Current Opinion in Neurobiology, 2001, 11, 272-280).

In the past decade, a considerable number of literature documentations linking Trk signalling with cancer have published. For example, while Trk's are expressed at low levels outside the nervous system in the adult, Trk expression is increased in late stage prostate cancers. Both normal prostate tissue and androgen- dependent prostate tumours express low levels of Trk A and undetectable levels of Trk B and C. However, all isoforms of Trk receptors as well as their cognate ligands are up-regulated in late stage, androgen-independent prostate cancer. There is additional evidence that these late stage prostate cancer cells become dependent on the Trk/neurotrophin axis for their survival. Therefore, Trk inhibitors may yield a class of apoptosis-inducing agents specific for androgen- independent prostate cancer (Weeraratna, A. T. et al The Prostate, 2000, 45, I40-I48).

Furthermore, very recent literature also shows that over-expression, activation, amplification and/or mutation of Trk's are associated with secretory breast carcinoma *(*Cancer Cell, 2002, 2, 367-376), colorectal cancer (Bardelli et al Science, 2003, 300, 949-949) and ovarian cancer (Davidson, B. et al Clinical Cancer Research, 2003, 9, 2248-2259)..

There are a few reports of selective Trk tyrosine kinase inhibitors. Cephalon described CEP₋751, CEP-701 (George, D. et al Cancer Research, 1999, 59, 2395-2341) and other indolocarbazole analogues (WO0114380) as Trk inhibitors. It was shown that CEP-701 and/or CEP751, when combined with surgically or chemically induced androgen ablation, offered better efficacy compared with mono-therapy alone. GlaxoSmithKline disclosed certain oxindole compounds as TrkA inhibitors in WO0220479 and W00220513. Recently, Japan Tobacco reported pyrazolyl condensed cyclic compounds as Trk inhibitors (JP2003231687A).

In addition to the above, Vertex Pharmaceuticals have described pyrazole compounds as inhibitors of GSK3, Aurora, etc. in WO0250065, WO0262789 and WO03027111; and AstraZeneca have reported pyrazole compounds as inhibitors against IGF-1 receptor kinase (WO0348133).

Amgen Inc application WO01/60816 discloses pyrimidine derivative kinase inhibitors for treating kinase mediated diseases.

### Summary of the invention

In accordance with the present invention, the applicants have hereby discovered novel pyrazole compounds, or pharmaceutically acceptable salts thereof, which possess Trk kinase inhibitory activity and are accordingly useful for their anti-proliferation and/or proapoptotic (such as anti-cancer) activity and in methods of treatment of the human or animal body. The invention also relates to processes for the manufacture of said pyrazole compounds, or pharmaceutically acceptable salts thereof, to pharmaceutical compositions containing them and to their use in the manufacture of medicaments for use in the production of an anti-proliferation and/or proapoptotic effect in warm-blooded animals such as man.

Also in accordance with the present invention the applicants provide methods of using such pyrazole compounds, or pharmaceutically acceptable salts thereof, in the treatment of cancer.

The properties of the compounds claimed, in this invention are expected to be of value in the treatment of disease states associated with cell proliferation such as cancers (solid tumours and leukaemia), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

Furthermore; the compounds, or pharmaceutically acceptable salts thereof, of the invention are expected to be of value in the treatment or prophylaxis of cancers selected from oesophageal cancer, myeloma, hepatocellular, pancreatic, cervical cancer, ewings tumour, neuroblastoma, kaposis sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, renal cancer, lymphoma and leukaemia; particularly ovarian cancer, breast cancer, colorectal cancer, prostate cancer and lung cancer - NSCLC and SCLC; more particularly prostate cancer; and more particularly hormone refractory prostate cancer.

### Detailed description of the invention

Accordingly, the present invention provides a compound of formula (I): 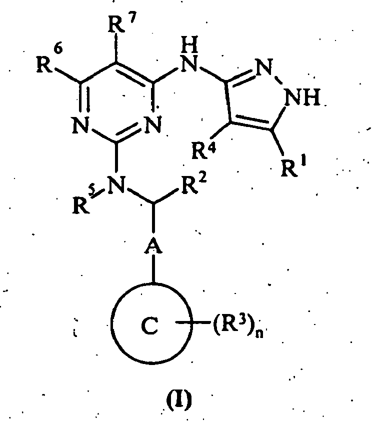 wherein:
**A** is a direct bond;
**Ring C** is carbocyclyl or heterocyclyl;
**R¹** and **R⁴** are independently selected from hydrogen, halo, nitro; cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoylox, *N*-(C₁-₆alkyl)amino,
   *N,N*-(C₁₋₆alkyl)₂amino, C₁₋₆alkanoylamino, *N*-(C₁₋₆alkyl)carbamoyl,
   *N,N*-(C₁₋₆alkyl)₂carbamoyl, C₁₋₆alkylS(O)ₐ wherein a is 0 to 2, C₁₋₆alkoxycarbonyl,
   *N*-(C₁₋₆alkyl)sulphamoyl, *N,N*-(C₁₋₆alkyl)₂sulphamoyl, C₁₋₆alkylsulphonylamino, carbocyclyl or heterocyclyl; wherein R¹ and R⁴ independently of each other may be optionally substituted on carbon by one or more R⁸; and wherein if said heterocyclyl contains an -NH- moiety that nitrogen may be optionally substituted by a group selected from R⁹;
**R²** is selected from C₁₋₆alkyl; wherein R² may be optionally substituted on carbon by one or more R¹⁰;
**R¹⁰** is selected from halo, hydroxy, carboxy, amine, C₁₋₆alkoxy,
   *N,N*-(C₁₋₆alkyl)₂amino, C₁₋₆alkanoylamino, *N*-(C₁₋₆alkyl)carbamoyl,
   *N,N*-(C₁₋₆alkyl)₂carbamoyl or heterocyclyl; wherein R¹⁰ may be optionally substituted on carbon by one or more R^{19a}; and wherein if said heterocyclyl contains an -NH- moiety that nitrogen may be optionally substituted by a group selected from R^{20a};
**R^{19a}** is selected from hydroxy or C₁₋₆alkoxy;
**R^{20a}** is selected from C₁₋₆alkyl;
**R³** is fluoro;
**R⁵** is hydrogen or optionally substituted C₁₋₆alkyl; wherein said optional substituents are selected from one or more R¹⁴;
**R⁶** and **R⁷** are independently selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂-₆alkynyl, C₁₋₆alkoxy, C₁₋₆alkanoyl, C₁₋₆alkanoyloxy, *N*-(C₁₋₆alkyl)amino,
   *N,N*-(C₁₋₆alkyl)₂amino, C₁₋₆alkanoylamino, *N*-(C₁₋₆alkyl)carbamoyl,
   *N,N*-(C₁₋₆alkyl)₂carbamoyl, C₁₋₆alkylS(O)ₐ wherein a is 0 to 2, C₁₋₆alkoxycarbonyl,
   *N*-(C₁₋₆alkyl)sulphamoyl, *N,N*-(C₁₋₆alkyl)₂sulphamoyl, C₁₋₆alkylsulphonylamino, carbocyclyl or heterocyclyl; wherein R⁶ and R⁷ independently of each other may be optionally substituted on carbon by one or more R¹⁵; and wherein if said heterocyclyl contains an -NH- moiety that nitrogen may be optionally substituted by a group selected from R¹⁶;
or **R⁶** and **R⁷** together with the pyrimidine bond to which they are attached form a 5 or 6 membered carbocyclic ring or a 5 or 6 membered heterocyclic ring wherein said ring is fused to the pyrimidine of formula **(I)**; wherein the double bonds of the resulting bicyclic ring may be further delocalised across the whole of the bicyclic ring; and wherein said carbocyclic ring or heterocyclic ring may be optionally substituted on carbon by one or more R¹⁷ and
   wherein if said heterocyclic ring contains an -NH- moiety that nitrogen may be optionally substituted by a group selected from R¹⁸;
**n** = 1;
**R⁸, R¹⁴, R¹⁵** and **R¹⁷** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino,
   *N,N*-(C₁₋₆alkyl)₂amino, C₁₋₆alkanoylamino, *N*-(C₁₋₆alkyl)carbamoyl,
   *N,N*-(C_{1*-*6}alkyl)₂carbamoyl, C₁₋₆alkylS(O)ₐ wherein a is 0 to 2, C₁₋₆alkoxycarbonyl,
   *N*-(C₁₋₆alkyl)sulphamoyl, *N,N*-(C₁₋₆alkyl)₂sulphamoyl, C₁₋₆alkylsulphonylamino, carbocyclyl or heterocyclyl; wherein R⁸, R¹⁴, R¹⁵ and R¹⁷ independently of each other may be optionally substituted on carbon by one or more R¹⁹; and wherein if said heterocyclyl contains an -NH- moiety that nitrogen may be optionally substituted by a group selected from R²⁰;
**R⁹, R¹⁶, R¹⁸** and **R²⁰** are independently selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N,N*-(C₁₋₆alkyl)₂carbamoyl, benzyl, benzyloxycarbonyl, benzoyl and phenylsulphonyl; wherein R⁹, R¹⁶, R¹⁸ and R²⁰ independently of each other may be optionally substituted on carbon by on or more R²¹;
**R¹⁹** and **R²¹** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino,
   *N,N*-(C₁₋₆alkyl)₂amino, C₁₋₆alkanoylamino, *N*-(C₁₋₆alkyl)carbamoyl,
   *N,N*-(C₁₋₆alkyl)₂carbamoyl, C₁₋₆alkylS(O)ₐ wherein a is 0 to 2, C₁₋₆alkoxycarbonyl,
   *N*-(C₁₋₆alkyl)sulphamoyl, *N,N*-(C₁₋₆alkyl)₂sulphamoyl, C₁₋₆alkylsulphonylamino, carbocyclyl or heterocyclyl; wherein R¹⁹ and R²¹ independently of each other may be optionally substituted on carbon by one or more R²³; and wherein if said heterocyclyl contains an -NH- moiety that nitrogen may be optionally substituted by a group selected from R²⁴;
**R²³** is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl,
   *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl,
   *N*-methylsulphamoyl, *N*-ethylsulphamoyl, *N*,*N*-dimethylsulphamoyl, *N,N*-diethylsulphamoyl or *N*-methyl-*N*-ethylsulphamoyl; and
R²⁴ is selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N,N*-(C₁₋₆alkyl)₂carbamoyl, benzyl, benzyloxycarbonyl, benzoyl and phenylsulphonyl;
wherein a "carbocyclyl" is a saturated, partially saturated or unsaturated, mono or bicyclic carbon ring that contains 3-12 atoms; wherein a -CH₂- group can optionally be replaced by a -C(O)-;
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked,
wherein a -CH₂- group can optionally be replaced by a -C(O)-, and a ring sulphur atom may be optionally oxidised to form the S-oxides;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I)**; wherein a -CH₂- group can optionally be replaced by a -C(O)-; or a pharmaceutically acceptable salt thereof.

In a further aspect of the invention there is provided a compound of formula (I) wherein:
**A** is a direct bond;
**Ring C** is carbocyclyl or heterocyclyl;
**R¹** and **R⁴** are independently selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino,
   *N,N*-(C₁₋₆alkyl)₂amino, C₁₋₆alkanoylamino, *N*-(C₁₋₆alkyl)carbamoyl,
   *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, C₁₋₆alkylsulphonylamino, carbocyclyl or heterocyclyl; wherein R¹ and R⁴ independently of each other may be optionally substituted on carbon by one or more R⁸; and wherein if said heterocyclyl contains an -NH- moiety that nitrogen may be optionally substituted by a group selected from R⁹;
**R²** is selected from C₁₋₆alkyl; wherein R² may be optionally substituted on carbon by one or more R¹⁰;
**R¹⁰** is selected from halo, hydroxy, carboxy, amino, C₁₋₆alkoxy,
   *N,N*-(C₁₋₆alkyl)₂amino, C₁₋₆alkanoylamino, *N-*(C₁₋₆alkyl)carbamoyl,
   *N,N*-(C₁₋₆alkyl)₂carbamoyl or heterocyclyl; wherein R¹⁰ may be optionally substituted on carbon by one or more R^{19a}; and wherein if said heterocyclyl contains an -NH- moiety that nitrogen may be optionally substituted by a group selected from R^{20a};
**R^{19a}** is selected from hydroxy or C₁₋₆alkoxy;
**R^{20a}** is selected from C₁₋₆alkyl;
**R³** is fluoro;
**R⁵** is hydrogen or optionally substituted C₁₋₆alkyl; wherein said optional substituents are selected from one or more R¹⁴;
**R⁶** and **R⁷** are independently selected from selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino,
   *N,N*-(C₁₋₆alkyl)₂amino, C₁₋₆alkanoylamino, *N*-(C₁₋₆alkyl)carbamoyl,
   *N,N*-(C₁₋₆alkyl)₂carbamoyl, C₁₋₆alkylS(O)ₐ wherein a is 0 to 2, C₁₋₆alkoxycarbonyl,
   *N*-(C₁₋₆alkyl)sulphamoyl, *N,N*-(C₁₋₆alkyl)₂sulphamoyl, C₁₋₆alkylsulphonylamino, carbocyclyl or heterocyclyl; wherein R⁶ and R⁷ independently of each other may be optionally substituted on carbon by one or more R¹⁵ ; and wherein if said heterocyclyl contains an -NH- moiety that nitrogen may be optionally substituted by a group selected from R¹⁶;
or **R⁶** and **R⁷** together with the pyrimidine bond to which they are attached form a 5 or 6 membered carbocyclic ring or heterocyclic ring wherein said ring is fused to the pyrimidine of formula **(I)**; and wherein said carbocyclic ring or heterocyclic ring may be optionally substituted on carbon by one or more R¹⁷; and wherein if said heterocyclic ring contains an -NH- moiety that nitrogen may be optionally substituted by a group selected from R¹⁸;
**n** = 1;
**R⁸, R¹⁴, R¹⁵** and **R¹⁷** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino,
   *N,N*-(C₁₋₆alkyl)₂amino, C₁₋₆alkanoylamino, *N*-(C₁₋₆alkyl)carbamoyl,
   *N,N*-(C₁₋₆alkyl)₂carbamoyl, C₁₋₆alkylS(O)ₐ wherein a is 0 to 2, C₁₋₆alkoxycarbonyl,
   *N*-(C₁₋₆alkyl)sulphamoyl, *N,N*-(C₁₋₆alkyl)₂sulphamoyl, C₁₋₆alkylsulphonylamino, carbocyclyl or heterocyclyl; wherein R⁸, R¹⁴, R¹⁵ and R¹⁷ independently of each other may be optionally substituted on carbon by one or more R¹⁹; and wherein if said heterocyclyl contains an -NH- moiety that nitrogen may be optionally substituted by a group selected from R²⁰;
**R⁹, R¹⁶, R¹⁸** and **R²⁰** are independently selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N,N*-(C₁₋₆alkyl)₂carbamoyl, benzyl, benzyloxycarbonyl, benzoyl and phenylsulphonyl; wherein R⁹, R¹⁶, R¹⁸ and R²⁰ independently of each other may be optionally substituted on carbon by on or more R²¹;
**R¹⁹** and **R²¹** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino,
   *N,N*-(C₁₋₆alkyl)₂amino, C₁₋₆alkanoylamino, *N*-(C₁-₆alkyl)carbamoyl,
   *N,N*-(C₁₋₆alkyl)₂carbamoyl, C₁₋₆alkylS(O)ₐ wherein a is 0 to 2, C₁₋₆alkoxycarbonyl,
   *N*-(C₁₋₆alkyl)sulphamoyl, *N,N-*(C₁₋₆alkyl)₂sulphamoyl, C₁₋₆alkylsulphonylamino, carbocyclyl or heterocyclyl; wherein R¹⁹ and R²¹ independently of each other may be optionally substituted on carbon by one or more R²³; and wherein if said heterocyclyl contains an -NH- moiety that nitrogen may be optionally substituted by a group selected from R²⁴;
**R²³** is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl,
   *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl,
   *N*-methylsulphamoyl, *N*-ethylsulphamoyl, *N,N*-dimethylsulphamoyl, *N,N*-diethylsulphamoyl or *N*-methyl-*N*-ethylsulphamoyl; and
**R²⁴** is selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N,N*-(C₁₋₆alkyl)₂carbamoyl, benzyl, benzyloxycarbonyl, benzoyl and phenylsulphonyl;
   wherein a "carbocyclyl" is a saturated, partially saturated or unsaturated, mono or bicyclic carbon ring that contains 3-12 atoms; wherein a -CH₂- group can optionally be replaced by a -C(O)-;
   wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides;
   wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a C(O)-, and a ring sulphur atom may be optionally oxidized to form the S-oxides; and
   wherein where "R⁶ and R⁷ together with the bond to which they are attached form a 5 or 6 membered carbocyclic ring".said ring is a partially saturated or unsaturated, mono or bicyclic carbon ring that contains 5 or 6 atoms two atoms of which are shared with the pyrimidine ring of formula **(I);** wherein a -CH₂- group can optionally be replaced by a -C(O)-; or a pharmaceutically acceptable salt thereof.

Preferred values of the variable groups contained in formula **(I)** are as follows. Such values may be used, where appropriate, with any of the definitions, claims or embodiments defined hereinbefore or hereinafter.

Ring C is carbocyclyl; wherein a "carbocyclyl" is a saturated, partially saturated or ' unsaturated, mono or cyclic carbon ring that contains 3-12 atoms; wherein a -CH₂- group can optionally be replaced by a -C(O)-.

Ring C is heterocyclyl; wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

Ring C is phenyl or thienyl.

Ring C is phenyl.

Ring C is thienyl.

Ring C is thienyl, pyridyl, thiazolyl.

Ring C is thien-2-yl, pyrid-2-yl, thiazol-2-yl.

Ring C is phenyl or thien-2-yl.

Ring C is phenyl, thienyl, pyridyl, thiazolyl.

Ring C is phenyl, thien-2-yl, pyrid-2-yl, thiazol-2-yl.

Ring C is not pyridyl or isoxazolyl.

Ring C is not pyrid-2-yl, pyrid-3-yl or isoxazol-5-yl.

Ring C and (R³)ₙ together are 4-fluorophenyl.

R¹ is selected from hydrogen, C₁₋₆alkyl, C₁₋₆alkoxy, *N,N*-(C₁₋₆alkyl)₂amino, C₁₋₆alkylS(O)ₐ wherein a is 0 or carbocyclyl; wherein R¹ may be optionally substituted on carbon by one or more R⁸; wherein
R⁸ is selected from halo or carbocyclyl;
wherein a "carbocyclyl" is a saturated, partially saturated or unsaturated, mono or bicyclic carbon ring that contains 3-12 atoms; wherein a -CH₂- group can optionally be replaced by a -C(O)-.

R¹ is selected from hydrogen, C₁₋₆alkyl, C₁₋₆alkoxy, *N,N*-(C₁₋₆alkyl)₂amino, C₁₋₆alkylS(O)ₐ wherein a is 0 or carbocyclyl; wherein a "carbocyclyl" is a saturated, partially saturated or unsaturated, mono or bicyclic carbon ring that contains 3-12 atoms; wherein a -CH₂- group can optionally be replaced by a -C(O)-.

R¹ is selected from hydrogen, methyl, ethyl, isopropyl, t-butyl, methoxy, ethoxy, propoxy, isopropoxy, sec-butoxy" dimethylamino, methylthio or cyclopropyl; wherein

R⁸ is selected from fluoro, cyclopropyl or phenyl.

R¹ is selected from hydrogen, methyl, ethyl, t-butyl, methoxy, ethoxy, dimethylamino, methylthio or cyclopropyl.

R¹ is selected from hydrogen, methyl, ethyl, isopropyl, t-butyl, trifluoromethyl, cyclopropylmethyl, benzyl, methoxy, ethoxy, propoxy, isopropoxy, sec-butoxy,
dimethylamino, methylthio or cyclopropyl.

R¹ is selected from hydrogen, methyl, ethyl, t-butyl, methoxy, dimethylamino, methylthio or cyclopropyl.

R¹ is cyclopropyl.

R⁴ is hydrogen.

R² is selected from C₁₋₆alkyl.

R² is selected from methyl, ethyl or isopropyl.

R² is selected from C₁₋₆alkyl; wherein R² may be optionally substituted on carbone by one or more R¹⁹.

R² is selected from methyl, ethyl or isopropyl;- wherein R² may be optionally substituted on carbon by one or more R¹⁰.

R² is selected from C₁₋₆alkyl; wherein R² may be optionally substituted on carbon by one or more R¹⁰;

R¹⁰ is selected from halo, hydroxy, carboxy, amino, C₁₋₆alkoxy,
*N,N*-(C₁₋₆alkyl)₂-amino*,* C₁₋₆alkanoylamino, *N*-(C₁₋₆alkyl)carbamoyl,
*N,N*-(C₁₋₆alkyl)₂carbamoyl or heterocyclyl; wherein R¹⁰; may be optionally substituted on carbon by one or more R¹⁹; and wherein if said heterocyclyl contains an -NH- moiety that nitrogen may be optionally substituted by a group selected from R²⁰;

R¹⁹ is selected from hydroxy or C₁₋₆alkoxy;

R²⁰ is selected from C₁₋₆alkyl,
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R² is selected from C₁₋₆alkyl; wherein R² may be optionally substituted on carbon by one or more R¹⁰; wherein

R¹⁰ is selected from hydroxy, carboxy, C₁₋₆alkoxy, *N,N*-(C₁₋₆alkyl)₂amino or heterocyclyl; wherein R¹⁰ may be optionally substituted on carbon by one or more R¹⁹; and wherein if said heterocyclyl contains an -NH- moiety that nitrogen may be optionally substituted by a group selected from R²⁰;

R²⁰ is selected from C₁₋₆alkyl; and

R¹⁹ is selected from hydroxy or C₁₋₆alkoxy;
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R² is selected from methyl, ethyl or isopropyl; wherein R² may be optionally substituted on carbon by one or more R¹⁰;

R¹⁰ is selected from fluoro, hydroxy, carboxy, amino, methoxy, dimethylamino, *N-*methyl-*N-*ethylamino, acetylamino, *N-*methylcarbamoyl, *N-*ethylcarbamoyl, *N,N*-dimethylcarbamoyl, pyrrolidin-1-yl, piperazinyl or morpholino; wherein R¹⁰ may be optionally substituted on carbon by one or more R¹⁹; and wherein if said piperazinyl contains an -NH- moiety that nitrogen may be optionally substituted by a group selected from R²⁰;

R¹⁹ is selected from hydroxy or methoxy;

R²⁰ is selected from methyl.

R² is selected from methyl, ethyl or isopropyl; wherein R² may be optionally substituted on carbon by one or more R¹⁰; wherein

R¹⁰ is selected from hydroxy, carboxy, methoxy, *N*-methyl-*N*-ethylamino, diethylamino, pyrrolidinyl, piperazinyl or morpholinyl; wherein R¹⁰ may be optionally substituted on carbon by one or more R¹⁹; and wherein said piperazinyl may be optionally substituted on nitrogen by a group selected from R²⁰;

R²⁰ is selected from methyl; and

R¹⁹ is selected from hydroxy or methoxy.

R² is selected from methyl, ethyl or isopropyl; wherein R² may be optionally substituted on carbon by one or more R¹⁰; wherein

R¹⁰ is selected from hydroxy, carboxy, methoxy, *N*-methyl-*N*-ethylamino, diethylamino, pyrrolidin-1-yl, piperazin-1-yl or morpholino; wherein R¹⁰ may be optionally substituted on carbon by one or more R¹⁹; and wherein said piperazinyl may be optionally substituted on nitrogen by a group selected from R²⁰;

R²⁰ is selected from methyl; and

R¹⁹ is selected from hydroxy or methoxy.

R² is selected from methyl, ethyl, trifluoromethyl, hydroxymethyl, carboxymethyl, aminomethyl, methoxymethyl, morpholinomethyl, 1-hydroxyethyl, 2-hydroxyethyl, 1-carboxyethyl, 2-dimethylaminoethyl, 2-diethylaminoethyl, acetamidomethyl, 2-[*N*-methyl-*N-*(2-methoxyethyl)amino]ethyl, 2-[*N*-methyl-*N*-(2-hydroxyethyl)amino]ethyl, 2-(*N-*methylcarbamoyl)ethyl, 2-[*N*-(2-hydroxyethyl)carbamoyl]ethyl, 2-(*N,N-*dimethylcarbamoyl)ethyl, 2-morpholinoethyl, 2-pyrrolidin-1-ylethyl or 2-(1-methylpiperazin-4-yl)ethyl, 1-methyl-2-hydroxyethyl.

R² is selected from methyl; wherein R² may be optionally substituted on carbon by one or more R¹⁰; wherein

R¹⁰ is selected from hydroxy.

R⁵ is hydrogen.

R⁵ is C₁₋₆alkyl.

R⁵ is optionally substituted C₁₋₆alkyl; wherein said optional substituents are selected from one or more R¹⁴.

R⁵ is hydrogen or optionally substituted C₁₋₆alkyl; wherein said optional substituents are selected from one or more R¹⁴; wherein

R¹⁴ is selected from hydroxy.

R⁵ is hydrogen, methyl or optionally substituted ethyl; wherein said optional substituents are selected from one or more R¹⁴; wherein

R¹⁴ is selected from hydroxy.

R⁵ is hydrogen or optionally substituted ethyl; wherein said optional substituents are selected from one or more R¹⁴, wherein

R¹⁴ is selected from hydroxy.

R⁵ is hydrogen, methyl or 2-hydroxyethyl.

R⁵ is hydrogen or 2-hydroxyethyl.

R⁵ is hydrogen.

R⁶ and R⁷ are independently selected from hydrogen, halo, nitro, cyano, amino, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbarnoyl, C₁₋₆alkylS(O)ₐ wherein a is 0 to 2, C₁₋₆alkoxycarbonyl, *N*-(C₁₋₆alkyl)sulphamoyl, *N*,*N*-(C₁₋₆alkyl)₂sulphamoyl, C₁₋₆alkylsulphonylamino, carbocyclyl or heterocyclyl; wherein R⁶ and R⁷ independently of each other may be optionally substituted on carbon by one or more R¹⁵; and wherein if said heterocyclyl contains an -NH- moiety that nitrogen may be optionally substituted by a group selected from R¹⁶; wherein a "carbocyclyl" is a saturated, partially saturated or unsaturated, mono or bicyclic carbon ring that contains 3-12 atoms; wherein a -CH₂- group can optionally be replaced by a -C(O)-; and wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R⁶ and R⁷ are independently selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆alkyl)₂amino; C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N*,*N*-(C₁₋₆alkyl)₂sulphamoyl, C₁₋₆alkylsulphonylamino, carbocyclyl or heterocyclyl; wherein R⁶ and R⁷ independently of each other may be optionally substituted on carbon by one or more R¹⁵; and wherein if said heterocyclyl contains an -NH- moiety that nitrogen may be optionally substituted by a group selected from R¹⁶; wherein a "carbocyclyl" is a saturated, partially saturated or unsaturated, mono or bicyclic carbon ring that contains 3-12 atoms; wherein a -CH₂- group can optionally be replaced by a -C(O)-; and wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R⁶ and R⁷ are independently selected from hydrogen, halo, C₁₋₆alkyl, *N*-(C₁₋₆alkyl)amino, *N*-(C₁₋₆alkyl)carbamoyl or C₁₋₆alkoxycarbonyl; wherein R⁶ and R⁷ independently of each other may be optionally substituted on carbon by one or more, R¹⁵.

R⁶ and R⁷ are independently selected from hydrogen, halo, nitro, cyano, amino, C₁₋₆alkyl, *N*-(C₁₋₆alkyl)amino, *N*,*N*-(C₁₋₆alkyl)₂amino, *N*-(C₁₋₆alkyl)carbamoyl, C₁₋₆alkoxycarbonyl or heterocyclyl; wherein R⁶ and R⁷ independently of each other may be optionally substituted on carbon by one or more R¹⁵; and wherein if said heterocyclyl contains an -NH- moiety that nitrogen may be optionally substituted by a group selected from R¹⁶; wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R⁶ and R⁷ are independently selected from hydrogen, fluoro, chloro, bromo, methyl, methylamino, ethylamino, propylamino, *N*-(ethyl)carbamoyl, methoxycarbonyl, ethoxycarbonyl or butoxycarbonyl; wherein R⁶ and R⁷ independently of each other may be optionally substituted on carbon by one or more R¹⁵.

R⁶ and R⁷ are independently selected from hydrogen, fluoro, chloro, bromo, nitro, - cyano, amino, methyl, methylamino, ethylamino, propylamino, isopropylamino, dimethylamino, *N*-methyl-*N*-propylamino, *N*-ethylcarbamoyl, methoxycarbonyl, ethoxycarbonyl, butoxycarbonyl, morpholino, pyrrolidinyl or piperazinyl; wherein R⁶ and R⁷ independently of each other may be optionally substituted on carbon by one or more R¹⁵; and wherein said piperazinyl may be optionally substituted on nitrogen by a group selected from R¹⁶.

R⁶ and R⁷ are independently selected from hydrogen, fluoro, chloro, bromo, methyl, ethylamino, propylamino, *N*-(ethyl)carbamoyl, methoxycarbonyl, ethoxycarbonyl or butoxycarbonyl; wherein R⁶ and R⁷ independently of each other may be optionally substituted on carbon by one or more R¹⁵.

R⁶ and R⁷ together with the pyrimidine bond to which they are attached form a 5 or 6 membered carbocyclic ring or a 5 or 6 membered heterocyclic ring wherein said ring is fused to the pyrimidine of formula **(I)**; wherein the double bonds of the resulting bicyclic ring may be further delocalised across the whole of the bicyclic ring; and wherein said carbocyclic ring or heterocyclic ring may be optionally substituted on carbon by one or more R¹⁷; and wherein if said heterocyclic ring contains an -NH- moiety that nitrogen may be optionally substituted by a group selected from R¹⁸;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I)**; wherein a -CH₂- group can optionally be replaced by a -C(O)-.

R⁶ and R⁷ together with the pyrimidine bond to which they are attached form a 5 or 6 membered carbocyclic ring or heterocyclic ring wherein said ring is fused to the pyrimidine of formula **(I)**; and wherein said carbocyclic ring or heterocyclic ring may be optionally substituted on carbon by one or more R¹⁷; and wherein if said heterocyclic ring contains an -NH- moiety that nitrogen may be optionally substituted by a group selected from R¹⁸;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I)**; wherein a -CH₂- group can optionally be replaced by a -C(O)-.

R⁶ and R⁷ together with the pyrimidine bond to which they are attached form a 5 or 6 membered carbocyclic ring or heterocyclic ring wherein said ring is fused to the pyrimidine of formula **(I)**; and wherein said carbocyclic ring or heterocyclic ring may be optionally substituted on carbon by one or more R¹⁷;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I)**; wherein a -CH₂- group can optionally be replaced by a -C(O)-.

R⁶ and R⁷ together with the pyrimidine to which they are attached form a bicyclic ring selected from quinazolinyl, thieno[3,2-d]pyrimidinyl, thieno[2,3-d]pyrimidinyl, 1*H-*pyrazolo[3,4-d]pyrimidinyl, thieno[3,4-d]pyrimidinyl, pyrido[2,3-d]pyrimidinyl, 5,6,7,8-tetrahydro-pyrido[4,3-d]pyrimidinyl, 5,6,7,8-tetrahydro-pyrido[2,3-d]pyrimidinyl or 5,6,7,8-tetrahydro-pyrido[3,4-*d*]pyrimidinyl; and wherein said bicyclic ring may be optionally substituted on carbon by one or more R¹⁷; and wherein said 5,6,7,8-tetrahydro-pyrido[4,3-d]pyrimidinyl, 5,6,7,8-tetrahydro-pyrido[2,3-d]pyrimidinyl or 5,6,7,8-tetrahydro-pyrido[3,4-d]pyrimidinyl may be optionally substituted on nitrogen by a group selected from R¹⁸.

R⁶ and R⁷ together with the pyrimidine to which they are attached form quinazolinyl, thieno[3,2-d]pyrimidinyl, thieno[2,3-d]pyrimidinyl, thieno[3,4-d]pyrimidinyl, 1*H-*pyrazolo[3,4-d]pyrimidinyl or pyrido[2,3-d]pyrimidinyl; and wherein said quinazolinyl, thieno[3,2-d]pyrimidinyl, thieno[2,3-d]pyrimidinyl, thieno[3,4-d]pyrimidinyl, 1*H-*pyrazolo[3,4-d]pyrimidinyl or pyrido[2,3-d]pyrimidinyl may be optionally substituted on carbon by one or more R¹⁷.

R⁶ and R⁷ are independently selected from hydrogen, halo, nitro, cyano, amino, C₁₋₆alkyl, *N*-(C₁₋₆alkyl)amino, *N*,*N*-(C₁₋₆alkyl)₂amino, *N*-(C₁₋₆alkyl)carbamoyl, C₁₋₆alkoxycarbonyl or heterocyclyl; wherein R⁶ and R⁷ independently of each other may be optionally substituted on carbon by one or more R¹⁵; and wherein if said heterocyclyl contains an -NH- moiety that nitrogen, may be optionally substituted by a group selected from R¹⁶;
or R⁶ and R⁷ together with the pyrimidine bond to which they are attached form a 6 membered carbocyclic ring or a 5 or 6 membered heterocyclic ring wherein said ring is fused to the pyrimidine of formula **(I)**; wherein the double bonds of the resulting bicyclic ring may be further delocalised across the whole of the bicyclic ring; and wherein said carbocyclic ring or heterocyclic ring may be optionally substituted on carbon by one or more R¹⁷ and wherein if said heterocyclic ring contains an -NH- moiety that nitrogen may be optionally substituted by a group selected from R¹⁸;
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 6 membered carbocyclic ring" said ring is a partially saturated or unsaturated, mono or bicyclic carbon ring that contains atoms two atoms of which are shared with the pyrimidine ring of formula **(I)**; wherein a -CH₂- group can optionally be replaced by a -C(O)-.

R⁶ and R⁷ are independently selected from hydrogen, halo, C₁₋₆alkyl, *N*-(C₁₋₆alkyl)amino, *N*-(C₁₋₆alkyl)carbamoyl or C₁₋₆alkoxycarbonyl; wherein R⁶ and R⁷ independently of each other may be optionally substituted on carbon by one or more R¹⁵;
or R⁶ and R⁷ together with the pyrimidine bond to which they are attached form a 5 or 6 membered carbocyclic ring or heterocyclic ring wherein said ring is fused to the pyrimidine of formula (I); and wherein said carbocyclic ring or heterocyclic ring may be optionally substituted on carbon by one or more R¹⁷;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I)**; wherein a -CH₂- group can optionally be replaced by a -C(O)-.

R⁶ and R⁷ are independently selected from hydrogen, fluoro, chloro, bromo, nitro, cyano, amino, methyl, methylamino, ethylamino, propylamino, isopropylamino, dimethylamino, *N*-methyl-*N*-propylamino, *N*-ethylcarbamoyl, methoxycarbonyl, ethoxycarbonyl, butoxycarbonyl, morpholino, pyrrolidinyl or piperazinyl; wherein R⁶ and R⁷ independently of each other may be optionally substituted on carbon by one or more R¹⁵; and wherein said piperazinyl may be optionally substituted on nitrogen by a group selected from R¹⁶;
or R⁶ and R⁷ together with the pyrimidine to which they are attached form a bicyclic ring selected from quinazolinyl, thieno[3,2-d]pyrimidinyl, thieno[2,3-d]pyrimidinyl, 1*H-*pyrazolo[3,4-d]pyrimidinyl, thieno[3,4-d]pyrimidinyl, pyrido[2,3-d]pyrimidinyl, 5,6,7,8-tetrahydro-pyrido[4,3-d]pyrimidinyl, 5,6,7,8-tetrahydro-pyrido[2,3-d]pyrimidinyl or 5,6,7,8-tetrahydro-pyrido[3,4-d]pyrimidinyl; and wherein said bicyclic ring may be optionally substituted on carbon by one or more R¹⁷; and wherein said 5,6,7,8-tetrahydro-pyrido[4,3-d]pyrimidinyl, 5,6,7,8-tetrahydro-pyrido[2,3-d]pyrimidinyl or 5,6,7,8-tetrahydro-pyrido[3,4-d]pyrimidinyl may be optionally substituted on nitrogen by a group selected from R¹⁸.

R⁶ and R⁷ are independently selected from hydrogen, fluoro, chloro, bromo, methyl, methylamino, ethylamino, propylamino, *N*-(ethyl)carbamoyl, methoxycarbonyl, ethoxycarbonyl or butoxycarbonyl; wherein R⁶ and R⁷ independently of each other may be optionally substituted on carbon by one or more R¹⁵;
or R⁶ and R⁷ together with the pyrimidine to which they are attached form quinazolinyl, thieno[3,2-d]pyrimidinyl, thieno[2,3-d]pyrimidinyl, thieno[3,4-d]pyrimidinyl, 1*H-*pyrazolo[3,4-d]pyrimidinyl or pyrido[2,3-d]pyrimidinyl; and wherein said quinazolinyl, thieno[3,2-d]pyrimidinyl, thieno[2,3-d]pyrimidinyl, thieno[3,4-d]pyrimidinyl, 1*H-*pyrazolo[3,4-d]pyrimidinyl or pyrido[2,3-d]pyrimidinyl may be optionally substituted on carbon by one or more R¹⁷.

R⁶ and R⁷ are independently selected from hydrogen, fluoro, chloro, bromo, methyl, ethylamino, propylamino, *N*-(ethyl)carbamoyl, methoxycarbonyl, ethoxycarbonyl or butoxycarbonyl; wherein R⁶ and R⁷ independently of each other may be optionally substituted on carbon by one or more R¹⁵;
or R⁶ and R⁷ together with the pyrimidine to which they are attached form quinazolinyl, thieno[3,2-d]pyrimidinyl, thieno[2,3-d]pyrimidinyl, thieno[3,4-d]pyrimidinyl, 1*H-*pyrazolo[3,4-d]pyrimidinyl or pyrido[2,3-d]pyrimidinyl; and wherein said quinazolinyl, thieno[3,2-d]pyrimidinyl, thieno[2,3-d]pyrimidinyl, thieno[3,4-d]pyrimidinyl, 1*H-*pyrazolo[3,4-d]pyrimidinyl or pyrido[2,3-d]pyrimidinyl may be optionally substituted on carbon by one or more R¹⁷.

R⁶ and R⁷ are independently selected from hydrogen, halo, nitro, cyano, amino, C₁₋₆alkyl, *N*-(C₁₋₆alkyl)aminb, *N*,*N*-(C₁₋₆alkyl)₂amino, *N*-(C₁₋₆alkyl)carbamoyl, C₁₋₆alkoxycarbonyl or heterocyclyl; wherein R⁶ and R⁷ independently of each other may be optionally substituted on carbon by one or more R¹⁵; and wherein if said heterocyclyl contains an -NH- moiety that nitrogen may be optionally substituted by a group selected from R¹⁶;
or R⁶ and R⁷ together with the pyrimidine bond to which they are attached form a 6 membered carbocyclic ring or a 5 or 6 membered heterocyclic ring wherein said ring is fused to the pyrimidine of formula (I); wherein the double bonds of the resulting bicyclic ring may be further delocalised across the whole of the bicyclic ring; and wherein said carbocyclic ring or heterocyclic ring may be optionally substituted on carbon by one or more R¹⁷; and wherein if said heterocyclic ring contains an -NH- moiety that nitrogen may be optionally substituted by a group selected from R¹⁸;

R¹⁵ is selected from halo, hydroxy, amino, C₁₋₆alkoxy, *N,N*-(C₁₋₆alkyl)₂amino, carbocyclyl or heterocyclyl; wherein R¹⁵ may be optionally substituted on carbon by one or more R¹⁹; and wherein if said heterocyclyl contains an -NH- moiety that nitrogen may be optionally substituted by a group selected from R²⁰;

R¹⁷ is selected from halo, C₁₋₆alkyl or C₁₋₆alkoxy; wherein R¹⁷ may be optionally substituted on carbon by one or more R¹⁹;

R¹⁶ is selected from C₁₋₆alkyl;

R¹⁸ is selected from C₁₋₆alkanoyl;

R¹⁹ is selected from halo, hydroxy, C₁₋₆alkoxy or heterocyclyl; and wherein if said heterocyclyl contains an -NH- moiety that nitrogen may be optionally substituted by a group selected from R²⁴;

R²⁰ is selected from C₁₋₆alkyl; and

R²⁴ is selected from C₁₋₆alkyl;
wherein a "carbocyclyl" is a saturated, partially saturated or unsaturated, mono or bicyclic carbon ring that contains 3-12 atoms; wherein a -CH₂- group can optionally be replaced by a -C(O)-
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 6 membered carbocyclic ring" said ring is a partially saturated or unsaturated, mono or bicyclic carbon ring that contains 6 atoms two atoms of which are shared with the pyrimidine ring of formula (I); wherein a -CH₂- group can optionally be replaced by a -C(O)-.

R⁶ and R⁷ are independently selected from hydrogen, halo, C₁₋₆alkyl, *N*-(C₁₋₆alkyl)amino, *N*-(C₁₋₆alkyl)carbamoyl or C₁₋₆alkoxycarbonyl; wherein R⁶ and R⁷ independently of each other may be optionally substituted on carbon by one or more R¹⁵;
or R⁶ and R⁷ together with the pyrimidine bond to which they are attached form a 5 or 6 membered carbocyclic ring or heterocyclic ring wherein said ring is fused to the pyrimidine of formula (I); and wherein said carbocyclic ring or heterocyclic ring may be optionally substituted on carbon by one or more R¹⁷; wherein

R¹⁵ is selected from halo, hydroxy, carbocyclyl or heterocyclyl; wherein R¹⁵ may be optionally substituted on carbon by one or more R¹⁹; and wherein if said heterocyclyl contains an -NH- moiety that nitrogen may be optionally substituted by a group selected from R²⁰;

R¹⁷ is selected from halo, C₁₋₆alkyl or C₁₋₆alkoxy; wherein R¹⁷ may be optionally substituted on carbon by one or more R¹⁹;

R²⁰ is selected from C₁₋₆alkyl;

R¹⁹ is selected from halo, C₁₋₆alkoxy or heterocyclyl; wherein if said heterocyclyl contains an -NH- moiety that nitrogen may be optionally substituted by a group selected from R²⁴; and

R²⁴ is selected from C₁₋₆alkyl;
wherein a "carbocyclyl" is a saturated, partially saturated or unsaturated, mono or bicyclic carbon ring that contains 3-12 atoms; wherein a -CH₂- group can optionally be replaced by a -C(O)-;
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen; sulphur or oxygen; wherein a -CH₂-group can optionally be replaced by a -C(O)-, and a ring sulphur atom may be optionally oxidized to form the S-oxides; and
herein where "R⁶ and R⁷together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I)**; wherein a -CH₂- group can optionally be replaced by a -C(O)-.

R⁶ and R⁷ are independently selected from hydrogen, fluoro, chloro, bromo, nitro, cyano, amino, methyl, methylamino, ethylamino, propylamino, isopropylamino, dimethylamino, *N*-methyl-*N*-propylamino, *N*-ethylcarbamoyl, methoxycarbonyl, ethoxycarbonyl, butoxycarbonyl, morpholino, pyrrolidinyl or piperazinyl; wherein R⁶ and R⁷ independently of each other may be optionally substituted on carbon by one or more R¹⁵; and wherein said piperazinyl may be optionally substituted on nitrogen by a group selected from R¹⁶;
or R⁶ and R⁷ together with the pyrimidine to which they are attached form a bicyclic ring selected from quinazolinyl, thieno[3,2-d]pyrimidinyl, thieno[2,3-d]pyrimidinyl, 1*H-*pyrazolo[3,4-d]pyrimidinyl, thieno[3,4-d]pyrimidinyl, pyrido[2,3-d]pyrimidinyl, 5,6,7,8-tetrahydro-pyrido[ 4,3-d]pyrimidinyl, 5,6,7,8-tetrahydro-pyrido[2,3-d]pyrimidinyl or 5,6,7,8-tetrahydro-pyrido[3,4-d]pyrimidinyl; and wherein said bicyclic ring may be optionally substituted on carbon by one or more R¹⁷ ; and wherein said 5,6,7,8-tetrahydro-pyrido[4,3-d]pyrimidinyl, 5,6,7,8-tetrahydro-pyrido[2,3-d]pyrimidinyl or 5,6,7,8-tetrahydro-pyrido[3,4-*d*]pyrimidinyl may be optionally substituted on nitrogen by a group selected from R¹⁸;

R¹⁵ is selected from fluoro, hydroxy, amino, ethoxy, dimethylamino, phenyl, pyrrolidinyl, piperazinyl or morpholino; wherein R¹⁵ may be optionally substituted on carbon by one or more R¹⁹; and wherein said piperazinyl may be optionally substituted on nitrogen by a group selected from R²⁰;

R¹⁷ is selected from fluoro, chloro, methyl, methoxy, ethoxy or propoxy wherein R¹⁷ may be optionally substituted on carbon by one or more R¹⁹ ;

R¹⁶ is selected from methyl;

R¹⁸ is selected from acetyl;

R¹⁹ is selected from fluoro, hydroxy, methoxy, piperazinyl, pyrrolidinyl or morpholino; and wherein said piperazinyl may be optionally substituted on nitrogen by a group selected from R²⁴;

R²⁰ is selected from methyl; and

R²⁴ is selected from methyl.

R⁶ and R⁷ are independently selected from hydrogen, fluoro, chloro, bromo, methyl, ethylamino, propylamino, *N*-(ethyl)carbarnoyl, methoxycarbonyl, ethoxycarbonyl or butoxycarbonyl; wherein R⁶ and R⁷ independently of each other may be optionally substituted on carbon by one or more R¹⁵;
or R⁶ and R⁷ together with the pyrimidine to which they are attached form quinazolinyl, thieno[3,2-d]pyrimidinyl, thieno[2,3-d]pyrimidinyl, thieno[3,4-d]pyrimidinyl, 1*H*-pyrazolo[3,4-d]pyrimidinyl or pyrido[2,3-d]pyrimidinyl; and wherein said quinazolinyl, thieno[3,2-d]pyrimidinyl, thieno[2,3-d]pyrimidinyl, thieno[3,4-d]pyrimidinyl, 1*H* pyrazolo[3,4-d]pyrimidinyl or pyrido[2,3-d]pyrimidinyl may be optionally substituted on carbon by one or more R¹⁷; wherein

R¹⁵ is selected from fluoro, hydroxy, phenyl, piperazinyl, pyrrolidinyl or morpholino; wherein R¹⁵ may be optionally substituted on carbon by one or more R¹⁹; and wherein if said piperazinyl contains an -NH- moiety that nitrogen may be optionally substituted by a group selected from R²⁰;

R¹⁷ is selected from fluoro, chloro, methyl, methoxy or ethoxy; wherein R¹⁷ may be optionally substituted on carbon by one or more R¹⁹.

R²⁰ is selected from methyl;

R¹⁹ is selected from fluoro, methoxy, piperazinyl, pyrrolidinyl or morpholino; wherein if said piperazinyl contains an -NH- moiety that nitrogen may be optionally substituted by a group selected from R²⁴; and

R²⁴ is selected from methyl.

R⁶ and R⁷ are independently selected from hydrogen, chloro, bromo or propylamino; wherein R⁶ and R⁷ independently of each other may be optionally substituted on carbon by one or more R¹⁵; wherein R¹⁵ is selected from hydroxy;
or R⁶ and R⁷ together with the Pyrimidine to which they are attached form quinazolinyl

R¹⁰ is selected from halo, hydroxy, carboxy, amino, C₁₋₆alkoxy,
-*N,N*-(C₁₋₆alkyl)₂amino, C₁₋₆alkanoylamino, *N*-(C₁₋₆alkyl)carbamoyl,
*N,N*-(C₁₋₆alkyl)₂carbamoyl or heterocyclyl; wherein R¹⁰ may be optionally substituted on carbon by one or more R¹⁹; and wherein if said heterocyclyl contains an -NH- moiety that nitrogen may be optionally substituted by a group selected from R²⁰, wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R¹⁰ is selected from hydroxy, carboxy, C₁₋₆alkoxy, *N,N*-(C₁₋₆alkyl)₂amino or heterocyclyl; wherein R¹⁰ may be optionally substituted on carbon by one or more R¹⁹; and wherein if said heterocyclyl contains an -NH- moiety that nitrogen may be optionally substituted by a group selected from R²⁰; wherein a "heterocyclyl" is a saturated, partially saturated or unsaturated, mono or bicyclic ring containing 4-12 atoms of which at least one atoms is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R¹⁰ is selected from fluoro, hydroxy, carboxy, amino, methoxy, dimethylamino, N-methyl-*N*-ethylamino, acetylamino, *N-*methylcarbamoyl, *N*-ethylcarbamoyl, N,N-dimethylcarbamoyl, pyrrolidin-1-yl, piperazinyl or morpholino; wherein R¹⁰ may be optionally substituted on carbon by one or more R¹⁹; and wherein if said piperazinyl contains an -NH- moiety that nitrogen may be optionally substituted by a group selected from R²⁰.

R¹⁰ is selected from hydroxy, carboxy, methoxy, *N*-methyl-*N*-ethylamino, diethylamino, pyrrolidinyl, piperazinyl, or morpholinyl; wherein R¹⁰ may be optionally substituted on carbon by one or more R¹⁹; and wherein said piperazinyl may be optionally substituted on nitrogen by a group selected from R²⁰.

R¹⁰ is selected from hydroxy, carboxy, methoxy, *N*-methyl-*N*-ethylamino, diethylamino, pyrrolidin-1-yl, piperazin-1-yl or morpholino; wherein R¹⁰ may be optionally substituted on carbon by one or more R¹⁹; and wherein said piperazinyl may be optionally substituted on nitrogen by a group selected from R²⁰.

R¹⁴ is selected from hydroxy.

R¹⁵ is selected from halo, hydroxy, carbocyclyl or heterocyclyl; wherein R¹⁵ may be optionally substituted on carbon by one or more R¹⁹; and wherein if said heterocyclyl contains an -NH- moiety that nitrogen may be optionally substituted by a group selected from R²⁰; wherein a "carbocyclyl" is a saturated, partially saturated or unsaturated, mono or bicyclic carbon ring that contains 3-12 atoms; wherein a -CH₂- group can optionally be replaced by a -C(O)- and wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R¹⁵ is selected from fluoro, hydroxy, phenyl, piperazinyl, pyrrolidinyl or morpholino; wherein R¹⁵ may be optionally substituted on carbon by one or more R¹⁹; and wherein if said piperazinyl contains an -NH- moiety that nitrogen may be optionally substituted by a group selected from R²⁰.

R¹⁷ is selected from halo, C₁₋₆alkyl or C₁₋₆alkoxy; wherein R¹⁷ may be optionally substituted on carbon by one or more R¹⁹.

R¹⁷ is selected from fluoro, chloro, methyl, Methoxy or ethoxy; wherein R¹⁷ may be optionally substituted on carbon by one or more R¹⁹.

R²⁰ is selected from C₁₋₆alkyl.

R²⁰ is selected from methyl.

R¹⁹ is selected from halo, C₁₋₆alkoxy or heterocyclyl; wherein if said heterocyclyl contains an -NH- moiety that nitrogen may be optionally substituted by a group selected from R²⁴; wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides.

R¹⁹ is selected from fluoro, methoxy, piperazinyl, pyrrolidinyl or morpholino; wherein if said piperazinyl contains an -NH- moiety that nitrogen may be optionally substituted by a group selected from R²⁴.

R¹⁹ is selected from hydroxy or C₁₋₆alkoxy.

R¹⁹ is selected from hydroxy or methoxy.

R²⁴ is selected from C₁₋₆alkyl.

R²⁴ is selected from methyl.

Therefore in a further aspect of the invention there is provided a compound of formula **(I)** (as depicted herein above) wherein:
A is a direct bond;
Ring C is carbocyclyl or heterocyclyl;
R¹ is selected from hydrogen, C₁₋₆alkyl, C₁₋₆alkoxy, *N,N*-(C₁₋₆alkyl)₂amino, C₁₋₆alkylS(O)ₐ wherein a is 0 or carbocyclyl; wherein R¹ may be optionally substituted on carbon by one or more R⁸;
R² is selected from C₁₋₆alkyl; wherein R² may be optionally substituted on carbon by one or more R¹⁰;
R³ is fluoro;
R⁴ is hydrogen;
R⁵ is hydrogen or optionally substituted C₁₋₆alkyl; wherein said optional substituents are selected from one or more R¹⁴;
R⁶ and R⁷ are independently selected from hydrogen, halo, nitro, cyano, amino, C₁₋₆alkyl, *N*-(C₁₋₆alkyl)amino, *N,N*-(C₁₋₆alkyl)₂amino, *N*-(C₁₋₆alkyl)carbamoyl, C₁₋₆alkoxycarbonyl or heterocyclyl; wherein R⁶ and R⁷ independently of each other may,be optionally substituted on carbon by one or more R¹⁵; and wherein if said heterocyclyl contains an -NH- moiety that nitrogen may be optionally substituted by a group selected from R¹⁶;
   or R⁶ and R⁷ together with the pyrimidine bond to which they are attached form a 6 membered carbocyclic ring or a 5 or 6 membered heterocyclic ring wherein said ring is fused to the pyrimidine of formula (I); wherein the double bonds of the resulting bicyclic ring may be further delocalised across the whole of the bicyclic ring; and wherein said carbocyclic ring or heterocyclic ring may be optionally substituted on carbon by one or more R¹⁷; and wherein if said heterocyclic ring contains an -NH- moiety that nitrogen may be optionally substituted by a group selected from R¹⁸;
R⁸ is selected from halo or carbocyclyl;
R¹⁰ is selected from halo, hydroxy, carboxy, amino, C₁₋₆alkoxy,
   *N,N-*(C₁₋₆alkyl)₂amino, C₁₋₆alkanoylamino, *N*-(C₁₋₆alkyl)carbamoyl,
   NN-(C₁₋₆alkyl)₂carbarnoyl or heterocyclyl; wherein R¹⁰ may be optionally substituted on carbon by one or more R¹⁹ ; and wherein if said heterocyclyl contains an -NH- moiety that nitrogen may be optionally substituted by a group selected from R²⁰;
R¹⁴ is selected from hydroxy;
R¹⁵ is selected from halo, hydroxy, amino, C₁₋₆alkoxy, *N,N*-(C₁₋₆alkyl)₂amino, carbocyclyl or heterocyclyl; wherein R¹⁵ may be optionally substituted on carbon by one or more R¹⁹; and wherein if said heterocyclyl contains an -NH- moiety that nitrogen may be optionally substituted by a group selected from R²⁰;
R¹⁶ is selected from C₁₋₆alkyl;
R¹⁷ is selected from halo, C₁₋₆alkyl or C₁₋₆alkoxy; herein R¹⁷ may be optionally substituted on carbon by one or more R¹⁹;
R¹⁸ is selected from C₁₋₆alkanoyl;
R¹⁹ is selected from halo, hydroxy, C₁₋₆alkoxy or heterocyclyl; and wherein if said heterocyclyl contains an -NH- moiety that nitrogen may be optionally substituted by a group selected from R²⁴;
R²⁰ is selected from C₁₋₆alkyl;
R²⁴ is selected from C₁₋₆alkyl; and
n= 1;
wherein a "carbocyclyl" is a saturated, partially saturated or unsaturated, mono or bicyclic carbon ring that contains 3-12 atoms; wherein a -CH₂- group can optionally be replaced by a -C(O)-;
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I);** of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 6 membered carbocyclic ring" said ring is a partially saturated or unsaturated, mono or bicyclic carbon ring that contains 6 atoms two atoms of which are shared with the pyrimidine ring of formula **(I)**; wherein a -CH₂- group can optionally be replaced by a -C(O)-; or a pharmaceutically acceptable salt thereof

Therefore in a further aspect of the invention there is provided a compound of formula (I) (as depicted herein above) wherein:
A is a direct bond;
Ring C is carbocyclyl or heterocyclyl;
R¹ is selected from hydrogen, C₁₋₆alkyl, C₁₋₆alkoxy, *N,N*-(C₁₋₆alkyl)₂amino, C₁₋₆alkylS(O)ₐ wherein a is 0 or carbocyclyl;
R² is selected from C₁₋₆alkyl; wherein R² may be optionally substituted on carbon by one or more R¹⁰;
R³ is fluoro;
R⁴ is hydrogen;
R⁵ is hydrogen or optionally substituted C₁₋₆alkyl; wherein said optional substituents are selected from one or more R¹⁴;
R⁶ and R⁷ are independently selected from hydrogen, halo, C₁₋₆alkyl, *N*-(C₁₋₆alkyl)amino, *N*-(C₁₋₆alkyl)carbamoyl or C₁₋₆alkoxycarbonyl; wherein R⁶ and R⁷ independently of each other may be optionally substituted on carbon by one or more R¹⁵;
   or R⁶ and R⁷ together with the pyrimidine bond to which they are attached form a 5 or 6 membered carbocyclic ring or heterocyclic ring wherein said ring is fused to the pyrimidine of formula (I); and wherein said carbocyclic ring or heterocyclic ring may be optionally substituted on carbon by one or more R¹⁷;
R¹⁰ is selected from hydroxy, carboxy, C₁₋₆alkoxy, *N,N*-(C₁₋₆alkyl)₂amino or heterocyclyl; wherein R¹⁰ may be optionally substituted on carbon by one or more hydroxy or C₁₋₆alkoxy; and wherein if said heterocyclyl contains an -NH- moiety that nitrogen may be optionally substituted by a group selected from R²⁰;
R¹⁴ is selected from hydroxy;
R¹⁵ is selected from halo, hydroxy, carbocyclyl or heterocyclyl; wherein R¹⁵ may be optionally substituted on carbon by one or more R¹⁹; and wherein if said heterocyclyl contains an -NH- moiety that nitrogen may be optionally substituted by a group selected from R²⁰;
R¹⁷ is selected from halo, C₁₋₆alkyl or C₁₋₆alkoxy; wherein R¹⁷ may be optionally substituted on carbon by one or more R¹⁹; wherein R¹⁹ is selected from halo, C₁₋₆alkoxy or heterocyclyl; wherein if said heterocyclyl contains an -NH- moiety that nitrogen may be optionally substituted by a group selected from R²⁴;
R²⁰ is selected from C₁₋₆alkyl;
R²⁴ is selected from C₁₋₆alkyl; and
n= 1;
wherein a "carbocyclyl" is a saturated, partially saturated or unsaturated, mono or bicyclic carbon ring that contains 3-12 atoms; wherein a -CH₂- group can optionally be replaced by a -C(O)-;
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I)**; wherein a -CH₂- group can optionally be replaced by a -C(O)-; or a pharmaceutically acceptable salt thereof.

Therefore in a further aspect of the invention there is provided a compound of formula **(I)** (as depicted herein above) wherein:
A is a direct bond;
Ring C is phenyl, thienyl, pyridyl, thiazolyl;
R¹ is selected from hydrogen, methyl, ethyl, isopropyl, t-butyl, trifluoromethyl, cyclopropylmethyl, benzyl, methoxy, ethoxy, propoxy, isopropoxy, sec-butoxy, dimethylamino, methylthio or cyclopropyl;
R² is selected from methyl, ethyl, trifluoromethyl, hydroxymethyl, carboxymethyl, aminomethyl, methoxymethyl, morpholinomethyl, 1-hydroxyethyl, 2-hydroxyethyl, 1-carboxyethyl, 2-dimethylaminoethyl, 2-diethylaminoethyl, acetamidomethyl, 2-[*N*-methyl-*N-*(2-methoxyethyl)amino]ethyl, 2-[*N*-methyl-*N*-(2-hydroxyethyl)amino]ethyl, 2-(*N-*methylcarbamoyl)ethyl, 2-[*N*-(2-hydroxyethyl)carbamoyl]ethyl, 2-(*N,N-*dimethylcarbamoyl)ethyl, 2-morpholinoethyl, 2-pyrrolidin-1-ylethyl or 2-(1-methylpiperazin-4-yl)ethyl, 1-methyl-2-hydroxyethyl;
R³ is selected fluoro;
R⁴ is hydrogen;
R⁵ is hydrogen, methyl or 2-hydroxyethyl;
R⁶ and R⁷ are independently selected from hydrogen, fluoro, chloro, bromo, nitro, cyan, amino, methyl, methylamino, ethylamino, propylamino, isopropylamino, dimethylamino, *N*-methyl-*N*-propylamino, *N*-ethylcarbamoyl, methoxycarbonyl, ethoxycarbonyl, butoxycarbonyl, morpholino, pyrrolidinyl or piperazinyl; wherein R⁶ and R⁷ independently of each other may be optionally substituted on carbon by one or more R¹⁵; and wherein said piperazinyl may be optionally substituted on nitrogen by a group selected from R¹⁶;
   or R⁶ and R⁷ together with the pyrimidine to which they are attached form a bicyclic ring selected from quinazolinyl, thieno[3,2-d]pyrimidinyl, thieno[2,3-d]pyrimidinyl, 1*H-*pyrazolo[3,4-d]pyrimidinyl, thieno[3,4-d]pyrimidinyl, pyrido[2,3-d]pyrimidinyl, 5,6,7,8-tetrahydro-pyrido[4,3-d]pyrimidinyl, 5,6,7,8-tetrahydro-pyrido[2,3-*d*]pyrimidinyl or 5,6,7,8-tetrahydro-pyrido[3,4-*d*]pyrimidinyl; and wherein said bicyclic ring may be optionally substituted on carbon by one or more R¹⁷; and wherein said 5,6,7,8-tetrahydro-pyrido[4,3-d]pyrimidinyl, 5,6,7,8-tetrahydro-pyrido[2,3-*d*]pyrimidinyl or 5,6,7,8-tetrahydro-pyrido[3,4-*d*]pyrimidinyl may be optionally substituted on nitrogen by a group selected from R¹⁸;
R¹⁵ is selected from fluoro, hydroxy, amino, ethoxy, dimethylamino, phenyl, pyrrolidinyl, piperazinyl or morpholino; wherein R¹⁵ may be optionally substituted on carbon by one or more R¹⁹; and wherein said piperazinyl may be optionally substituted on nitrogen by a group selected from R²⁰;
R¹⁶ is selected from methyl;
R¹⁷ is selected from fluoro, chloro, methyl, methoxy, ethoxy or propoxy; wherein R¹⁷ may be optionally substituted on carbon by one or more R¹⁹;
R¹⁸ is selected from acetyl;
R¹⁹ is selected from fluoro, hydroxy, methoxy, piperazinyl, pyrrolidinyl or morpholino; and wherein said piperazinyl may be optionally substituted on nitrogen by a group selected from R²⁴;
R²⁰ is selected from methyl;
R²⁴ is selected from methyl;
n = 1;
or a pharmaceutically acceptable salt thereof.

Therefore in a further aspect of the invention there is provided a compound of formula (I) (as depicted herein above) wherein:
A is a direct bond;
Ring C is phenyl or thien-2-yl;
R¹ is selected from hydrogen, methyl, ethyl, t-butyl, methoxy, dimethylamino, methylthio or cyclopropyl;
R² is selected from methyl, ethyl or isopropyl; wherein R² may be optionally substituted on carbon by one or more R¹⁰;
R³ is fluoro;
R⁴ is hydrogen;
R⁵ is hydrogen or 2-hydroxyethyl;
R⁶ and R⁷ are independently selected from hydrogen, fluoro, chloro, bromo, methyl, ethylamino, propylamino, *N*-(ethyl)carbamoyl, methoxycarbonyl, ethoxycarbonyl or butoxycarbonyl; wherein R⁶ and R⁷ independently of each other may be optionally substituted on carbon by one or more R¹⁵;
   or R⁶ and R⁷ together with the pyrimidine to which they are attached form quinazolinyl, thieno[3,2-d]pyrimidinyl, thieno[2,3-d]pyrimidinyl, thieno[3,4-d]pyrimidinyl, 1*H*-pyrazolo[3,4-d]pyrimidinyl or pyrido[2,3-d]pyrimidinyl; and wherein said quinazolinyl, thieno[3,2-d]pyrimidinyl, thieno[2,3-d]pyrimidinyl, thieno[3,4-d]pyrimidinyl, 1*H-*pyrazolo[3,4-d]pyrimidinyl or pyrido[2,3-d]pyrimidinyl, may be optionally substituted on carbon by one or more R¹⁷;
R¹⁰ is selected from hydroxy, carboxy, methoxy, *N*-methyl-*N*-ethylamino, diethylamino, pyrrolidin-1-yl, piperazin-1-yl or morpholino; wherein R¹⁰ may be optionally substituted on carbon by one or more hydroxy or methoxy; and wherein said piperazinyl may be optionally substituted on nitrogen by a group selected from R²⁰;
R¹⁵ is selected from fluoro, hydroxy, phenyl, piperazinyl, pyrrolidinyl or morpholino; wherein R¹⁵ may be optionally substituted on carbon by one or more R¹⁹; and wherein if said piperazinyl contains an -NH- moiety that nitrogen may be optionally substituted by a group selected from R²⁰;
R¹⁷ is selected from fluoro, chloro, methyl, methoxy or ethoxy; wherein R¹⁷ may be optionally substituted on carbon by one or more R¹⁹;
R¹⁹ is selected from fluoro, methoxy, piperazinyl, pyrrolidinyl or morpholino; wherein if said piperazinyl contains an -NH- moiety that nitrogen may be optionally substituted by a group selected from R²⁴;
R²⁰ is selected from methyl;
R²⁴ is selected from methyl;
n= 1;
or a pharmaceutically acceptable salt thereof.

Additional embodiments of the invention are as follows.

In another aspect of the invention, preferred compounds of the invention are any one of the Examples or a pharmaceutically acceptable salt thereof.

In an additional embodiment the present invention provides a compound of formula **(I)**, or a pharmaceutically acceptable salt thereof, for use as a medicament.

In an additional embodiment the present invention provides a compound of formula **(I)**, or a pharmaceutically acceptable salt thereof, for use in the manufacture of a medicament for use in the inhibition of Trk activity.

In an additional embodiment the present invention provides a compound of formula **(I)**, or a pharmaceutically acceptable salt thereof, for use in the manufacture of a medicament for use in the treatment or prophylaxis of cancer.

In an additional embodiment the present invention provides a compound of the formula **(I)**, or a pharmaceutically acceptable salt thereof, for use in the manufacture of a medicament for use in the treatment of cancer in a warm-blooded animal such as man.

In an additional embodiment the present invention provides a compound of the formula **(I)**, or a pharmaceutically acceptable salt thereof, for use in the manufacture of a medicament for use in the treatment or prophylaxis of cancers (solid tumors and leukemia); fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation in a warm-blooded animal such as man.

In an additional embodiment the present invention provides a compound of formula **(I)**, or a pharmaceutically acceptable salt thereof, for use in the manufacture of a medicament for use in the production of an anti-proliferative effect.

In an additional embodiment there is a method of inhibiting Trk activity comprising administering to a host in need of such treatment a therapeutically effective amount of a compound of formula **(I)**, or a pharmaceutically acceptable salt thereof.

In an additional embodiment there is a method for the treatment of cancer comprising administering to a host in need of such treatment a therapeutically effective amount of a compound of formula **(I)**, or a pharmaceutically acceptable salt thereof.

In an additional embodiment there is a method for the treatment or prophylaxis of cancer comprising administering a therapeutically effective amount of a compound of formula **(I)** or a pharmaceutically acceptable salt thereof.

In an additional embodiment there is a method for the treatment or prophylaxis of cancers (solid tumors and leukemia), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation in a warm-blooded animal such as man comprising administering a therapeutically effective amount of a compound of formula **(I)** or a pharmaceutically acceptable salt thereof.

In an additional embodiment there is a method of producing an anti-proliferative effect in a warm-blooded animal, such as man, in need of such treatment which comprises administering to said animal an effective amount of a compound of formula (I), or a pharmaceutically acceptable salt thereof.

In an additional embodiment the present invention provides a pharmaceutical composition comprising a compound of formula **(I)**, or a pharmaceutically acceptable salt thereof, together with at least one pharmaceutically acceptable carrier, diluent or excipient.

In an additional embodiment the present invention provides a pharmaceutical composition comprising a compound of formula **(I)**, or a pharmaceutically acceptable salt thereof, together with at least one pharmaceutically acceptable carrier, diluent or excipient for use in the inhibition of Trk activity.

In an additional embodiment the present invention provides a pharmaceutical composition comprising a compound of formula **(I)**, or a pharmaceutically acceptable salt thereof, together with at least one pharmaceutically acceptable carrier, diluent or excipient for use in the treatment of cancer.

In an additional embodiment the present invention provides a pharmaceutical composition comprising a compound of formula **(I)**, or a pharmaceutically acceptable salt thereof, together with at least one pharmaceutically acceptable carrier, diluent or excipient for use in the treatment or prophylaxis of cancer.

In an additional embodiment the present invention provides a pharmaceutical composition comprising a compound of formula **(I)**, or a pharmaceutically acceptable salt thereof, together with at least one pharmaceutically acceptable carrier, diluent or excipient for use in the treatment or prophylaxis of cancers (solid tumors and leukemia), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

In an additional embodiment the present invention provides a pharmaceutical composition comprising a compound of formula **(I)**, or a pharmaceutically acceptable salt thereof, together with at least one pharmaceutically acceptable carrier, diluent or excipient for use in the production of an anti-proliferative effect in a warm-blooded animal such as man.

In an additional embodiment the present invention provides a compound of formula **(I)**, or a pharmaceutically acceptable salt thereof, for use in the inhibition of Trk activity.

In an additional embodiments the present invention provides a compound of formula **(I)**, or a pharmaceutically acceptable salt thereof, for use in the treatment or prophylaxis of cancer.

In an additional embodiment the present invention provides a compound of the formula **(I)**, or a pharmaceutically acceptable salt thereof, for use in the treatment of cancer in a warm-blooded animal such as man.

In an additional embodiment the present invention provides a compound of the formula **(I)**, or a pharmaceutically acceptable salt thereof, for use in the treatment or prophylaxis of cancers (solid tumors and leukemia), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation in a warm-blooded animal such as man.

In an additional embodiment the present invention provides a compound of formula **(I)**, or a pharmaceutically acceptable salt thereof, for use in the production of an anti-proliferative effect.

Where the inhibition of Trk activity is referred to particularly this refers to the inhibition of TrkB activity.

Where the treatment (or prophylaxis) of cancer is referred to, particularly it refers to the treatment (or prophylaxis) of oesophageal cancer, myeloma, hepatocellular, pancreatic, cervical cancer, ewings tumour, neuroblastoma, kaposis sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, renal cancer, lymphoma, leukaemia, tumours of the central and peripheral nervous system, melanoma, fibrosarcoma and osteosarcoma. More particularly it refers to prostate cancer. In addition, more particularly it refers to SCLC, NSCLC, colorectal cancer, ovarian cancer and / or breast cancer. In a further aspect it refers to hormone refractory prostate cancer.

In an additional embodiment the present invention provides a process for preparing a compound of structural formula **(I)** as claimed in claim 1 or a pharmaceutically acceptable salt thereof which process comprises:

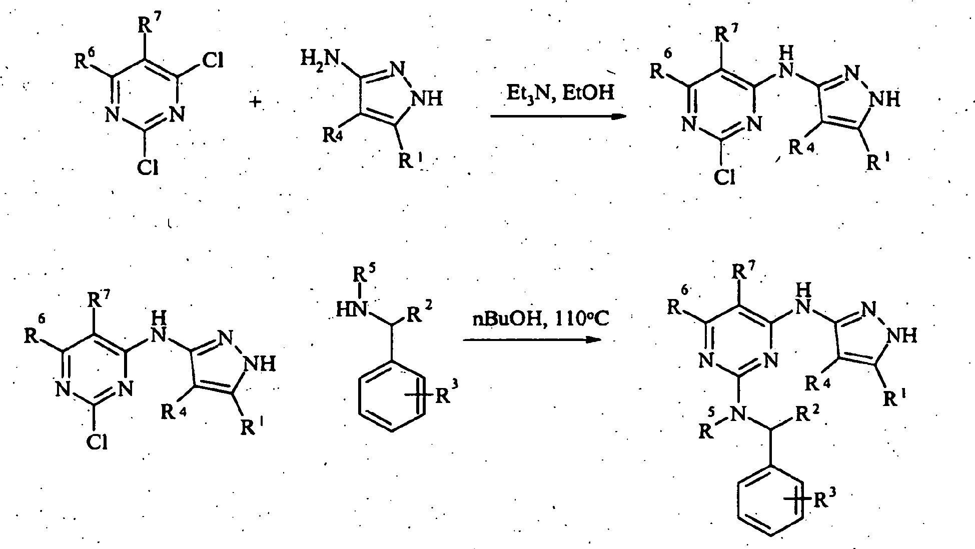

In a further aspect of the present invention provides a process for preparing a compound of formula (I) or a pharmaceutically acceptable salt thereof which process (wherein variable groups are, unless otherwise specified, as defined in formula (I)) comprises of:
*Process a*) reaction of a pyrimidine of formula **(II)**: 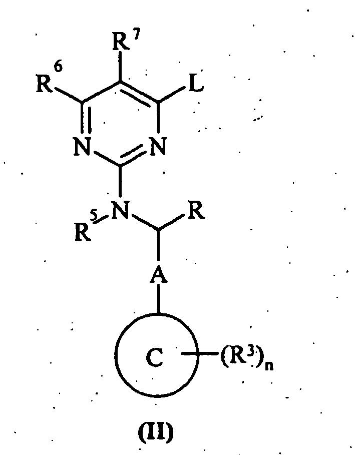 wherein L is a displaceable group; with an pyrazole amine of formula **(III)**: 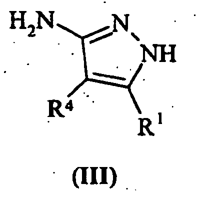 or
*Process b*) reacting a pyrimidine of formula **(IV)**: 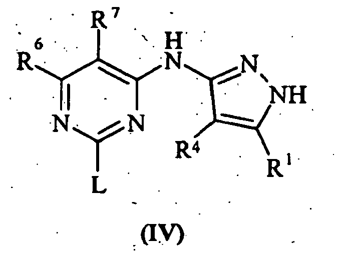 wherein L is a displaceable group; with a compound of formula **(V)**: 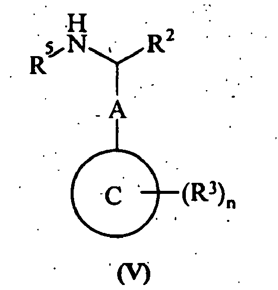
*Process c*) reacting a compound of formula **(VI)**: 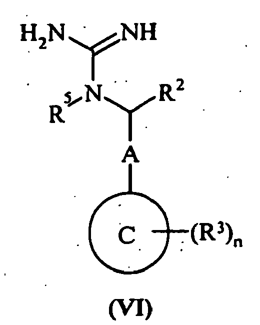 with a compound of formula **(VII)**: 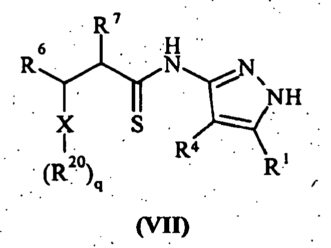 wherein X is an oxygen atom and q is 1; or X is a nitrogen atom and q is 2; and wherein each R²⁰ independently represents a C₁₋₆alkyl group; or
*Process d*) reacting a compound of formula **(VIII):** 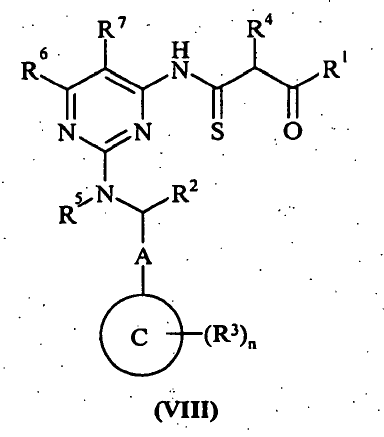 with hydrazine; or
   and thereafter if necessary
   i) converting a compound of the formula **(I)** into another compound of the formula **(I);**
   ii) removing any protecting groups;
   iii) forming a pharmaceutically acceptable salt.

L is a displaceable group, suitable values for L are for example, a halo or sulphonyloxy group, for example a chloro, bromo, methanesulphonyloxy or toluene-4-sulphonyloxy group,

Specific reaction conditions for the above reactions are as follows.
*Process a*) Pyrimidines of formula **(II)** and pyrazole amine of formula **(III)** may be reacted together:
a) in the presence of a suitable solvent for example a ketone such as acetone or an alcohol such as ethanol or butanol or an aromatic hydrocarbon such as toluene or *N* methyl pyrrolid-2-one, optionally in the presence of a suitable acid for example an inorganic acid such as hydrochloric acid or sulphuric acid, or an organic acid such as acetic acid or formic acid (or a suitable Lewis acid) and at a temperature in the range from 0°C to reflux, particularly reflux; or
b) under standard Buchwald conditions (for example see J. Am. Chem. Soc., 118, 7215; J. Am. Chem. Soc., 119, 8451; J. Org. Chem., 62, 1568 and 6066) for example in the presence of palladium acetate, in a suitable solvent for example an aromatic solvent such as toluene, benzene or xylene, with a suitable base for example an inorganic base such as caesium carbonate or an organic base such as potassium-*t*-butoxide, in the presence of a suitable ligand such as 2,2'-bis(diphenylphosphino)-1,1'-binaphthyl and at a temperature in the range from 25 to 80°C.

Pyrimidines of the formula (II) may be prepared according to *Scheme 1:*

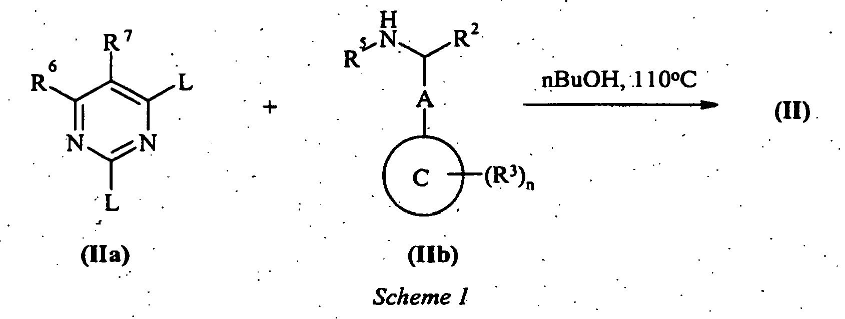

Pyrazole amines of formula **(III)** and compound of formula **(IIa)** and **(IIb)** are commercially available compounds, or they are known in the literature, or they are prepared by standard processes known in the art.
*Process b*) Compounds of formula **(IV)** and formula **(V)** may be reacted together under the same conditions as outlined in *Process a*).

Compounds of the formula **(IV)** may be prepared according to *Scheme 2*:

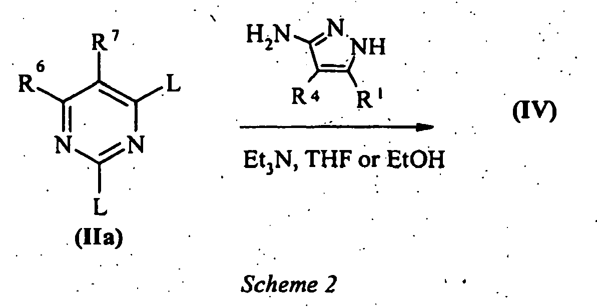

Compounds of the formula **(V)** are commercially available compounds, or they are known in the literature, or they are prepared by standard processes known in the art.
*Process c*) may conveniently be carried out in a suitable solvent such as
*N*-methylpyrrolidinone or butanol at a temperature in the range from 100-200°C, in particular in the range from 150-170°C. The reaction is preferably conducted in the presence of a suitable base such as, for example, sodium methoxide or potassium carbonate.

Compounds of the formula **(VI)** may be prepared according to *Scheme 3*:

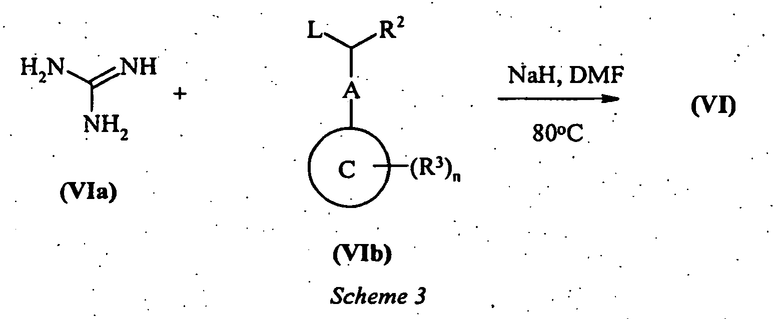

Compounds of the formula **(VII)** may be prepared according to *Scheme 4*: 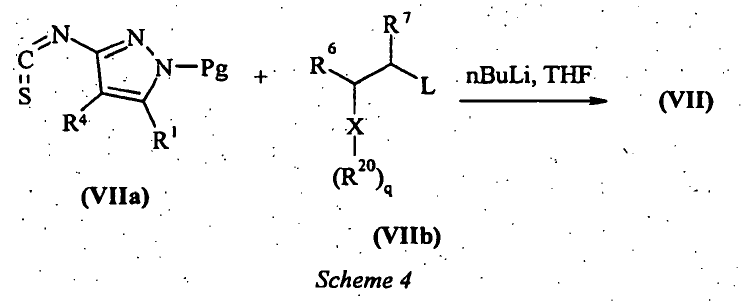 wherein Pg is a suitable nitrogen protecting group. Suitable values for Pg are defined below.

Compounds of the formula **(VIa), (VIb), (VIIa)** and **(VIIb)** are commercially available compounds, or they are known in the literature, or they are prepared by standard processes known in the art.
*Process d*) may be carried out in a suitable solvent, for example, an alcohol such as ethanol or butanol at a temperature in the range from 50-120°C, in particular in the range from 70-100°C.

Compounds of the formula **(VIII)** may be prepared according to *Scheme 5*:

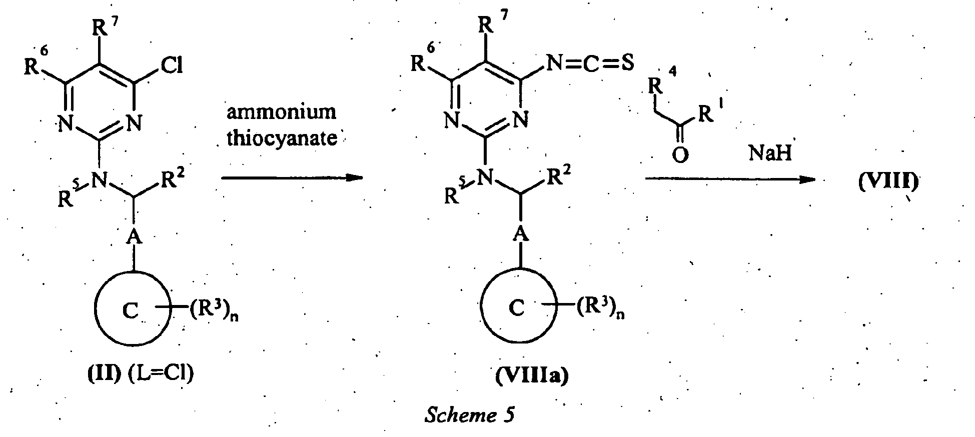

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *t-*butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t-*butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *t-*butyl group which may be removed, for example, by treatment with an acid, for example an organic acid such as trifluoroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

### Definitions

In this specification the term "alkyl" includes both straight and branched chain alkyl groups but references to individual alkyl groups such as "propyl" are specific for the straight chain version only. For example, "C₁₋₆alkyl" and "C₁₋₄alkyl" include methyl, ethyl, propyl, isopropyl and *t-*butyl. However, references to individual alkyl groups such as 'propyl' are specific for the straight-chained version only and references to individual branched chain alkyl groups such as 'isopropyl' are specific for the branched-chain version only. A similar convection applies to other radicals. The term "halo," refers to fluoro, chloro, bromo and iodo.

Where optional substituents are chosen from "one or more" groups it is to be understood that this definition includes all substituents being chosen from one of the specified groups or the substituents being chosen from two or more of the specified groups.

A "heterocyclyl" is a saturated, partially saturated or unsaturated, mono or bicyclic ring containing 4-12 atoms of which at least one atom is chosen from nitrogen; sulphur or oxygen, which may, unless otherwise specified, be carbon or nitrogen linked, wherein a -CH₂- group can optionally be replaced by a -C(O)-, and a ring sulphur atom may be optionally oxidised to form the S-oxides. Examples and suitable values of the term "heterocyclyl" are morpholino, piperidyl, pyridyl, pyranyl, pyrrolyl, isothiazolyl, indolyl, quinolyl, thienyl, 1,3-benzodioxolyl, thiadiazolyl, piperazinyl; thiazolidinyl, pyrrolidinyl, thiomorpholino, pyrrolinyl, homopiperazinyl, 3,5-dioxapiperidinyl, tetrahydropyranyl, imidazolyl, pyrimidyl, pyrazinyl, pyridazinyl, isoxazolyl, *N*-methylpyrrolyl, 4-pyridone, 1-isoquinolone, 2-pyrrolidone, 4-thiazolidone, pyridine-*N*-oxide and quinoline-*N*-oxide. Further examples and suitable values of the term "heterocyclyl" are morpholino, piperazinyl and pyrrolidinyl. In one aspect of the invention a "heterocyclyl" is a saturated, partially saturated or unsaturated, mono or bicyclic ring containing 5 or 6 atoms of which at least one atom is chosen from nitrogen, sulphur or oxygen, it may, unless otherwise specified, be carbon or nitrogen linked, a -CH₂- group can optionally be replaced by a -C(O)-and a ring sulphur atom may be optionally oxidised to form the S-oxides.

A "carbocyclyl" is a saturated, partially saturated or unsaturated, mono or bicyclic carbon ring that contains 3-12 atoms; wherein a -CH₂- group can optionally be replaced by a -C(O)-. Particularly "carbocyclyl" is a monocyclic ring containing 5 or 6 atoms or a bicyclic ring containing 9 or 10 atoms. Suitable values for "carbocyclyl" include cyclopropyl, cyclobutyl, 1-oxocyclopentyl, cyclopentyl, cyclopentenyl, cyclohexyl, cyclohexenyl, phenyl, naphthyl, tetralinyl, indanyl or 1-oxoindanyl.

Where "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I)**; of which at least one atom is chosen from nitrogen, sulphur or oxygen; therein a -CH₂- group can optionally be replaced by a -C(O)-, and a ring sulphur atom may be optionally oxidized to form the S-oxides. Said ring is fused to the pyrimidine ring of formula **(I)** to make a 9 or 10 membered bicyclic ring. Suitable values for "R⁶ and R⁷ together with the bond to which they are attached form a 5 or 6 membered heterocyclic ring wherein said ring is fused to the pyrimidine of formula **(I)**" are pteridinyl, purinyl, thieno[3,2-d]pyrimidinyl, thieno[2,3-d]pyrimidinyl, thieno[3,4-d]pyrimidinyl, 1*H*-pyrazolo[3,4-d]pyrimidinyl or pyrido[2,3-d]pyrimidinyl. Further suitable values for "R⁶ and R⁷ together with the bond to which they are attached form a 5 or 6 membered heterocyclic ring wherein said ring is fused to the pyrimidine of formula **(I)**" are thieno[3,2-d]pyrimidinyl, thieno[2,3-d]pyrimidinyl, thieno[3,4-d]pyrimidinyl, 1*H*-pyrazolo[3,4-d]pyrimidinyl or pyrido[2,3-d]pyrimidinyl. Additional suitable values for "R⁶ and R⁷ together with the bond to which they are attached form a 5 or 6 membered heterocyclic ring wherein said ring is fused to the pyrimidine of formula **(I)**" are thieno[3,2-d]pyrimidinyl, thieno[2,3-d]pyrimidinyl, 1*H-*pyrazolo[3,4-d]pyrimidinyl, thieno[3,4-d]pyrimidinyl, pyrido[2,3-d]pyrimidinyl, 5,6,7,8-tetrahydro-pyrido[4,3-d]pyrimidinyl, 5,6,7,8-tetrahydro-pyrido[2,3-d]pyrimidinyl and 5,6,7,8-tetrahydro-pyrido[3,4-d]pyrimidinyl.

Where "R⁶ and R⁷ together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I)**; wherein a -CH₂- group can optionally be replaced by a -C(O)-. Said ring is fused to the pyrimidine ring of formula **(I)** to make a 9 or 10 membered bicyclic ring. Suitable values for "R⁶ and R⁷ together with the bond to which they are attached form a 5 or 6 membered carbocyclic ring wherein said ring is fused to the pyrimidine of formula **(I)**" are quinazolinyl.

The term "Cₘ₋ₙ" or "Cₘ₋ₙ" group" used alone or as a prefix, refers to any group having m to n carbon atoms.

The term "optionally substituted" refers to either groups, structures, or molecules that are substituted and those that are not substituted.

An example of "C₁₋₆alkanoyloxy" is acetoxy. Examples of "C₁₋₆alkoxycarbonyl" include C₁₋₄alkoxycarbonyl, methoxycarbonyl, ethoxycarbonyl, *n*- and *t*-butoxycarbonyl. Examples of "C₁₋₆alkoxy" include C₁₋₄alkoxy, C₁₋₃alkoxy, methoxy, ethoxy and propoxy. Examples of "C₁₋₆alkoxyimino" include C₁₋₄alkoxyimino, C₁₋₃alkoxyimino, methoxyimino, ethoxyimino and propoxyimino. Examples of "C₁₋₆alkanoylamino" include formamido, acetamido and propionylamino. Examples of "C₁₋₆alkylS(O)ₐ wherein a is 0 to 2" include C₁₋₄alkylsulphonyl, methylthio, ethylthio, methylsulphinyl, ethylsulphinyl, mesyl and ethylsulphonyl. Examples of "C₁₋₆alkylthio" include methylthio and ethylthio. Examples of "C₁₋₆alkylsulphonylamino" include methylsulphonylamino and ethylsulphsulphonylamino. Examples of "C₁₋₆alkanoyl" include C₁₋₄alkanoyl, propionyl and acetyl. Examples of "*N*-(C₁₋₆alkyl)amino" include methylamino and ethylamino. Examples of "*N*,*N*-(C₁₋₆alkyl)₂amino" include di-*N*-methylamino, di-(*N*-ethyl)amino and *N*-ethyl-*N*-methylamino. Examples of "C₂₋₆alkenyl" are vinyl, allyl and 1-propenyl. Examples of "C₂₋₆alkynyl" are ethynyl, 1-propynyl and 2-propynyl. Examples of "*N*-(C₁₋₆alkyl)sulphamoyl" are *N*-(methyl)sulphamoyl and *N*-(ethyl)sulphamoyl. Examples of "*N*-(C₁₋₆alkyl)₂sulphamoyl" are *N*,*N*-(dimethyl)sulphamoyl and *N*-(methyl)-*N*-(ethyl)sulphamoyl. Examples of "*N*-(C₁₋₆alkyl)carbamoyl" are *N*-(C₁₋₄alkyl)carbamoyl, methylaminocarbonyl and ethylaminocarbonyl. Examples of "*N*,*N*-(C₁₋₆alkyl)₂carbamoyl" are *N*,*N*-(C₁₋₄alkyl)₂carbamoyl, dimethylaminocarbonyl and methylethylaminocarbonyl.

"RT" or "rt" means room temperature.

A first ring group being "fused" with a second ring group means the first ring and the second ring share at least two atoms there between.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

It should be noted that the pyrazoles claimed in this invention are capable to exist in different resonance structures and thus the pyrazoles claimed herein include all possible resonance structures, for example optical isomers, diastereoisomers and geometric isomers and all tautomeric forms of the compounds of the formula **(I)**.

It is also to be understood that certain compounds of the formula **(I)** can exist in solvated as well as unsolvated forms such as, for example, hydrated forms. It is to be understood that the invention encompasses all such solvated forms.

### Formulations

Compounds of the present invention may be administered orally, parenteral, buccal, vaginal, rectal, inhalation, insufflation, sublingually, intramuscularly, subcutaneously, topically, intranasally, intraperitoneally, intrathoracially, intravenously, epidurally, intrathecally, intracerebroventricularly and by injection into the joints.

The dosage will depend on the route of administration, the severity of the disease, age and weight of the patient and other factors normally considered by the attending physician, when determining the individual regimen and dosage level as the most appropriate for a particular patient.

An effective amount of a compound of the present invention for use in therapy of cancer is an amount sufficient to symptomatically relieve in a warm-blooded animal, particularly a human the symptoms of cancer, to slow the progression of cancer, or to reduce in patients with symptoms of cancer the risk of getting worse.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substance, which may also act as diluents, flavoring agents, solubilizers, lubricants, suspending agents, binders, or tablet disintegrating agents; it can also be an encapsulating material.

In powders, the carrier is a finely divided solid, which is in a mixture with the finely divided active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture is then poured into convenient sized molds and allowed to cool and solidify.

Suitable carriers include magnesium carbonate, magnesium stearate, talc, lactose, sugar, pectin, dextrin, starch, tragacanth, methyl cellulose, sodium carboxymethyl cellulose, a low-melting wax, cocoa butter, and the like.
Some of the compounds of the present invention are capable of forming salts with various inorganic and organic acids and bases and such salts are also within the scope of this invention. Examples of such acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Base salts include ammonium salts, alkali metal salts such as sodium, lithium and potassium salts, alkaline earth metal salts such as aluminum, calcium and magnesium salts, salts with organic bases such as dicyclohexylamine salts, N-methyl-D-glucamine, and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates like dimethyl, diethyl, dibutyl; diamyl sulfates; long chain halides such as decyl, lauryl, myristyl and stearyl halides; aralkyl halides like benzyl bromide and others. Non-toxic physiologically-acceptable salts are preferred, although other salts are also useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

In order to use a compound of the formula **(I)** or a pharmaceutically acceptable salt thereof for the therapeutic treatment (including prophylactic treatment) of mammals including humans, it is normally formulated in accordance with standard pharmaceutical practice as a pharmaceutical composition.

In addition to the compounds of the present invention, the pharmaceutical composition of this invention may also contain, or be co-administered (simultaneously or sequentially) with, one or more pharmacological agents of value in treating one or more disease conditions referred to herein.

The term composition is intended to include the formulation of the active component or a pharmaceutically acceptable salt with a pharmaceutically acceptable carrier. For example this invention may be formulated by means known in the art into the form of, for example, tablets, capsules, aqueous or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aqueous or oily solutions or suspensions or sterile emulsions.

Liquid form compositions include solutions, suspensions, and emulsions. Sterile water or water-propylene glycol solutions of the active compounds may be mentioned as an example of liquid preparations suitable for parenteral administration. Liquid compositions can also be formulated in solution in aqueous polyethylene glycol solution. Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

The pharmaceutical compositions can be in unit dosage form. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms.

### Combinations

The anti-cancer treatment defined herein may be applied as a sole therapy or may involve, in addition to the compound of the invention, conventional surgery or radiotherapy or chemotherapy. Such chemotherapy may include one or more of the following categories of anti-tumour agents:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as
   N-(3-chloro-4-fluorophenyl)-7-methoxy-6-(3-morpholinopropoxy)quinazolin-4-amine (gefitinib, AZD 1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention, or pharmaceutically acceptable salts thereof, within the dosage range described hereinbefore and the other pharmaceutically-active agent within its approved dosage range.

### Synthesis

The compounds, or pharmaceutically acceptable salts thereof, of the present invention can be prepared in a number of ways well known to one skilled in the art of organic synthesis. The compounds, or pharmaceutically acceptable salts thereof, of the present invention can be synthesized using the methods described below, together with synthetic methods known in the art of synthetic organic chemistry, or variations thereon as appreciated by those skilled in the art. Such methods include, but are not limited to, those described below. All references cited herein are hereby incorporated in their entirety by reference.

The novel compounds, or pharmaceutically acceptable salts thereof, of this invention may be prepared using the reactions and techniques described herein. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents, which are compatible with the reaction conditions, will be readily apparent to one skilled in the art and alternate methods must then be used.

The invention will now be further described with reference to the following illustrative examples in which, unless stated otherwise:
(i) temperatures are given in degrees Celsius (°C); operations are carried out at room temperature or ambient temperature, that is, in a range of 18-25 °C;
(ii) organic solutions were dried over anhydrous sodium sulfate; evaporation of organic solvent was carried out using a rotary evaporator under reduced pressure (4.5 - 30 mmHg) with a bath temperature of up to 60 °C;
(iii) chromatography means flash chromatography on silica gel; thin layer chromatography (TLC) was carried out on silica gel plates;
(iv) in general, the course of reactions was followed by TLC or liquid chromatography/mass spectroscopy (LC/MS) and reaction times are given for illustration only;
(v) final products have satisfactory proton nuclear magnetic resonance (NMR) spectra and/or mass spectra data;
(vi) yields are given for illustration only and are not necessarily those which can be obtained by diligent process development; preparations were repeated if more material was required;
(vii) when given, NMR data is in the form of delta values for major diagnostic protons, given in part per million (ppm) relative to tetramethylsilane (TMS) as an internal standard, determined at 300 MHz in d6-DMSO unless otherwise stated;
(viii) chemical symbols have their usual meanings;
(ix) solvent ratio was given in volume:volume (v/v) terms.
(x) Purification of the compounds were carried out using one or more of the following methods:
a) flash chromatography on regular silica gel;
b) flash chromatography on silica gel using Isco Combiflash® separation system: RediSep normal phase flash column, flow rate, 30-40 ml/min;
c) Gilson semiprep HPLC separation system: YMC pack ODS-AQ column, 100x20 mm, S 5µm 12 nm, water (0.1 % trifluoroacetic acid) and acetonitrile (0.1 % trifluoroacetic acid) as solvents, 20 min run; and

(xvi) the following abbreviations have been used:

| | |
|---|---|
| DMF | dimethylformamide; |
| EtOAc | ethyl acetate; |
| ether | diethyl ether; |
| EtOH | ethanol; |
| THF | tetrahydrofuran; |
| MeOH | methanol; and |
| DCM | dichloromethane. |

### Example 1

### 5-Chloro-N⁴-(5-cyclopropyl-1H-pyrazol-3-yl)-N²-(1-phenylethyl)pyrimidine-2,4-diamine R

A mixture of 1-phenylethylamine (73 µL, 0.56 mmol), 2,5-dichloro-4-(5-cyclopropyl-1*H*-pyrazole-3-ylamino)pyrimidine (Method 1, 76 mg, 0.28 mmol) in 1-butanol (1.0 ml) was heated at 110°C for 18 hrs. The solvent was removed and EtOAc was added. The solution was washed with water and was concentrated. Semi-prep HPLC (Gilson system) provided product as a solid (91 mg, 92 %). ¹H NMR (CDCl₃): 0.72 (m, 2 H), 0.96 (m, 2 H), 1.56 (d, 3 H), 1.85 (m, 1 H), 5.08 (m, 1 H), 5.41 (br s, 1 H), 6.08 (br s, 1 H), 7.22-7.41 (m, 5 H), 7.92 (s, 1 H).

### Example 2-127

Following a similar procedure to Example 1, the following compounds were synthesized via reaction of a suitable pyrimidine (method of production of which is also listed) and a suitable amine.

| **Ex.** | **Compound** | **¹H NMR** | **SM** |
|---|---|---|---|
| **2** | 5-Bromo-N⁴-(3-ethyl-1H-pyrazol-5-yl)-N²-(1-phenylethyl) pyrimidine-2,4-diamine | 1.19 (t, J = 9 Hz, 3 H,), 1.45 (d, J = 9 Hz, 3 H), 2.59 (q, J = 9 Hz, 2 H), 4.99 (t, J = 9 Hz, 1 H), 6.04 (s, 1 H), 7.30 (m, 5 H), 8.18 (1 br s, 1H), 8.48 (s, 1 H), 9.36 (s, 1 | Method 2 |
| **3** | N⁴-(3-tert-Butyl-1H-pyrazol-5-yl)-5-chloro-N²-(1-phenylethyl)pyrimidine-2,4-diamine | 1.27 (s, 9 H), 1.48 (d, J = 6 Hz, 3 H), 5.04 (t, J = 6 Hz, 1 H), 6.25 (s, 1 H), 7.29 (m, 5 H), 8.18 (s, 1 H), 8.77 (s, 1 H), 10.02 (s, 1 H) | Method 3 |
| **4** | N⁴-(3-Cyclopropyl-1H-pyrazol-5-yl)-N²-(1-phenyl ethyl)-5-(trifluoromethyl) pyrimidine-2,4-diamine | 0.66 (m, 2 H), 0.94 (m, 2 H), 1.44 (d, J = 9 Hz, 3 H), 1.90 (m, 1 H), 4.96 (m, 1 H), 5.95 (s, 1 H), 7.28 (m, 5 H), 8.28 (s, 1 H), 8.53 (s, 1 H), 8.87 (s, 1 H) | Method 4 |
| **5** | 5-Bromo-N⁴-(3-cyclopropyl-1H-pyrazol-5-yl)-N²-[(1S)-1-(4-fluorophenyl)ethyl] pyrimidine-2,4-diamine | 0.66 (s, 2 H), 0.92 (m, 2 H), 1.44 (d, 3 H), 1.88 (m, 1 H), 4.95 (m, 1 H), 5.95 (s, 1 H), 7.12 (m, 2 H), 7.31 (m, 2 H), 8.14 (s, 1 H), 8.27 (s, 1 H), 8.92 (br s, 1 H), 9.23 (br s, 1 H) | Method 5 |
| **6** | 5-Bromo-N⁴-(3-cyclopropyl-1H-pyrazol-5-yl)-N²-[(1S)-1-phenylpropyl]pyrimidine-2,4-diamine | 0.69 (s, 2 H), 0.85-1.00 (m, 5 H), 1.78-1.91 (m, 3 H), 4.72 (m, 1 H), 6.03 (s, 1 H), 7.24-7.31 (m, 5 H), 8.02 (br s, 1 H), 8.14 (s, 1 H), 8.24 (br s, 1 H), 9.29 (br s, 1 H) | Method 5 |
| **7** | 5-Bromo-N⁴-(3-cyclopropyl-1H-pyrazol-5-yl)-N²⁻[(1S)-1-(4-nitrophenyl)ethyl] pyrimidine-2,4-diamine | 0.65 (m, 2 H), 0.94 (m, 2 H), 1.49 (d, J = 9 Hz, 3 H), 1.89 (m, 1 H), 5.01 (m, 1 H), 5.82 (s, 1 H), 6.27 (s, 1 H), 7.63 (m, 2 H), 8.16 (m, 3 H), 8.41 (br s, 1 H), 9.14 (br s, 1 H) | Method 5 |
| **8** | (2R)-2-({5-Bromo-4-[(3-cyclopropyl-1H-pyrazol-5-yl)amino]pyrimidin-2-yl}amino)-2-phenylethanol | 0.69 (m, 2 H), 0.95 (m, 2 H), 1.90 (m, 1 H), 3.68 (d, 2 H), 4.93 (t, 1 H), 5.95 (s, 1 H), 7.30 (m, 5 H), 8.22 (s, 1 H), 8.40 (br s, 1 H), 9.45 (br s, 1 H) | Method 5 |
| **9** R | 5-Bromo-N⁴-(5-cyclopropyl-1H-pyrazol-3-yl)-N²-(1-phenylethyl)pyrimidine-2,4-diamine | (CDCl₃): 0.71 (m, 2 H), 0.95 (m, 2 H), 1.56 (d, 3 H), 1.85 (m, 1 H), 5.08 (m, 1 H), 5.63 (br s, 1 H), 6.05 (br s, 1 H), 7.22-7.41 (m, 5 H), 7.99 (s, 1 H). | Method 5 |
| **10** R | 5-Chloro-N⁴-(5-cyclopropyl-1H-pyrazol-3-yl)-N²-(1-phenylpropyl)pyrimidine-2,4-diamine | (CDCl₃): 0.89 (m, 2 H), 1.00 (t, 3 H), 1.17 (m, 2 H), 2.00 (m, 3 H), 4.85 (dt, 1 H), 6.34 (s, 1 H), 7.28-7.39 (m, 5 H), 7.86 (s, 1 H), 8.88 (s, 1 H), 10.10 (d, 1 H) | Method 1 |
| **11** R | 5-Chloro-N⁴-(5-cyclopropyl-1H-pyrazol-3-yl)-N²-[(1S)-1-phenylethyl]pyrimidine-2,4-diamine | (CDCl₃): 0.72 (m, 2 H), 0.98 (m, 2 H), 1.58 (d, 3 H), 1.86 (m, 1 H), 5.07 (m, 1 H), 6.08 (br s, 1 H), 7.23-7.41 (m, 5 H), 7.53 (br s, 1 H), 7.88 (s, 1 H) | Method 1 |
| **12** R | 5-Chloro-N⁴-(5-cyclopropyl-1H-pyrazol-3-yl)-N²-[(1R)-1-phenylethyl]pyrimidine-2,4-diamine | (CDCl₃): 0.72 (m, 2 H), 0.99 (m, 2 H), 1.51 (d, 3 H), 1.93 (m, 1 H), 5.04 (m, 1 H), 6.04 (br s, 1 H), 7.26-7.50 (m, 5 H), 8.19 (s, 1 H), 7.53 (br s, 1 H), 8.65 (br s, 1 H), 9.95 (br s, 1 H) | Method 1 |
| **13** R | 5-Bromo-N⁴-(5-cyclopropyl-1H-pyrazol-3-yl)-14²-(1-phenylpropyl)pyrimidine-2,4-diamine | (CDCl₃): 0.75 (m, 2 H), 0.90 (t, 3 H), 0.99 (m, 2 H), 1.84 (m, 2 H), 1.96 (m, 1 H), 4.77 (m, 1 H), 6.08 (s, 1 H), 7.29-7.39 (m, 5 H), 8.25 (s, 1 H), 8.60 (br s, 1 H), 9.48 (br s, 1 H) | Method 5 |
| **14** R | 5-Bromo-N⁴-(5-cyclopropyl-1H-pyrazol-3-yl)-N²-[(1S)-1-phenylethyl]pyrimidine-2,4-diamine | 0.66 (m, 1 H), 0.75 (m, 1 H), 0.87 (m, 1 H), 0.99 (m, 1 H), 1.46 (d, 3 H), 1.92 (m, 1 H), 5.04 (m, 1 H), 5.88 (br s, 1 H), 7.20-7.44 (m, 5 H), 7.95 (s, 1 H), 8.01 (s, 1 H), 12.14 (s, 1 H) | Method 5 |
| **15** | N⁴-(5-tert-Butyl-1H-pyrazol-3-yl)-5-chloro-N²-[(1S)-1-(4-fluorophenyl)ethyl]pyrimidine-2,4-diamine | 1.26 (s, 9 H), 1.46 (d, 3 H), 5.01 (m, 1 H), 6.22 (br s, 1 H), 7.11 (m, 2 H), 7.32 (m, 2 H), 8.07 (s, 1 H) | Method 3 |
| **16** | 5-Bromo-N⁴-(5-tert-butyl-1H-pyrazol-3-yl)-N²-[(1S)-1-(4-fluorophenyl)ethyl]pyrimidine-2,4-diamine | 1.26 (s, 9 H), 1.46 (d, 3 H), 4.99 (m, 1 H), 6.20 (br s, 1 H), 7.10 (m, 2 H), 7.28 (m, 2 H), 8.17 (s, 1 H), 8.38 (br s, 1 H), 9.42 (br s, 1 H) | Method 6 |
| **17** | 5-Bromo-N⁴-(5-cyclopropyl-1H-pyrazol-3-yl)-N²-[(1S)-1-(4-fluorophenyl)ethyl]-6-methylpyrimidine-2,4-diamine | 0.64 (m, 2 H), 0.96 (m, 2 H), 1.47 (d, 3 H), 1.90 (m, 1 H), 2.39 (s, 3 H), 4.94 (m, 1 H), 5.92 (s, 1 H), 7.13 (m, 2 H), 7.29 (m, 2 H), 8.37 (br s, 1 H), 9.63 (br s, 1 H) | Method 7 |
| **18** R | (2R)-2-({5-Bromo-4-[(5-cyclopropyl-1H-pyrazol-3-yl)amino]-6-methylpyrimidin-2-yl} amino)-2-phenylethanol | (CD₃OD): 0.74 (m, 2 H), 1.05 (m, 2 H), 1.93 (m, 1 H), 3.84 (m, 2 H), 5.05 (m, 1 H), 5.99 (br s, 1 H), 7.33 (m, 5 H) | Method 7 |
| **19** | N⁴-(3-Cyclopropyl-1H-pyrazol-5-yl)-N²-(1-phenylethyl) pyrimidine-2,4-diamine | 0.6 (m, 2 H), 0.9 (m, 2 H), 1.5 (m, 3 H), 1.9 (m, 1 H), 5.1 (m, 1 H), 6.1 (m, 1 H), 6.3 (m, 1 H), 7.3-7.4 (m, 5 H), 7.6 (m, 1 H), 8.7 (s, 1 H) | Method 8 |
| **20** | N⁴-(3-Cyclopropyl-1H-pyrazol-5-yl)-5-methyl-N²-(1-phenylethyl)pyrimidine-2,4-diamine | 0.6 (m, 2 H), 0.9 (m, 2 H), 1.5 (m, 3 H), 1.9 (m, 1 H), 2.0 (s, 3 H), 5.1 (m, 1 H), 6.1 (m, 1 H), 6.3 (m, 1 H), 7.3-7.4 (m, 5 H), 7.7 (m, 1 H), 8.6 (s, 1 H), 10.1 (s, 1 H) | Method 9 |
| **21** | (2S)-2-({5-Biomo-4-[(3-cyclopropyl-1H-pyrazol-5-yl)amino]pyrimidin-2-yl}amino)-2-phenylethanol | 0.9 (m, 2 H), 1.2 (m, 2 H), 2.0 (m, 1 H), 4.1 (m, 2 H), 4.2 (m, 1 H), 5.3 (m, 1 H), 6.2 (m, 1 H), 7.3-7.4 (m, 5 H), 8.1 (m, 1 H), 8.7 (s, 1 H), 10.1 (m, 1 H) | Method 5 |
| **22** | 5-Chloro-N⁴-(3-cyclopropyl-1H-pyrazol-5-yl)-N²-[(1S)-1-(4-fluorophenyl)ethyl] pyrimidine-2,4-diamine | 0.6 - 0.8 (m, 2 H), 0.9-1.2 (m, 2 H),1.6 (m, 3 H), 2.0 (m, 1 H), 5.1 (m, 1 H), 6.4 (m, 1 H), 7.0-7.4 (m, 4 H), 7.7 (m, 1 H), 8.2 (s, 1 H), 8.4 (m, 1 H), 10.4 (m, 1 H) | Method 1 |
| **23¹** | Butyl 6-[(3-cyclopropyl-1H-pyrazol-5-yl)amino]-2-{[1-(4-fluorophenyl)-2-hydroxyethyl] amino}pyrimidine-4-Carboxylate | 0.69 (m, 2 H), 0.95 (m, 5 H), 1.41 (m, 2 H), 1.67 (m, 2 H), 1.87 (m, 1 H), 3.69 (m, 2 H), 4.33 (m, 2 H), 5.06 (m, 1 H), 6.02 (s, 1 H), 7.17 (m, 2 H), 7.41 (m, 2 H), 8.29 (br s, 1 H), 12.33 (br s, 1 H) | Method 10 |
| **24** | (2R)-2-({S-Chloro-4-[(3-cyclopropyl-1H-pyrazol-5-yl)amino]pyrimidin-2-yl}amino)-2-(4-fluorophenyl)ethanol | 0.62-0.71 (m, 2 H), 0.82-0.94 (m, 2 H), 1.88 (m, 1 H), 3.60 (m, 2 H), 4.88 - 4.93 (m, 2 H), 5.79 (s, 1 H), 6.07-6.47 (m, 1 H), 7.10 (m, 2 H), 7.35 (m, 2 H), 7.89 (s, 1 H), 8.36 (s, 1H), 12.08 (s, 1 H) | Method 1, and Method 104 |
| **25** | (25)-2-({5-Chloro-4-[(3-cyclopropyl-1H-pyrazol-5-yl)amino]pyrimidin-2-yl}amino)-2-(4-fluorophenyl)ethanol | 0.69 (m, 2 H), 0.85-0.94 (m, 2 H), 1.88 (m, 1 H), 3.60 (m, 2 H), 4.88 - 4.93 (m, 2 H), 5.77 (s, 1 H), 6.01-6.44 (m, 1 H), 7.10 (m, 2 H), 7.35 (m, 2 H), 7.89 (s, 1 H), 8.36 (s, 1 H), 12.08 (s, 1 H) | Method 1, and Method 105 |
| **26** | (2R)-2-({5-Bromo-4-[(3-cyclopropyl-1H-pyrazol-5-yl)amino]pyrimidin-2-yl}amino)-2-(4-fluorophenyl)ethanol | 0.68 (m, 2 H), 0.98 (m, 2 H), 1.87 (m, 1 H), 3.60 (m, 2 H), 4.90 (m, 1 H), 5.99 (br s, 1 H), 7.10 (m, 2 H), 7.36 (m, 2 H), 7.98 (s, 1 H), 8.19 (br s, 1 H), 12.08 (br s, 1H) | Method 5, and Method 104 |
| **27** | (2S)-2-({5-Bromo-4-[(3-cyclopropyl-1H-pyrazol-5-yl)amino]pyrimidin-2-yl}amino)-2-(4-fluorophenyl)ethanol | 0.68 (m, 2 H), 0.98 (m, 2 H), 1.87 (m, 1 H), 3.60 (m, 2 H), 4.90 (m, 1H), 5.93 (m, 1 H), 7.10 (m, 2 H), 7.35 (m, 2 H), 7.97 (s, 1H), 9.30 (br s, 1 H), 12.10 (br s, 1 H) | Method 5, and Method 105 |
| **28** | Methyl 6-[(3-cyclopropyl-1H-pyrazol-5-yl)amino]-2-{[(1R)-1-(4-fluorophenyl)-2-hydroxyethyl]amino}pyrimidin e-4-carboxylate | 0.65 (m, 2 H), 0.95 (m, 2 H), 1.88 (m, 1 H), 3.72 (m, 5 H), 5.07 (m, 1 H), 6.02 (s, 1 H), 6.79 (s, 1 H), 7.19 (m, 2 H), 7.39 (m, 2 H). 8.39 (s, 1 H), 11.41 (s, 1 H), 12.40 (br s, 1 H) | Method 10, and Method 104 |
| **29** | 6-[(3-Cyclopropyl-1H-pyrazol-5-yl)amino]-N-[(1R)-1-(4-fluorophenyl)-2-hydroxy ethyl]-2-{[(1R)-1-(4-fluorophenyl)-2-hydroxyethyl] amino}pyrimidine-4-carboxamide | (Acetone-d6): 0.76 (m, 2 H), 1.06 (m, 2 H), 2.58 (m, 1 H), 3.90 (m, 4 H), 5.11-5.29 (m, 2 H), 6.07 (s, 0.5 H), 6.17 (s, 0.5 H), 6.83 (s, 0.5 H), 7.07 (m, 4 H), 7.26 (s, 0.5 H), 7.54 (m, 4 H) | Method 10, and Method 104 |
| **30** R | (2R)-2-({5-Bromo-4-[(5-methyl-1H-pyrazol-3-yl)amino]pyrimidin-2-yl} amino)-2-phenylethanol | (CD₃OD): 2.34 (s, 3 H), 3.88 (m, 1H), 3.92 (m, 1H), 5.03 (m, 1H), 6.03 (br s, 1 H), 7.36 (m, 5 H), 8.15 (s, 1 H) | Method 11 |
| **31** | 5-Chloro-N²-[(1S)-1-(4-fluorophenyl)ethyl]-N⁴-(5-methyl-1H-pyrazol-3-yl)pyrimidine-2,4-diamine | (CD₃OD): 1.57 (d, 3 H), 2.34 (s, 3 H), 5.05 (m, 1 H), 6.08 (br s, 1 H), 7.07 (m, 2 H), 7.33 (m, 2 H), 8.02 (s, 1 H) | Method 12 |
| **32** | 5-Bromo-N²-[(1S)-1-(4-fluorophenyl)ethyl]-N⁴-(5-methyl-1H-pyrazol-3-yl)pyrimidine-2,4-diamine | (CD₃OD): 1.57 (d, 3 H), 2.34 (s, 3 H), 5.02 (m, 1H), 6.08 (br s, 1 H), 7.07 (m, 2 H), 7.32 (m, 2 H), 8.09 (s, 1H) | Method 11 |
| **33** | (2R)-2-({4-[(3-Cyclopropyl-1H-pyrazol-5-yl)amino]-5-fluoropyrimidin-2-yl) amino)-2-phenylethanol | 0.64 (m, 2 H), 0.92 (m, 2 H), 1.82 (m, 1 H), 3.63 (m, 2 H), 4.88 (m, 1 H), 5.99 (s, 1 H), 7.27 (m, 5 H), 8.03 (s, 1 H) | Method 13 |
| **34** | Ethyl 6-[(3-cyclopropyl-1H-pyrazol-5-yl)amino]-2-{[(1S)-1-(4-fluorophenyl)ethyl] amino}pyrimidine-4-carboxylate | 0.65 (m, 2 H), 0.95 (m, 2 H), 1.30 (t, 3 H), 1.46 (d, 3 H), 1.87 (m, 1H), 4.34 (m, 2 H), 5.11 (m, 1 H), 6.05 (s, 1 H), 6.79 (s, 1 H), 7.15 (m, 2 H), 7.42 (m, 2 H), 8.02 (br s, 1 H) | Method 17 |
| **35** | 2-({5-Chloro-4-[(3-cyclopropyl-1H-pyrazol-5-yl)amino]pyrimidin-2-yl}amino)-2-(4-fluorophenyl)ethanol | 0.69 (m, 2 H), 0.96 (m, 2 H), 1.91 (m, 1 H), 3.67 (m, 2 H), 4.93 (m, 1 H), 5.98-6.24 (m, 1.H), 7.14 (m, 2 H), 7.33 (m, 2 H), 8.22 (s, 1 H), 8.76 (s, 1 H), 10.07 (br s, 1 H) | Method 1, and Method 106 |
| **36** | N⁴-(3-Cyclopropyl-1H-pyrazol-5-yl)-N²-[(1S)-1-(4-fluorophenyl)ethyl]-6-methylpyrimidine-2,4-diamine | 0.66 (m, 2 H), 0.95 (m, 2 H), 1.51 (d, 3 H), 1.87 (m, 1 H), 2.25 (s, 3 H), 5.14 (m, 1 H), 6.08 (m, 1 H), 7.18 (m, 2 H), 7.41 (m, 2 H), 8.78 (s, 1H), 10.99 (br s, 1 H), 12.34 (br s, 1 H), 12.72 (br s, 1H) | Method 14 |
| **37** | 2-({5-Bromo-4-[(3-cyclopropyl-1H-pyrazol-5-yl)amino]pyrimidin-2-yl}amino)-2-(4-fluorophenyl)ethanol | 0.45 (m, 2 H), 0.71 (m, 2 H), 1.65 (m, 1 H), 3.41 (d, 2 H), 4.96 (m, 1 H), 5.72 (br s, 1 H), 6.89 (m, 2 H), 7.09 (m, 2 H), 7.88 (br s, 1 H) | Method 5, and Method 106 |
| **38** | 6-Chloro-N⁴-(3-cyclopropyl-1H-pyrazol-5-yl)-N²-[(1S)-1-(4-fluorophenyl)ethyl] pyrimidine-2,4-diamine | 0.66 (m, 2 H), 0.92 (m, 2 H), 1.40 (d, 3 H), 1.84 (m, 1 H), 5.03 (m, 1 H), 5.93-6.17 (m, 1H), 7.12 (m, 2 H), 7.38 (m, 2 H), 7.78 (br s, 1 H), 9.63 (br s, 1 H) | Method 15 |
| **39** | 5,6-Dichloro-N⁴-(3-cyclopropyl-1H-pyrazol-5-yl)-N²-[(1S)-1-(4-fluorophenyl) ethyl]pyrimidine-2,4-diamine | 0.67 (m, 2 H), 0.92 (m, 2 H), 1.39 (m, 3 H), 1.87 (m, 1 H), 4.92 (m, 1 H), 5.93-6.21 (m, 1 H), 7.10 (m, 2 H), 7.33 (m, 2 H), 7.95 (m, 1 H), 8.83 (br s, 1 H), 9.19 (br s, 1 H) | Method 16 |
| **40** | N⁴-(3-Cyclopropyl-1H-pyrazol-5-yl)-N²-[(1S)-1-(4-fluorophenyl)ethyl]-5-methylpyrimidine-2,4-diamine | 0.66 (m, 2 H), 0.95 (m, 2 H), 1.49 (d, 3 H), 1.90 (m, 1 H), 2.05 (s, 3 H), 5.01 (m, 1 H), 6.03 (br s, 1 H), 7.16 (m, 2 H), 7.35 (m, 2 H), 7.70 (s, 1 H), 8.65 (br s, 1 H), 10.04 (s, 1 H), 12.01 (br s, 1H) | Method 9 |
| **41** | N⁴-(3-Cyclopropyl-1H-pyrazol-5-yl)-5-fluoro-N²-[(1S)-1-(4-fluorophenyl)ethyl] pyrimidine-2,4-diamine | 0.66 (m, 2 H), 0.95 (m, 2 H), 1.46 (d, 3 H), 1.88 (m, 1 H), 4.98 (m, 1 H), 6.07 (br s, 1 H), 7.15 (m, 2 H), 7.38 (m, 2 H), 8.04 (s, 1 H) | Method 13 |
| **42** | N`'-(3-Cyclopropyl-1H-pyrazol-5-yl)-N²-[(1S)-1-(4-fluorophenyl)ethyl]pyrimidine-2,4-diamine | 0.66 (m, 2 H), 0.95 (m, 2 H), 1.52 (d, 3 H), 1.86 (m, 1 H), 5.12 (m, 1 H), 6.06 (s, 1 H), 6.28 (m, 1 H), 7.19 (m, 2 H), 7.41 1 (m, 2 H), 7.83 (m 1 H), 8.91 (br s, 1 H), 11.11 (s, 1H), 12.02 (br s, 1H) | Method 8 |
| **43** | (2S)-2-({5-Chloro-4-[(3-cyclopropyl-1H-pyrazol-5-yl)amino]pyrimidin-2-yl}amino)-2-phenylethanol | 0.70 (m, 2 H), 0.95 (m, 2 H), 1.90 (m, 1 H), 3.67 (m, 2 H), 4.93 (m, 1 H), 5.97 (s, 1 H), 7.31 (m, 5 H), 8.16 (s, 1 H), 8.46 (br s, 1 H), 9.97 (br s, 1 H) | Method 1 |
| **44** | (2R)-2-({5-Chloro-4-[(3-cyclopropyl-1H-pyrazol-5-yl)amino]pyrimidin-2-yl}amino)-2-phenylethanol | 0.69 (m, 2 H), 0.95 (m, 2 H), 1.89 (m, 1 H), 3.66 (m, 2 H), 4.92 (m, 1H), 5.97 (s, 1H), 7.31 (m, 5 H), 8.15 (s, 1 H), 9.88 (brs, 1H) | Method 1 |
| **45** | 3-({5-Bromo-4-[(3-cyclopropyl-1H-pyrazol-5-yl)amino]pyrimidin-2-yl}amino)-3-(4-fluorophenyl) propanoic acid | 0.69 (m, 2 H), 0.93 (m, 2 H), 1.87 (m, 1 H), 2.64 (d, 2 H), 5.5 (m, 1 H), 6.10-6.20 (m, 1 H), 7.12 (m, 2 H), 7.36 (m 2 H), 8.07 (m, 1 H), 8.78 (br s, 1 H) | Method 5 |
| **46** | 2-[{5-Bromo-4-[(3-cyclopropyl-1H-pyrazol-5-yl)amino]pyrimidin-2-yl}(1-phenylethyl)amino]ethanol | (CD₃OD): 0.60 (m, 1H), 0.75 (m, 1H), 1.64 (d, J = 9.0 Hz, 3 H), 1.86 (m, 1 H), 3.51 (m, 4 H), 5.94 (q, J = 9.0 Hz, 1 H), 6.12 (s, 1 H), 7.35 (m, 5 H), 8.18 (s, 1 H) | Method 5 |
| **47** | 5-Chloro-N⁴-(5-cyclopropyl-1H-pyrazol-3-yl)-N²-[(1R)-1-(4-fluorophenyl)-2-methoxy ethyl]pyrimidine-2,4-diamine | (CD₃OD): 0.6 (m, 2 H), 0.9 (m, 2 H), 1.8 (m, 1 H), 3.2 (s, 3 H), 3.5 (m, 2 H), 5.0 (br s, 1 H), 5.9 (br s, 1 H), 6.9 (m, 2 H), 7.2 (m, 2 H), 7.9 (m, 1 H) | Method 1, and Method 113 |
| **48** | 5-Chloro-N⁴-(5-cyclopropyl-1H-pyrazol-3-yl)-N²-[1-(4-fluorophenyl)-2-morpholin-4-ylethyl]pyrimidine-2,4-diamine | (CD₃OD): 0.9 (m, 2 H), 1.1 (m, 2 H), 2.0 (m, 1 H), 3.5 (m, 4 H), 3.9 (m, 6 H), 5.5 (br s, 1 H), 6.1 (br s, 1 H), 7.1 (m, 2 H), 7.5 (m, 2 H), 8.1 (s, 1 H) | Method 1, and Method 114 |
| **49** R | 2-({5-Chloro-4-[(5-cyclopropyl-1H-pyrazol-3-yl)amino]pyrimidin-2-yl}amino)-2-(2-thienyl)ethanol | 0.67 (m, 2 H), 0.93 (m, 2 H), 1.88 (m, 1 H), 3.73 (m, 2 H), 4.65-4.80 (m, 1H), 5.20 (m, 1H), 6.07 (s, 1H), 6.98 (m, 2 H), 7.46 (m, 1 H), 8.10 (br s, 1 H), 8.20 (s, 1 H), 8.65 (m, 1 H), 10.22 (m, 1 H) | Method 1, and Method 108 |
| **50** R | N⁴-(5-Cyclopropyl-1H-pyrazol-3-yl)-N²-[(1S)-1-phenylethyl]quinazoline-2,4-diamine | 0.67 (m, 2 H), 0.96 (m, 2 H), 1.57 (m, 3 H), 1.93 (m, 1 H), 5.27 (m, 1 H), 6.17 (br s, 1 H), 7.38 (m, 7 H), 7.82 (m, 1H), 8.59 (d, 1 H), 8.96 (br s, 1 H), 11.40 (br s, 1 H), 12.67 (br s, 1 H) | Method 55 |
| **51** | N⁴-(5-Cyclopropyl-1H-pyrazol-3-yl)-N2-[(1S)-1-(4-fluorophenyl)ethyl]quinazoline-2,4-diamine | 0.65 (m, 2 H), 0.98 (m, 2 H), 1.56 (m, 3 H), 1.93 (m, 1H), 5.25 (m, 1H), 6.15 (br s, 1 H), 7.18 (m, 2 H), 7.42 (m, 4 H), 7.82 (m, 1 H), 8.58 (d, 1 H), 9.05 (br s, 1 H), 11.40 (br s, 1 H), 12.87 (br s, 1 H) | Method 55 |
| **52** R | (2R)-2-({4-[(5-Cyclopropyl-IH-pyrazol-3-yl)amino] quinazolm-2-yl}ammo)-2-phenylethanol | 0.77 (m, 2 H), 0.99 (m, 2 H), 1.94 (m, 1 H), 3.80 (m, 2 H), 5.20 (m, 1 H), 6.16 (s, 1 H), 7.38 (m, 7 H), 7.83 (m, 1 H), 8.57 (d, 1 H), 8.70 (br s, 1 H), 11.43 (br s, 1 H), 12.52 (br s, 1H) | Method 55 |
| **53** | N⁴-(5-tert-Butyl-1H-pyrazol-3-yl)-N²-[(1S)-1-(4-fluorophenyl)ethyl]quinazoline-2,4-diamine | (CH₃OD): 1.37 (s, 9 H), 1.64 (d, 3 H), 2.35 (s, 3 H), 5.34 (m, 1 H), 6.45 (br s, 1 H), 7.04 (m, 2 H), 7.35 (m, 2 H), 7.48 (m, 2 H), 7.83 (m, 1 H), 8.29 (d, 1 H) | Method 56 |
| **54** | N²-[(1S)-1-(4-Fluorophenyl) ethyl]-N⁴-(5-methyl-1H-pyrazol-3-yl)quinazoline-2,4-diamine | (CH₃OD): 1.62 (d, 3 H), 2.35 (s, 3 H), 5.23 (m, 1 H), 6.16 (br s, 1 H), 7.07 (m, 2 H), 7.36 (m, 2 H), 7.50 (m, 2 H), 7.84 (m, 1 H), 8.31 (d, 1 H) | Method 57 |
| **55** R | (2R)-2-({4-[(5-Methyl-1H-pyrazol-3-yl)amino] quinazolin-2-yl}amino)-2-phenylethanol | 2.27 (s, 3 H), 3.79 (m, 2 H), 5.15 (m, 1 H), 6.10 (s, 1 H), 7.26-7.53 (m, 7 H), 7.83 (m, 1H), 8.57 (m, 1 H), 11.44 (s, 1 H), 12.81 (s, 1 H) | Method 57 |
| **56** R | (2R)-2-({4-[(5-Cyclopropyl-1H-pyrazol-3-yl)amino] thieno[3,2-d]pyrimidin-2-yl}amino)-2-phenylethanol | 0.6 (m, 2 H), 0.9 (m, 2 H), 1.9 (m, 1 H), 3.8 (m, 2 H), 5.1 (br s, 1 H), 6 (br s, 1 H), 7.1 - 7.6 (m, 6 H), 8.1 (br s, 1 H), 8.6 (br s, 1 H), 11.5 (s, 1 H), 13.0 (br s, 1 H) | Method 70 |
| **57** | N⁴-(5-Cyclopropyl-1H-pyrazol-3-yl)-N²-[(1S)-1-(4-fluorophenyl)ethyl]thieno[2,3-d]pyrimidine-2,4-diamine | 0.7 (m, 2 H), 1.0 (m, 2 H), 1.5 (m, 3 H), 1.9 (m, 1 H), 5.4 (m, 1 H), 6.4 (br s, 1 H), 7.1 - 7.6 (m, 3 H), 7.5 (m, 2 H), 7.8 cm, 1 H), 8.5 (br s, 1 H), 11 (br s, 1 H) | Method 71 |
| **58** | N⁴-(5-Cyclopropyl-1H-pyrazol-3-yl)-N²-[(1S)-1-(4-fluorophenyl)ethyl]thieno[3,2-d]pyrimidine-2,4-diamine | (CD₃OD): 0.6 (m, 2 H), 0.9 (m, 2 H), 1.4 (m, 3 H), 1.8 (m, 1 H), 5.0 (br s, 1 H), 6.0 (br s, 1 H), 6.9 (m, 2 H), 7.1 (m, 1 H), 7.25 (m, 2 H) | Method 70 |
| **59** R | (2R)-2-({4-[(5-Cyclopropyl-1H-pyrazol-3-yl)amino]-1H-pyrazolo[3,4-d]pyrimidin-6-yl}amino)-2-phenylethanol | 0.5-0.9 (m, 2 H), 1.0 (m, 2 H), 1.9 (m, 1 H), 4.2 (m, 2 H), 5.2 (br s, 1 H), 6.2 (br s, 1 H), 7.2 - 7.5 (m, 6 H), 8.4 - 8.7 (m, 2 H), 11.7 (s, 1 H), 12.5 (br s, 1 H) | Method 72 |
| **60** R | (2R)-2-({4-[(5-Cyclopropyl-1H-pyrazol-3-yl)amino] thieno[2,3-d]pyrimidin-2-yl} amino)-2-phenylethanol | 0.8 (m, 2 H), 1.0 (m, 2 H), 2.0 (m, 1 H), 3.9 (m, 2 H), 5.2 (m, 1 H), 6.3 (br s, 1 H), 7.2 (m, 1 H), 7.3 (m, 1 H), 7.4-7.5 (m, 5 H), 7.8 (m, 1 H), 8.0 (m, 1 H), 10.5 (br s, 1 H) | Method 71 |
| **61** | (2R)-2-({4-[(5-Cyclopropyl-1H-pyrazol-3-yl)aminol thieno[3,4-d]pyrimidin-2-yl}amino)-2-phenylethanol | (CD₃OD): 0.9 (m, 2 H), 1.1 (m, 2 H), 2.0 (m, 1 H), 3.7 (m,1 1 H), 4.0 (m, 2 H), 7.3-7.6 (m, 6 H), 8.7 (s, 1H) | Method 73 |
| **62** | N⁴-(5-Cyclopropyl-1H-pyrazol-3-yl)-N⁶-[(1S)-1-(4-fluorophenyl)ethyl]-1H-pyrazolo [3,4-d]pyrimidine-4,6-diamine | (CD₃OD): 1.1 (m, 2 H), 1.3 (m, 5 H), 1.9 (m, 1 H), 5.6 (m, 1H), 7.3 (m, 2H), 7.8 (m, 2 H), 8.1 (br s,1 H) . | Method 72 |
| **63** | (2R)-2-({4-[(S-Cyctopropyl-1H-pyrizol-3-yl)amino] thieno[3,2-d]pyrimidin-2-yl} amino)-2-(4-fludrophenyl)ethanol | (CD₃OD): 0.6 (m, 2 H), 0.9 (m, 2 H), 1.8 (m, 1 H), 3.7 (m, 2 H), 5.0 (br s,1 H), 6.0 (m, 2 H), 6.9 (m, 2 H), 7.1 (m, 1 H), 7.3 (m, 2 H), 8.0 (br s, 1H) | Method 70, and Methods 104 |
| **64** | (3S)-3-({4-[(5-Cyclopropyl-1H-pyrazol-3-yl)amino] thieno[3,2-d]pyrimidin-2-yl}amino)-3-(4-fluorophenyl)propan-l-ol | (CD₃OD): 1.0 (m, 2 H), 1.3 (m, 2 H), 2.2 (m, 1H), 2.4 (m, 2 H), 3.9 (m, 2.H), 5.6 (br s, 1 H), 6.4 (m, 2 H), 7.1-7.7 (m, 5 H), 8.4 (br s, 1H) | Method 70, and Method 107 |
| **65** | N⁴-(5-Cyclopropyl-1H-pyrazol-3-yl)-N²-[(1S)-1-(4-fluorophenyl)ethyl]pyrido[2,3-d]pyrimidine-2,4-diamine | 0.6 (m, 2 H), 0.85 (m, 2 H), 1.4 (d, J = 4 Hz, 3H), 1.8 (m, 1H), 5.1 (m, 1H), 6.1 (s, 1 H), 7.1 (m, 2 H), 7.3 (m, 3 H), 8.6 (m, 1 H), 9.0 (m 1 H) | Method 90 |
| **66** R | (2R)-2-({4-[(5-Cyclopropyl-1H-pyrazol-3-yl)anino] pyrido[2,3-d]pyrimidin-2-. yl}amino)-2-phenylethanol | 0.6-1 (m, 4 H), 1.9 (m, 1 H), 3.9 (m, 2 H), 5.2 (m, 1H), 6.2 (br s,1 H), 7.2-7.6 (m, 5 H) 8.8 (m, 1 H), 8.9 (m, 1 H), 9.1 (m, 1 H), 11.7 (br s, 1 H), 12.6 (br s,1 H) | Methods 90 |
| **67** | N⁴-(5-Cyclopropyl-1H-pyrazol-3-yl)-N²-[(1S)-1-(4-fluorophenyl)ethyl]-7-methylquinazoline-2,4-diamine | (120°C): 0.7 (m, 1 H), 1.0 (m, 2 H), 1.3 (m, 1 H), 1.6 (m, 3 H), 1.9 (m, 1 H), 2.5 (s, 3 H), 4.1 (m, 1 H), 5.3 (m, 1 H), 6.2 (s, 1 H), 7.1-7.5 (m, 6 H), 7.7 (m, 1 H), 8.4 (m, 1 H), 8.7 (br s, 1 H), 10.9 (br s, 1 H) | Method 91 |
| **68** | N⁴-(S-Cyclopropyl-1H-pyrazol-3-yl)-N²-[(1S)-1-(4-fluorophenyl)ethyl]-6-methylquinazoline-2,4-diamine | 0.7 (m, 2 H), 1.0 (m, 2 H), 1.6 (m, 3 H), 1.9 (s, 1 H), 2.5 (m, 3 H), 5.2 (m, 1 H), 6.2 (m, 1 H), 7.1 (m, 2 H), 7.3 (m, 3 H), 7.7 (m, 1 H), 8.1 (m, 1 H) | Method 92 |
| **69** | N⁴-(5-Cyclopropyl-1H-pyrazol-3-yl)-N²-[(1S)-1-(4-fluorophenyl)ethyl]-6-methoxyquinazoline-2,4-diamine | 0.7 (br s, 2 H), 1.0 (m, 2 H), 1.6 (m, 3 H), 1.9 (s, 1 H), 4.3 (m, 3 H), 5.2 (m, 1 H), 6.2 (m, 1 H), 7.1 (m, 2 H), 7.3 - 7.4 (m, 4 H), 7.8 (m, 1 H) | Method 93 |
| **70** | 7-Chloro-N⁴-(5-cyclopropyl-1H-pyrazol-3-yl)-N²-[(1S)-1-(4-fluorophenyl)ethyl] quinazoline-2,4-diamine | 0.7 (br s, 2 H), 1.0 (m, 2 H), 1.6 (m, 3 H), 1.9 (s, 1 H), 4.3 (m, 3 H), 5.2 (m, 1 H), 6.2 (m, 1 H), 7.1 (m, 2 H), 7.3 - 7.5 (m, 4 H), 8.3 (m, 1 H) | Method 94 |
| **71** | 6-Chloro-N⁴-(5-cyclopropyl-1H-pyrazol-3-yl)-N²-[(1S)-1-(4-fluorophenyl)ethyl] quinazoline-2,4-diamine | 0.7 (m, 2 H), 1.0 (m, 2 H), 1.6 (m, 3 H), 1.9 (s, 1 H), 4.3 (m, 3 H); 5.2 (m, 1 H), 6.2 (m, 1 H), 7.1 (m, 2 H), 7.3 - 7.5 (m, 4 H), 8.3 (m, 1 H) | Method 95 |
| **72** | N⁴-(5-Cyclopropyl-1H-pyrazol-3-yl)-N²-[(1S)-1-(4-fluoro phenyl)ethyl]-8-methoxy quinazoline-2,4-diamine | 0.7 (m, 2 H), 1.0 (m, 2 H), 1.6 (m, 3 H), 1.9 (s, 1 H), 4.3 (m, 3 H), 5.2 (m, 1 H), 6.2 (m, 1 H), 7.1 (m, 2 H), 7.3 - 7.5 (m, 3 H), 7.8 (m, 1 H), 8.4 (m, 1 H) | Method 96 |
| **73** | 8-Chloro-N⁴-(5-cyclopropyl-1H-pyrasol-3-yl)-N²-[(1S)-1-(4-fluorophenyl)ethyl]quinazoline-2,4-diamine | 0.7 (m, 2 H), 1.0 (m, 2 H), 1.6 (m, 3 H), 1.9 (s, 1 H), 5.2 (m, 1H), 6.2 (m, 1H), 7.1 (m, 2H), 7.3 - 7.5 (m, 5H), 7.9 (m, 1H), 8.3 (m, 1H) | Method 97 |
| **74** | (2R)-2-({4-[(5-Cyclopropyl-1H-pyrazol-3-yl)amino]-1H-pyrazolo[3,4-d]pyrimidin-6-yl}amino)-2-(4-fluorophenyl)ethanol | (CD₃OD): 0.6 (m, 2 H), 1.0 (m, 2 H), 1.9 (m, 1H), 3.7 (m, 2H), 5.0 (br s, 1H), 6.0 (m, 1H), 6.9 (m, 2 H), 7.3 (m, 2 H), 8.3(brs,1H) | Method 72, and Method 104 |
| **75** R | (2R)-2g ({6-Chloro-4-[(5-cyclopropyl-1H-pyrazol-3-yl)amino]quinazolin-2-yl}amino)-2-phenylethanol | 0.83 (m, 2 H), 1.1 (m, 2 H), 4.0 (m, 2 H), 5.5 (t, 1H), 6.5 (s, 1H), 7.3 - 7.5 (m, 5 H), 7.6 - 7.7 (m, 2 H), 8.4 (s, 1 H) | Method 95 |
| **76** R | (2R)-2-({7-Chloro-4-[(5-cyclopropyl-1H-pyrazol-3-yl)amino]quinazolin-2-yl}amino)-2-phenylethanol | 0.83 - 0.91 (m, 2 H), 1.1 (m, 2 H), 4.0 (m, 2 H), 5.5 (t,1H), 6.5 (s, 1H), 7.2- 7.4 (m, 4 H), 7.5 (m, 2 H), 7.6 (s, 1 H), 8.2 (d,1 H) | Method 94 |
| **77** | N⁴-(5-Cyclopropyl-1H-pyrazol-3-yl)-7-fluoro-N²-[(1S)-1-(4-fluorophenyl)ethyl] quinazoline-2,4-diamine | 0.70 (m, 2 H), 0.91 (m, 2 H), 1.46 (d, 3 H), 1.888 (m, 1 H), 5.23 (m, 1 H), 6.19 (br s, 1 H), 6.94 (m, 2 H), 7.11 (m, 2 H), 7.44 (m, 2 H), 7.66 (br s, 1H), 8.13 (s, 1 H), 8.31 (m, 1H), 10.15 (br s, 1H) | Methods 102 |
| **78** | (2R)-2-({4-[(5-Cyclopropyl-1H-pyrazol-3-yl)amino]-7-fluoroquinazolin-2-yl}amino)-2-(4-fluorophenyl)ethanol | (CD₃OD): 0.8 (m, 2 H), 1.0 (m, 2 H), 1.9 (m, 1 H), 3.9 (s, 2 H), 5.2 (d, 1 H), 6.1 (br s, 1H), 7.1 (m, 2H), 7.3 (m, 2 H), 7.4 (m, 1H), 7.5 (m, 1H), 8.4 (m, 1H) | Method 102, and Method 104 |
| **79** | (2R)-2-({4-[(5-Cyclopropyl-1H-pyrazol-3-yl)amino]-7-methylquinazolin-2-yl}amino)-2-(4-fluorophenyl)ethanol | 0.8 (m, 2 H), 1.1 (m, 2 H), 2.0 (in, 1 H), 2.5 (s, 3 H), 4:0 (m, 2 H), 5.5 (m, 1H), 6.5 (m, 1 H); 7.1 - 7.5 (m, 7 H), 8.2 (m, 1 H) | Method 91, and Method 104 |
| **80** | (2R)-2-({4-[(5-Cyclopropyl-1H-pyrazol-3-yl)amino]-6-methoxyquinazolin-2-yl } amino)-2-(4-fluorophenyl)ethanol | 0.8 (m, 2 H), 1.1 (m, 2 H), 2.0 (m, 1 H), 2.4 (m, 1 H), 3.4(m,1 H), 4.0 (m, 3 H), 5.4 (m, 1 H), 6.4 (s, 1 H), 7.1, (m, 2 H), 7.4 - 7.5 (m, 4 H), 7.9 (d, 1 H) | Method 93, and Method 104 |
| **81** | N⁴-(5-Cyclopropyl-1H-pyrazol-3-yl)-6-fluoro-N²-[(1S)-1-(4-auorophenyl)ethyl] quinazoline-2,4-diamine . | (CD₃OD): 0.7 (m, 2 H), 0.99 (m, 2 H), 1.59 (d, 3 H), 1.93 (m, 1 H), 5.22 (m, 1 H), 6.13 (s, 1 H), 7.03 (m, 2 H), 7.32 (m, 2 H), 7.60 (m, 2 H), 8.12 (d, 1 H) | Method 103 |
| **82** | (2R)-2-({5-Bromo-4-[(3-methoxy-1H-pyrazol-5-yl) amino]pyrimidin-2-yl}amino)-2-(4-fluorophenyl)ethanol | 3.63 (m, 2 H), 3.76 (s, 3 H), 4.94 (m, 1 H), 5.64 (s, 1 H), 7.13 (m, 2 H), 7.38 (m, 2 H), 8.04 (s, 1 H), 8.18 s (br s, 1 H), 9.49 (s, 1 H) | Method 115, and Method 104 |
| **83** | (2R)-2-[(5-Chloro-4-{[5-(methylthio)-1H-pyrazol-3-yl]amino}pyrimidin-2-yl)amino]-2-(4-fluorophenyl)ethanol | 2.45 (s, 3 H), 3.64 (m, 2 H), 4.94 (m, 1 H), 6.25 (s, 1 H), 7.13 (m, 2 H), 7.39 (m, 2 H), 7.98 (s, 1 H), 8.17 (s, 1 H), 9.74 (br s, 1 H) | Method 118, and Method 104 |
| **84** | (2R)-2-({4,5-Dichloro-6-[(3-cyclopropyl-1H-pyrazol-5-yl)amino]pyrimidin-2-yl} amino)-2-(4-fluorophenyl) ethanol | (CD₃OD): 0.73 (m, 2 H), 0.97 (m, 2 H), 1.90 (m, 1 H), 3.76 (m, 2 H), 5.00 (m, 1 H), 6.05 (br s, 1 H), 7.04 (m, 2 H), 7.37 (m, 2H) | Method 16, and Method 104 |
| **85** | (2R)-2-{[5-Chloro-4-(1H-pyrazol-5-ylamino)pyrimidin-2-yl]amino}-2-(4-fluorophenyl)ethanol | 3.65 (m, 2 H), 4.88 (m, 1H), 6.23 (s, 1 H), 7.13 (m, 2 H), 7.28 (m, 2 H), 7.73 (s, 1 H), 8.18 (s, 1 H), 10.14 (s, 1 H) | Method 120, and Method 104 |
| **86** | (2R)-2-[(5-Chloro-4-{[3-(dimethylamino)-1H-pyrazol-5-yl]amino}pyrimidin-2-yl)amino]-2-(4-fluorophenyl)ethanol | 2.90 (s, 6 H), 3.66 (m, 2 H), 4.99 (m, 1 H), 5.70 (s, 1 H), 7.13 (m, 2 H), 7.37 (m, 2 H), 7.96 (br s, 1 H), 8.06 (s, 1 H), 10.11 (br s, 1 H) | Method 121, and Method 104 |
| **87** | 3-({5-Bromo-4-[(3-cyclopropyl-1H-pyrazol-5-yl)amino]pyrimidin-2-yl}amino)-2-methyl-3-phenylpropanoic acid | 0.89 (m, 7 H), 1.3 (m, 0.5 H), 1.58 (m, 0.5 H), 1.95 (m, 1 H), 5.08 (m, 1 H), 6.21 (s, 1 H), 7.29 (m, 5 H), 8. 12 (s, 1 H) | Method 5 |
| **88** | 3-({5-Bromo-4-[(3-cyclopropyl-1H-pyrazol-5-yl)amino]pyrimidin-2-yl}amino)-2-methyl-3-phenylpropan-1-ol | 0.72-0.98 (m, 7 H), 1.90 (m, 1H), 2.03 (m, 1 H), 3.37 (m, 2 H), 4.90 (m, 1 H), 6.09 (s, 1 H), 7.28 (m, 5 H), 8.04 (br s, 1 H), 8.18 (s, 1H), 8.31 (br s, 1 H), 9.32 (br s, 1 H) | Method 5, and Method 109 |
| **89** | 5-Chloro-N⁴-(5-cyclopropyl-1H-pyrazol-3-yl)-14²-[(1S)-1-(4-fluorophenyl)-3-morpholin-4-ylpropyl]pyrimidine-2,4-diamine | (CD₃OD): 0.8 (m, 2 H), 1.2 (m, 2 H), 2.1 (m, 1 H), 2.4 (m, 1 H), 2.6 (m, 1 H), 3.2 (m, 3 H), 3.6 (m, 3 H), 3.8 (m, 2 H), 4.1 1 (m, 2 H), 5.1 (br s, 1 H), 6.1 (br s, 1 H), 7.1 (m, 2 H), 7.4 (m, 2 H), 8.1 (s, 1 H) | Method 1, and Method 125 |
| **90** | 5-Chloro-N⁴-(5-cyclopropyl-1H-pyrazol-3-yl)-N²-[(1S)-1-(4-fluorophenyl)-3-pyrrolidin-l-ylpropyl]pyrimidine-2A-diamine | (CD₃OD): 1.0 (m, 2 H), 1.3 (m, 2 H), 2.1 (m, 3 H), 2.3 (m, 2 H), 2.5 (m, 1 H), 2.6 (m, 1 H), 3.2 (m, 2 H), 3.4 (m, 2 H), 3.8 (m, 2 H), 5.2 (m, 1 H), 6.2 (br s, 1 H), 7.3 (m, 2 H), 7.6 (m, 2 H), 8.1 (s, 1 H) | Method 1, and Method 127 |
| **91** | S-Chloro-N⁴-(5-cyclopropyl-1H-pyrazol-3-yl)-N²-[(1S)-3-(diethylamino)-1-(4-fluoro phenyl)propyl]pyrimidine-2,4-diamine | (CD₃OD): 0.8 (m, 2 H), 1.1 (m, 2 H), 1.3 (m, 6 H), 2.1 (m, 1 H), 2.3 (m, 1 H), 2.4 (m, 1 H), 3.2 (m, 6 H), 5.1 (br s, 1 H), 6.1 (br s, 1 H), 7.2 (m, 2 H), 7.4 (m, 2 H), 8.1 (s, 1 H) | Method 1, and Method 128 |
| **92** | 5-Chloro-N⁴-(5-cyclopropyl-1H-pyrazol-3-yl)-N²-[(1S)-1-(4-fluorophenyl)-3-(4-methyl piperazin-1-yl)propyl] pyrimidine-2,4-diamine | (CD₃OD): 0.6 (m, 2 H), 0.9 (m, 2 H), 1.8 (m, 1 H), 2.2 (m, 2 H), 2.8 (s, 3 H), 3.1-3.8 (m, 10 H), 4.9 (br s, 1 H), 6.0 (br s, 1 H), 6.9 (m, 2 H), 7.2 (m, 2 H), 7.9 (s, 1 H) | Method 1, and Method 129 |
| **93** | 5-Chloro-N⁴-(5-cyclopropyl-1H-pyrazol-3-yl)-N²-{(1S)-1-(4-fluorophenyl)-3-[(2-methoxyethyl)(methyl)amino] propyl}pyrimidine-2,4-diamine | (CD₃OD): 0.6 (cm, 2 H), 0.9 (m, 2 H), 1.8 (m, 1H), 2.1 (m, 1H), 2.2 (m, 1 H), 2.6 (s, 3 H), 3.1 (m, 4 H), 3.2 (d, J = 4.8 Hz, 3 H), 3.5 (m, 2 H), 4.8 (m, 1H), 5.8 (br s, 1H), 6.9 (m, 2 H), 7.2 (m, 2 H), 7.9 (s, 1H) | Method 1, and Method 130 |
| **94** | 2-[[(3S)-3-({5-Chloro-4-[(5-cyclopropyl-1H-pyrazol-3-yl)amino]pyrimidin-2-yl} amino)-3-(4-fluorophenyl) propyl](methyl)amino]ethanol | (CD₃OD): 0.8 (m, 2 H), 1.1 (m, 2H), 2.0 (m, 1H), 2.3 (m, 1H), 2.4 (m, 1H), 2.9 (s,3H),3.2(m,4H),3.8(m,2H),5.1 (br s, 1H), 6.0 (br s, 1H), 7.1 (m, 2H), 7.4 (m, 2 H), 8.1 (s, 1H) | Method 1, and Method 131 |
| **95** | (3S)-3-({5-Chloro-4-[(5-cyclopropyl-1H-pyrazol-3-yl)amino]pyrimidin-2-yl}amino)-3-(4-fluorophenyl) propan-1-ol | (CD₃OD): 0.7 (m, 2 H), 1.1 (m, 2 H), 2.0 (m, 3 H), 3.5 (m, 2H), 5.1 (br s, 1 H), 6.1 (br s, 1H), 7.0 (m, 2 H), 7.2 (m, 2 H), 8.1 (s, 1H) | Method 1, Method 107 |
| **96** | (2R)-2-({4-[(5-Cyclopropyl-1H-pyrazol-3-yl)amino]-5-methylpyrimidin-2-yl}amino)-2-(4-fluorophenyl)ethanol | 0.68 (m, 2 H), 0.97 (m, 2 H), 1.90 (m, 1 H), 2.05 (s, 3 H), 3.66 (m, 2 H), 4.95 (m, 1H), 5.99 (br s, 1H), 7.15-7.20 (m, 2 H), 7.33 (m, 2 H), 7.73 (m, 1H), 8.49 (m, 1H), 10.06 (brs, 1H) | Method 9, Method 104 |
| **97** | (2R)-2-({4-[(5-Cyclopropyl-1H-pyrazol-3-yl)amino]-5-fluoropyrimidin-2-yl} amino)-2-(4-fluorophenyl)ethanol | 0.70 (m, 2 H), 0.97 (m, 2 H), 1.90 (m, 1 H), 3.66 (m, 2 H), 4.64 (m, 1 H), 4.95 (m, 1H), 5.36 (m, 1H), 6.02 (br s, 1H), 7.15-7.20 (m, 2 H), 7.37-7.48 (m, 2 H), 8.18 (m, 1H), 8.69 (m, 1H), 11.15 (m, 1 H) | Method 13, Method 104 |
| **98** | (R)-2-[4-(5-Cyclopropyl-1H-pyrazol-3-ylamino)-pyrido[2,3d]pyrimidin-2-ylamino]-2-(4-fluoro-phenyl)-ethanol | (CD₃OD): 0.76 (m, 2 H), 0.97 (m, 2 H), 1.96 (m, 1H), 3.84 (m, 2 H), 5.35 (m, 1 H), 7.04 (m, 3 H), 7.45 (m, 2 H), 8.49 (m, 1H), 8.64 (m, 1H) | Method 90, Method 104 |
| **99** R | 5-Chloro-N⁴-(5-cyclopropyl-1H-pyrazol-3-yl)-N²-[(S)-1-(2-methoxy-phenyl)-ethyl]-pyritmdine-2,4-dian-iine | (CD₃OD): 0.75 (m, 2 H), 1.04 (m, 2 H), 1.55 (d, 3 H), 1.96 (m, 1 H), 3.89 (s, 3 H), 5.33 (m, 1H), 6.20 (br s, 1 H), 6.92 (m, 1H), 7.02 (d, 1H), 7.21 (m, 1H), 7.28 (m, 1H), 8.01 (s, 1H) | Method 1 |
| **100** | (2R)-2-({4-[(5-Cyclopropyl-1H-pyrazol-3-yl)amino]-5-nitropyrimidin-2-yl}amino)-2-(4-fluorophenyl)ethanol | 0.67 (m, 2 H), 0.98 (m, 2 H), 1.93 (m, 1 H), 3.66 (m, 2 H), 5.01 (m,1H), 6.20 (m, 1H), 7.16 (m, 2 H), 7.40 (m, 2 H), 8.97 (s, 1H), 10.40 (br s, 1 H), 12.36 (m, 1H) | Method 18 |
| **101** | N⁴-(5-Cyclopropyl-1H-pyrazol-3-yl)-N²-[(1S)-1-(4-fluorophenyl)ethyl]-5-nitropyrimidine-2,4-diamine | 0.63 (m, 2 H), 0.94 (m, 2 H), 1.47 (m, 3 H), 1.90 (m, 1H), 5.12 (m, 1H), 6.13 (m, 2 H), 7.13 (m, 2 H), 7.40 (m, 2 H), 8.97 (s, 1H), 10.31 (br s, 1 H) | Method 18 |
| **102** R | 5-Chloro-N⁴-(5-cyclopropyl-1H-pyrazol-3-yl)-N²-methyl-N²-(1-pyridin-2-ylethyl) pyrimidine-2,4-diamine | 0.55 (m, 2 H), 0.82 (m, 2 H), 1.55 (m, 3 H), 1.80 (m, 1 H), 2.90 (s, 3 H), 5.80 (m, 1H), 5.90 (m, 1H), 7.55 (m, 2 H), 8.05 (m, 2 H), 8.55 (m, 1H), 9.50 (br s, 1H) | Method 1, Method 20 |
| **103** R | 1-({5-Chloro-4-[(5-cyclopropyl-1H-pyrazol-3-yl)amino]pyrimidin-2-yl} amino)-1-phenylpropan-2-ol | 0.60 (m, 2 H), 0.80 - 1.00 (m, 5 H), 1.80 (m, 1H), 3.45 - 3.55 (m, 1H), 3.85 (m, 1 H), 4.60 - 4.90 (m, 1H), 5.80 - 6.10 (m, 1H), 7.10 - 7.40 (m, 5 H), 7.80-8.40 (m, 1H) | Method 1, Method 21 |
| **104** | 5-Chloro-N²-[(1S)-1-(4-fluorophenyl)-ethyl]-N⁴-(5-trifluoromethyl-1H-pyrazol-3-yl)-pyrimidine-2,4-diamine | (400 MHz, CDCl₃): 1.54 (d, J = 6.8 Hz, 3 H), 4.94 (m, 1H), 5.73 (br s, 1H), 6.10 (s, 1 H), 7.04 (m, 2 H), 7.38 (m, 2 H), 8.00 (s, 1H), 11.80 (br s, 1H) | Method 19 |
| **105** R | 5-Bromo-N⁴-(5-cyclopropyl-1H-pyrazol-3-yl)-N²-(1-pyridin-2-ylethyl)-pyrimidine-2,4-diamine | (400 MHz, CDCl₃): 0.71 (m, 2 H), 0.89 (m, 2 H),1.53 (d, J = 9.6 Hz, 3 H), 1.84 (m, 1H), 5.13 (m, 1H), 6.04 (br s, 1 H), 6.42 (br s, 1H), 7.10 (t, J = 9.3 Hz, 2 H), 7.26 (m, 1H), 7.52 (s, 1H), 7.57 (m, 1 H), 7.96 (s, 1H), 8.54 (d, J = 6.6 Hz, 1 H) | Method 5, Method 24 |
| **106** | N⁴-(5-Benzyl-2H-pyrazol-3-yl)-5-chloro-N²-[(1S)-1-(4-fluorophenyl)ethyl]-pyrimidine-2,4-diamine | (400 MHz, CDCl₃): 1.47 - 1.49 (d, J = 7.0 Hz, 3 H), 3.94 (s, 2 H), 4.98 (q, 1H), 5.6 (br s, 1H), 6.15 (s, 1H), 6.96 (t, 2 H), 7.2 - 7.32 (m, 7 H), 7.86 (s, 1H) | Method 25 |
| **107** | 5-Chloro-N²-[(1S)-1-(4-fluorophenyl)-ethyl]-N⁴-(5-isopropyl-2H-pyrazol-3-yl)-pyrimidine-2,4-diamine | (400 MHz, CDCl₃): 1.24 - 1.26 (d, J = 7.0 Hz, 6 H), 1.50 - 1.52 (d, J = 7.0 Hz, 3 H), 2.89 - 2.96 (q, 1H), 5.02 - 5.09 (q, 1 H), 5.62 (br s, 1H), 6.24 (s, 1H), 6.96 (t, 2 H), 7.30 (t, 2 H), 7.87 (s, 1H) | Method 28 ' |
| **108** | 5-Chloro-N⁴-(5-cyclopropylmethyl-1H-pyrazol-3-yl)-N²-[(1S)-1-(4-fluorophenyl)ethyl]-pyrimidine-2,4-diamine | (400 MHz, CDCl₃): 0.00 (m, 2 H), 0.371 (m, 2 H), 0.772 (m, 1H), 1.31 - 1.33 (d, J = 7.0 Hz, 3 H), 2.32 - 2.33 (d, J = 7.0 Hz, 2 H), 4.84 (q, 1H), 6.02 (s, 1H), 6.77 (t, 2 H), 7.11 (t, 2 H), 7.66 (s, 1H) | Method 31 |
| **109** | 5-Chloro-N⁴-[5-(cyclopropylmethoxy)-1H-pyrazol-3-yl]-N²-[(1S)-1-(4-fluorophenyl)ethyl]pyrimidine-2,4-diamine | (400 MHz, CDCl₃): 0.021 (m, 2 H), 0.293 (m, 2 H), 0.947 (m, 1H), 1.20 (d, J = 6.6 Hz, 3 H), 3.61 (d, J = 7.4 Hz, 2 H), 4.64 (q, 1H), 5.04 (s, 1H), 6.69 (t, 2 H), 7.02 (t, 2 H), 7.62 (s, 1H) | Method 35 |
| **110** R | 5-Bromo-N⁴-(5-cyclopropyl-1H-pyrazol-3-yl)-N²-[(1S)-1-(4-trifluoromethyl-thiazol-2-yl)-ethyl]-pyrimidine-2,4-diamine | (400 MHz): 0.63 (m, 2 H), 0.87 (m, 2 H), 1.60 (m, 1H), 5.29 (br s, 1H), 5.92 (br s, 0.76 H), 6.52 (br, 0.24 H), 7.96 (br, 0.76 H), 8.09 (m, 2 H), 8.31 (s, 1H), 9.45 (br s, 0.24 H), 12.13 (s, 0.76 H, rotamer), 12.39 (br s, 0.24 H, rotamer) | Method 5, Method 49 |
| **111** R | N⁴-(5-Cyclopropyl-1H-pyrazol-3-yl)-N²-(1-thiazol-2-yl-ethyl)-pyrimidine-2,4-diamine | (400 MHz, CD₃CN): 0.71 (m, 2 H), 0.91 (m, 2 H), 1.63 (d, J = 6.8 Hz, 3 H), 1.84 (m, 1H), 5,41 (q, J = 7.2 Hz, 1H), 5.90 (br s, 1H), 6.20 (m, 1H), 6.23 (d, J = 9.0 Hz, 1H), 7.33 (d, J = 1.6 Hz, 1 H), 7.69 (d, J = 2.4 Hz, 1H), 7.88 (d, J = 5.6 Hz, 1H), 7.96 (br s, 1H) | Method 8, Method 52 |
| **112** | (2R)-2-({4-[(3-sec-Butoxy-1H-pyrazol-5-yl)amino]-5-chloropyrimidin-2-yl} amino)-2-(4-fluorophenyl)ethanol | 0.90 (t, 3 H), 1.25 (d, 3H), 1.65 (m, 2 H), 3.65 (m, 2 H), 4.49 (m, 1H), 4.96 (m, 1H), 5.63 (s, 1H), 7.15 (m, 2 H), 7.39 (m, 2 H), 8.03 (s, 1H), 8.43 (br s, 1 H), 10.01 (br s, 1H) | Method 36, Method 104 |
| **113** | (2R)-2-({5-Chloro-4-[(3-propoxy-1H-pyrazol-5-yl)amino]pyrimidin-2-yl} amino)-2-(4-fluorophenyl)ethanol | 0.95 (t, 3 H), 1.70 (q, 2 H), 3.64 (m, 2 H), 4.00 (t, 2 H), 4.94 (m, 1H), 5.61 (s, 1 H), 7.14 (m, 2 H), 7.38 (m, 2 H), 7.99 (s; 1 H), 8.27 (br s, 1H), 9.86 (br s, 1H) | Method 37, Method 104 |
| **114** | (2R)-2-({5-Chloro-4-[(3-isopropoxy-1H-pyrazol-5-yl)amino]pyrimidin-2-yl} amino)-2-(4-fluorophenyl) ethanol | 1.27 (d, 6 H), 3.63 (m, 2 H), 4.67 (m, 1 H), 4.93 (m, 1H), 5.57 (s, 1H), 7.13 (m, 2 H), 7.38 (m, 2 H), 7.98 (s, 1H), 8:21 (br s, 1H), 9.83 (br s, 1H) | Method 38, Method 104 |
| **115** | (2R)-2-({5-Chloro-4-[(3-ethoxy-1H-pyrazol-5-yl)amino]pyrimidin-2-yl} amino)-2-(4-fluorophenyl) ethanol | 1.30 (t, 3 H), 3.63 (m, 2 H), 4.09 (q, 2 H), 4.93 (m, 1H), 5.59 (s, 1H), 7.13 (m, 2 H), 7.38 (m, 2 H), 7.98 (s, 1H), 8.24 (br s, 1H), 9.84 (br s, 1H) | Method 39, Method 104 |
| **116** | 5-Chloro-N⁴-(5-cyclopropyl-1H-pyrazol-3-yl)-N²-[(1S)-3-(dimethylamino)-1-(4-fluoro phenyl)propyl]pyrimidine-2,4-diamine | (CD₃OD): 0.80 (m, 2 H), 1.10 - 1.40 (m, 4 H), 2.00 (m, 1H), 2.18 - 2.40 (m, 2 H), 2.80 (m, 6 H), 5.07 (m, 1H), 6.13 - 6.30 (m, 1H), 6.97 (m, 2 H), 7.30 (d, 2 H), 8.13 (s, 1H) | Method 1, Method 132 |
| **117** | (3S)-3-({5-Chloro-4-[(5-cyclopropyl-1H-pyrazol-3-yl)amino]pyrimidin-2-yl}amino)-3-(4-fluorophenyl)-N,N-dimethylpropanamide | (CD₃OD): 0.60 (m, 2 H), 0.90 (m, 2 H), 1.80 (m, 1H), 2.60 - 2.80 (m, 6 H), 2.95 (m, 2 H), 5.07 (m, 1H), 5.60 (br s, 1H), 6.30 (m, 1H), 6.80 (m, 2 H), 7.30 (d, 2 H), 7.93 (s, 1 H) | Method 1, Method 133 |
| **118** | (3S)-3-({5-Chloro-4-[(5-cyclopropyl-1H-pyrazol-3-yl)amino]pyrimidin-2-yl}amino)-3-(4-fluorophenyl)-N-methylpropanamide | (CD₃OD): 0.90 (m, 2 H), 1.25 (m, 2 H), 2.10 (m, 1H), 2.80 (m, 3 H), 2.95 (m, 2 H), 5.07 (m, 1H), 5.60 (br s, 1H), 6.30 (m, 1H), 7.20 (m, 2 H), 7.40 (d, 2 H), 8.23 (s, 1H) | Method 1, Method 134 |
| **119** | 3-({5-Chloro-4-[(5-cyclopropyl-1H-pyrazol-3-yl)amino]pyrimidin-2-yl}amino)-3-(2-fluorophenyl)propan-1-ol | (CD₃OD): 0.83 (m, 2 H), 1.12 (m, 2 H), 1.90 - 2.03 (m, 3 H), 3.60 (m, 2 H), 5.50 (m, 1 H), 6.39 (s, 1H), 6.98 (m, 2 H), 7.31 (d, 2 H), 8.12 (s, 1H) | Method 1 |
| **120** | (3S)-3-({5-Chloro-4-[(5-cyclopropyl-1H-pyrazol-3-yl)amino]pyrimidin-2-yl}amino)-3-(4-fluorophenyl)-N-(2-hydroxyethyl) propanamide | (CD₃OD): 0.87 (m, 2 H), 1.15 (m, 2 H), 2.03 (m, 1H), 2.90 (m, 2 H), 3.30 (m, 2 H), 3.60 (m, 2 H), 4.40 (m, 1H), 5.50 (m, 1H), 6.29 (s, 1H), 7.10 (m, 2 H), 7.41 (d, 2 H), 8.12 (s, 1 H) | Method 1, Method 135 |
| **121** R | 3-({5-Chloro-4-[(5-cyclopropyl-1H-pyrazol-3-yl)amino]pyrimidin-2-yl} amino)-3-(2-methoxyphenyl) propan-1-ol | (CD₃OD): 0.88 (m, 2 H),1.12 (m, 2 H), 2.03 (m, 1H), 2.11 (m, 2 H), 3.60 (m, 2 H), 3.86 (s, 3 H), 5.50 (m, 1H), 6.39 (s, 1H), 6.92 (m, 1H), 7.01 (d, 1H), 7.16 (m, 1H), 7.30 (m, 1H), 8.12 (s, 1H) | Method 1 |
| **122** R | 3-({5-Chloro-4-[(5-cyclopropyl-1H-pyrazol-3-yl)amino]pyrimidin-2-yl} amino)-3-(2-thienyl)propan-1-ol | (CD₃OD): 0.74 (m, 2 H), 1.02 (m, 2 H), 1.93 (m, 1H), 2.18 (m, 2 H), 3.65 (m, 2 H), 5.57 (m, 1H), 6.23 (s, 1H), 6.97 (m, 2 H), 7.30 (d, 1H), 8.03 (s, 1H) | Method 1 |
| **123** | 5-Chloro-N⁴-(5-cyclopropyl-1H-pyrazol-3-yl)-N²-[(1R)-1-(4-fluorophenyl)-2-morpholin-4-ylethyl]pyrimidine-2,4-diamine | (CD₃OD): 0.80 (m, 2 H), 1.08 (m, 2 H), 1.99 (m, 1H), 3.35 (m,2 H), 3.53 (m, 2 H), 3.76 (m, 2 H), 3.89 (m, 4 H), 5.56 (m, 1H), 6.05 (s, 1H), 7.14 (m, 2 H), 7.43 (m, 2 H), 8.11 (s, 1H) | Method 1, Method 151 |
| **124** | (2R)-2-({5-Fluoro-4-[(5-isopropoxy-1H-pyrazol-3-yl)amino]pyrimidin-2-yl} amino)-2-(4-fluorophenyl)ethanol | (100 °C): 1.35 (m, 6 H), 3.72 (m, 2 H), 4.64 (m, 1H), 4.97 (m, 1H), 5.54 (s, 1 H), 7.09 (m, 2 H), 7.42 (m, 2 H), 7.94 (s, 1 H), 9.90 (br s, 1H) | Method 43, Method 104 |
| **125** | N-[(2R)-2-({4-[(5-Cyclopropyl-1H-pyrazol-3-yl)amino]-5-chloropyrimidin-2-yl}amino)-2-(4-fluorophenyl)ethyl] acetamide | (CD₃OD): 0.60 (m, 2 H), 0.88 (m, 2 H), 1.99 (m, 4 H), 3.65 (m, 2 H, 5.16 (m, 1 H), 6.25 (s, 1 H), 7.04 (m, 2 H), 7.23 (m, 2 H), 8.11 (s, 1H) | Method 1, Method 137 |
| **126** | (2R)-2-({4-[(5-Ethoxy-1H-pyrazol-3-yl)amino]-5-fluoropyrimidin-2-yl) amino)-2-(4-fluorophenyl)ethanol | 1.30 (t, J = 6.0 Hz, 3 H), 3.66 (m, 2 H), 4.09 (m, 2 H), 4.93 (m, 1H, 5.53 (m, 1 H), 7.15 (m, 2 H), 7.37 (m, 2 H), 8.00 (m, 1H), 8.19 - 8.34 (m, 1H), 10.73 - 11.07 (m, 1H) | Method 42, Method 104 |
| **127** | (3S)-3-((4-[(5-Cyclopropyl-1H-pyrazol-3-yl)amino]-5-fluoropyrimidin-2-yl}amino)-3-(4-fluorophenyl)propan-1-ol | 0.68 (m, 2 H), 0.90 (m, 2 H), 1.90 (m, 3 H), 3.40 (m, 2 H), 5.53 (s, 1H), 4.98 (m, 1H), 6.12 (s, 1H), 7.09 (m, 2 H), 7.34 (m, 2 H), 7.80 (s, 1H), 9.31 (bs, 1H), 12.01 (bs, 1H) | Method 13, Method 107 |

| | | | |
|---|---|---|---|
| "R" stands for Reference Example. ¹ Trans-esterification occurred. | | | |

### Example 128

### N²-[(1R)-2-Amino-1-(4-fluorophenyl)ethyl]-5-chloro-N⁴-(5-cyclopropyl-1H-pyrazol-3-yl)pyrimidine-2,4-diamine

A mixture of 2,5-dichloro-4-(5-cyclopropyl-1*H*-pyrazole-3-ylamino)pyrimidine (Method 1; 150 mg, 0.56 mmol) and *tert*-butyl [(2*R*)-2-amino-2-(4-fluorophenyl)ethyl] carbamate (Method 136, 178 mg, 0.70 mmol) in n-butanol was heated at 120°C for 48 hours. Reverse phase HPLC (Gilson) gave the t-butoxycarbonyl protected title compound which was then dissolved in DCM (10 ml) and to it was added trifluoroacetic acid (10 ml) and the mixture was stirred at room temperature for 2 hours. Solvent was evaporated. Reverse phase HPLC (Gilson) gave the desired product that was then transformed to HCl salt. ¹H NMR (CD₃OD): δ 0.80 (m, 2 H), 1.20 (m, 2 H), 1.10 (m, 2 H), 1.95 (m, 1H), 5.25 (m, 1H), 6.10 (m, 2 H), 7.13 (m, 2 H), 7.40 (m, 2 H), 8.13 (s, 1H).

### Example 129

### N⁴-(5-Cyclopropyl-1H-pyrazol-3-yl)-N²-[(1S)-1-(4-fluorophenyl]ethyl]pyrimidine-2,4,5-triamine

A flask with 10% palladium on carbon (66 mg, 0.06 mmol) was evacuated and refilled with H₂ (balloon). To it was added a solution of *N*⁴-(5-cyclopropyl-1*H*-pyrazol-3-yl)-*N*²-[(1*S*)-1-(4-fluorophenyl)ethyl]-5-nitropyrimidine-2,4-diamine (Example 101,120 mg, 0.31 mmol) in EtOH (5 ml). The reaction mixture was stirred at room temperature for 20 hours. Filtration followed by concentration gave the desired product as a solid (100 mg, 91%). ¹H NMR: δ 0.70 (m, 2 H), 0.98 (m, 2 H), 1.47 (m, 3 H), 1.92 (m, 1H), 5.03 (m, 1H), 6.20 (m, 2 H), 7.13 (m, 2 H), 7.40 (m, 2 H), 8.83 (s, 1H), 10.40 (br s, 1H).

### Examples 130

Following a similar procedure to Example 129, the following compound was synthesized via reaction of a suitable aminopyrimidine (method of production of which is also listed) and palladium on activated carbon.

| **Ex.** | **Compound** | **¹H NMR** | **SM** |
|---|---|---|---|
| **130** | (2R)-2-({5-Amino-4-[(5-cyclopropyl-1H-pyrazol-3-yl) amino]pyrimidin-2-yl}amino)-2-(4-fluorophenyl)ethanol | 0.70 (m, 2 H), 0.98 (m, 2 H), 1.47 (m, 3 H), 1.92 (m, 1H), 3.58 (m, 2 H), 4.85 (m, 1H), 6.20 (m, 2 H), 7.13 (m, 2 H), 7.40 (m, 2 H), 8.43 (s, 1H) | Example 100 |

### Example 131

### 4-[(5-Cyclopropyl-1H-pyrazol-3-yl)amino]-2-{(1S)-1-(4-fluorophenyl)ethyl]amino} pyrimidine-5-carbonitrile

To a solution of 5-bromo-N⁴-(3-cyclopropyl-1*H*-pyrazol-5-yl)-N²-[(1*S*)-1-(4-fluorophenyl)ethyl]pyrimidine-2,4-diamine (Example 5, 250 mg, 0.6 mmol) in quinoline (2 ml) was added copper (I) cyanide (75 mg, 0.84 mmol) and the mixture was heated in microwave at 180°C for 5 hours. Reverse phase HPLC (Gilson) purification gave the desired product as a solid (67 mg, 30%). ¹H NMR: δ 0.60 (m, 2 H), 0.90 (m, 2 H), 1.47 (m, 3 H), 1.80 (m, 1H), 5.10 (m, 1H), 5.95 (m, 0.4 H), 6.20 (m, 0.6 H), 7.13 (m, 2 H), 7.35 (m, 2 H), 7.70 (s, 1 H), 8.80 (m, 1H), 10.95 (br s, 1H).

### Example 132

### 5-Chloro-N⁴-(5-cyclopropyl-1H-pyrazol-3-yl)-N²-(1R)-2,2,2-trifluoro-1-(4-fluorophenyl)ethyl]pyrimidine-2,4-diamine

### Example 133

### 5-Chloro-N⁴-(5-cyclopropyl-1H-pyrazol-3-yl)-N²-[(1S)-2,2,2-trifluoro-1-(4-fluorophenyl)ethyl]pyrimidine-2,4-diamine

The title compounds were syntheisised by purification of 5-chloro-*N*⁴-(5-cyclopropyl-1*H*-pyrazol-3-yl)-*N*²-[2,2,2-trifluoro-1-(4-fluorophenyl)ethyl]pyrimidine-2,4-diamine (Example 187) with a chiral HPLC with Diode Array Detection at 220 nm:
Column: Chiralcel OJ, 250 x 20 mm, 10u
Conditions: 50% EtOH 50% MeOH 0.1% diethylamine; Flow rate 10 ml/min
Chiral purity determined using chiral HPLC with Diode Array Detection at 220 nm:
   Column: Chiralcel OJ, 250 x 4.6 mm, 10u
   Conditions: 50% EtOH 50% MeOH 0.1 % diethylamine; Flow rate 0.5 ml/min
Enantiomeric excess (e.e.) > 99% fro each isomer, calculated using area percent at 220 nm for each enantiomer.
(*S*)-isomer: ¹H NMR: δ 0.73 (m, 2 H), 0.94 (m, 2 H), 1.92 (m, 1H), 5.96 (m, 1H), 6.16 (m, 2 H), 7.24 (m, 2 H), 7.63 (m, 2 H), 8.01 (s, 1H), 8.16 (br s, 1H), 8.80 (br s, 1 H).
(*R*)-isomer: ¹H NMR: δ 0.65 (m, 2 H), 0.86 (m, 2 H), 1.80 (m, 1H), 5.90 - 6.10 (m, 2 H), 7.20 (m, 2 H), 7.55 (m, 2 H), 7.95 (s, 1H), 8.40 (br s, 1H), 9.35 (br s, 1H).

### Example 134

### N⁴-(5-Cyclopropyl-1H-pyrazol-3-yl)-N²-[(1S)-1-(4-fluorophenyl)ethyl]-7-(2-methoxyethoxy)quinazoline-2,4-diamine

A mixture of N⁴-(5-cyclopropyl-1*H*-pyrazol-3-yl)-7-fluoro-N²-[(1*S*)-1-(4-fluorophenyl) ethyl]quinazoline-2,4-diamine (Example 77, 25 mg, 0.06 mmol), 2-methoxyethanol (0.18 ml, 2.26 mmol), potassium *tert*-butoxide (60 mg, 0.53 mmol) was heated at 120°C for 18 hours. EtOAc was added to the reaction mixture. The solution was washed with water and was concentrated. Flash column chromatography (pure EtOAc to EtOAc/MeOH = 9:1) provided product as a solid (30 mg, 60 %). ¹H NMR (CD₃OD): 0.73 (m, 2 H), 1.02 (m, 2 H), 1.61 (d, 3 H), 1.95 (m, 1 H), 3.41 (s, 3 H), 3.78 (m, 2 H), 4.26 (m, 2 H), 5.22 (m, 1H), 6.14 (br s, 1H), 6.95 (m, 1H), 7.06 (m, 3 H), 7.34 (m, 2 H), 8.18 (d, 1H).

### Examples 135-140

Following a similar procedure to Example 134, the following compounds were synthesized via reaction of a suitable quinazoline (method of production of which is also listed) and a suitable alcohol.

| **Ex.** | **Compound** | **¹H NMR** | **SM** |
|---|---|---|---|
| **135** | N⁴-(5-Cyclopropyl-1*H*-pyrazol-3-yl)-N²-[(1S)-1-(4-fluoro phenyl)ethyl]-7-(2-morpholin-4-ylethoxy)quinazoline-2,4-diamine | (CDCl₃): 0.7 (m, 2 H), 1.0 (m, 2 H), 1.6 (d, 3 H), 1.9 (m, 1H), 2.5 (m, 4 H), 2.8 (m, 2 H), 3.7 (m, 4 H), 4.1 (m, 2 H), 5.2 (m, 1 H), 6.2 (s, 1H), 6.6 (d, 1H), 6.8 (s, 1H), 7.0 (m, 2 H), 7.4 (m, 2 H), 7.6 (d, 1H) | Example 77 |
| **136** | N⁴-(5-Cyclopropyl-1*H*-pyrazol-3-yl)-N²-[(1S)-1-(4-fluoro phenyl)ethyl]-7-[2-(4-methyl piperazin-1-yl)ethoxy] quinazoline-2,4-diamine | (CD₃OD): 0.7 (m, 2 H), 1.0 (m, 2 H), 1.6 (d 3 H), 2.0 (m, 1H), 2.9 (s, 3 H), 3.2 (m, 4 H), 3.4 (m, 4 H), 3.5 (m, 2 H), 4.4 (m, 2 H), 5.2 (m, 1H), 6.1 (br s, 1 H), 7.1 (m, 4 H), 7.3 (d, 2 H), 7.9 (s, 1H), 8.2 (d, 1H) | Example 77 |
| **137** | (2R)-2-{[4-[(5-Cyclopropyl-1*H*-pyrazol-3-yl)amino]-7-(2-pyrrolidin-1-ylethoxy) quinazolin-2-yl]amino}-2-(4-fluorophenyl)ethanol | (CD₃OD): 0.78 (m, 2 H), 1.06 (d, 2 H), 1.98 (m, 1H), 2.06 (m, 2 H), 2.2 (m, 2 H), 3.27 (m, 2 H), 3.73 (m, 4 H), 3.88 (d, 2 H), 4.51 (d, 2 H), 5.24 (d, 1 H), 6.16 (br s, 1H), 7.05 (m, 4 H), 7.36 (m, 2 H), 8.26 (d, 1H) | Example 78 |
| **138** | (2R)-2-{[4-[(5-Cyclopropyl-1*H*-pyrazol-3-yl)amino]-7-(2-morpholin-4-ylethoxy) quinazolin-2-yl]amino}-2-(4-fluorophenyl)ethanol | (CDCl₃): 0.71 (m, 2 H), 1.05 (m, 2 H), 1.93 (m, 1H), 2:5 (m, 4 H), 3.64 (m, 2 H) 3.72 (m, 2 H), 3.86 (d, 2 H), 4.05 (m, 4 H), 4.57 (m, 2H) 5.23 (br s, 1 H), 6.2 (s, 1 H), 6.15 (d, 1H), 7.1 (m, 4H), 7.36 (m, 2 H), 8.26 (d, 1H) | Example 78 |
| **139** | N⁴-(5-Cyclopropyl-1*H*-pyrazol-3-yl)-N²-[(1S)-1-(4-fluorophenyl)ethyl]-7-(2-pyrrolidin-1-ylethoxy) quinazoline-2,4-diamine | (CD₃OD): 0.67 (m, 2 H), 0.9 (m, 2 H), 1.5 (d, 3 H), 1.8 (m, 4 H), 1.99 (m, 1 H) 2.73 (s, 4 H), 2.9 (m, 2 H), 4.17 (m, 2 H), 5.2 (br s, 1H), 6.78 (m, 2 H), 7.0 (m, 2 H), 7.37 (s, 2 H), 7.91 (d, 1H) | Example 78 |
| **140** | N⁴-(5-Cyclopropyl-1*H*-pyrazol-3-yl)-N²-[(1S)-1-(4-fluorophenyl)ethyl]-6-(2-pyrrolidin-1-ylethoxy) quinazoline-2,4-diamine | (CD₃OD): 0.74 (m, 2 H), 1.04 (m, 2 H), 1.60 (d, 3 H), 1.93 (m, 1H), 1.99 (m, 2 H), 2.00 (m, 2 H), 3.23 (m, 2 H), 3.74 (m, 4 H), 4.51 (d, 2 H), 5.22 (m, 1H), 6.14 (s, 1H), 7.02 (m, 2 H), 7.09 (m, 2 H), 7.33 (m, 2 H), 8.25 (d, 1 H) | Example 81 |

### Example 141

### (2S)-3-[(4-[(5-Cyclopropyl-1H-pyrazol-3-yl)amino]-2-{[(1R)-1-(4-fluorophenyl)-2-hydroxyethyl]amino}quinazolin-7-yl)oxy]propane-1,2-diol

A mixture of (2R)-2-({4-[(5-cyclopropyl-1*H*-pyrazol-3-yl)amino]-7-fluoroquinazolin-2-yl]amino)-2-(4-fluorophenyl)ethanol (Example 78, 50 mg, 0.12 mmol), [(4*R*)-2,2-dimethyl-1,3-dioxolan-4-yl]methanol (0.4 ml) and potassium *tert*-butoxide (100 mg, 0.9 mmol) was stirred at 120°C overnight. Aqueous work up provided a residue. To a solution of this residue in MeOH (2 ml) was added two drops of water and para-toluenesulfonic acid monohydrate (7 mg, 0.037 mmol) and the reaction mixture was stirred at room temperature for 20 hours. Reverse phase HPLC (Gilson) purification gave the desired product as a solid. ¹H NMR: δ 0.70 (m, 2 H), 0.97 (m, 2 H), 1.91 (m, 1H), 3.45 (m, 2 H), 3.60 - 4.00 (m, 4 H), 4.18 (m, 1 H), 5.14 (m, 1 H), 6.09 (m, 1H), 6.90 - 7.50 (m, 6 H), 8.50 (m, 1H), 8.63 (m, 1H), 11.22 (m, 1H), 12.21 (br s, 1 H).

### Example 142

Following a similar procedure to Example 141, the following compound was synthesized via reaction of a suitable quinazoline (method of production of which is also listed).

| **Ex.** | **Compound** | **¹H NMR** | **SM** |
|---|---|---|---|
| **142** | (2R)-3-[(4-[(5-Cyclopropyl-1H-pyrazol-3-yl)amino]-2-{[(1R)-1-(4-fluorophenyl)-2-hydroxyethyl]amino}quinazolin-7-yl)oxy]propane-1,2-diol | 0.70 (m, 2 H), 0.97 (m, 2 H), 1.91 (m, 1 H), 3.45 (m, 2 H), 3.60-4.00 (m, 4 H), 4.18 (m, 1H), 5.14 (m, 1 H); 6.09 (m, 1 H), 6.90 - 7.50 (m, 6 H), 8.50 (m, 1H), 8.63 (m, 1H), 11.22 (m, 1H), 12.21 (br s, 1H) | Example 78 |

### Example 143

### (2R)-2-({5-Chloro-4-[(3-cyclopropyl-1H-pyrazol-5-yl)amino]-6-[(2-morpholin-4-ylethyl) amino]pyrimidin-2-yl}amino)-2-(4-fluorophenyl)ethanol

A mixture of (2-morpholin-4-ylethyl)amine (44 µl, 0.34 mmol), (2*R*)-2-({4,5-dichloro-6-[(3-cyclopropyl-1*H*-pyrazol-5-yl)amino]pyrimidin-2-yl} amino)-2-(4-fluorophenyl)ethanol (Example 84, 70 mg, 0.16 mmol) in 1-butanol (1.0 ml) was heated at 120°C for 18 hours. The solvent was removed and EtOAc was added. The solution was washed with water and was concentrated. Semi-prep HPLC (Gilson system) provided product as a solid (92 mg). ¹H NMR (CDCl₃): 0.67 (m, 2H), 0.92 (m, 2 H), 1.86 (m, 1H), 3.01 (m, 2 H), 3.22 (m, 2 H), 3.46 (m, 2 H), 3.63 (m, 4 H), 3.86 (m, 2 H), 3.98 (m, 2 H), 4.89 (m, 1 H), 5.93 (s, 1 H), 7.14 (m, 2 H), 7.38 (m, 2 H), 7.95 (br s, 1H), 9.15 (br s, 1H), 9.54 (br s, 1H).

### Example 144-176

Following a similar procedure to Example 143, the following compounds were synthesized via reaction of a suitable pyrimidine or quinazoline (method of production of which is also listed) and a suitable amine.

| **Ex.** | **Compound** | **¹H NMR** | **SM** |
|---|---|---|---|
| **144** | 3-[(5-Chloro-6-[(3-cyclopropyl-1H-pyrazol-5-yl)amino]-2-{[(1*R*)-1-(4-fluorophenyl)-2-hydroxyethyl]amino}pyrimidin-4-yl)amino]propane-1,2-diol | 0.69 (m, 2 H), 0.98 (m, 2 H), 1.93 (m, 1 H), 3.13-3.65 (m, 7 H), 4.96 (m, 1 H), 5.86 (s, 1 H), 7.13 (m, 2 H), 7.39 (m, 2 H), 8.67 (br s, 1H), 9.73 (br s, 1 H) | Example 84 |
| **145** | 3-[(5-Chloro-6-[(3-cyclopropyl-1H-pyrazol-5-yl)amino]-2-{[(1*R*)-1-(4-fluorophenyl)-2-hydroxyethyl]amino}pyrimidin-4-yl)amino]propan-1-ol | 0.69 (m, 2 H), 0.98 (m, 2 H), 1.51 (m, 2 H), 1.91 (m, 1H), 3.30-3.64 (m, 6 H); 4.92 (m, 1H), 5.86 (s, 1H), 7.13 (m, 2 H), 7.36 (m, 2 H), 7.64 (br s, 1 H), 8.65 (br s, 1H), 9.68 (br s, 1H) | Example 84 |
| **146** | (2*R*)-2-[(5-Chloro-4-{(3-cyclopropyl-1*H*-pyrazol-5-yl)amino]-6-{[3-(4-methylpiperazin-,1-yl)propyl]amino} pyrimidin-2-yl)amino]-2-(4-fluorophenyl)ethanol | 0.69 (m, 2 H), 0.96 (m, 2 H), 1.63 (m, 2 H), 1.93 (m, 1 H), 2.77 (m, 5 H), 3.17-3.33 (m, 10 H), 3.66 (m, 2 H), 4.92 (m, 1H), 5.88 (s, 1H), 7.15 (m, 2 H), 7.39 (m, 2 H), 7.61 (br s, 1H), 8.53 (br s, 1H), 9.67 (br s, 1 H) | Example 84 |
| **147** | (2*R*)-2-({5-Chloro-4-[(3-cyclopropyl-1*H*-pyrazol-5-yl)amino]-6-[(2-pyrrolidin-1-ylethyl)amino]pyrimidin-2-yl} amino)-2-(4-fluoropheiiyl) ethanol | 0.67 (m, 2 H), 0.91 (m, 2 H), 1.88 (m, 5 H), 2.80-3.63 (m, 10 H), 4.89 (m, 1 H), 5.93 (s, 1H), 7.14 (m, 2 H), 7.38 (m, 2 H), 7.95 (br s, 1H), 9.2 (br s, 1 H), 9.28 (br s, 1H) | Example 84 |
| **148** | 6-[(3-Cyclopropyl-1*H*-pyrazol-5-yl)amino]-2-{[(1*R*)-1-(4-fluorophenyl)-2-hydroxyethyl]amino}-N-(2-morpholin-4-ylethyl)pyrimidine-4-carboxamide | 0.69 (m, 2 H), 0.93 (m, 5 H), 1.86 (m, 1 H), 3.12 (m, 2 H), 3.64-3.96 (m, 12 H), 5.03 (m, 1H), 5.34 (s, 1H), 6.03 (s, 1H), 6.68 (s, 1 H), 7.15 (m, 2 H), 7.41 (m, 2 H). 8.66 (br s, 1 H), 9.08 (br s, 1H), 9.73 (br s, 1H), 9.97 (br s, 1H) | Example 28 |
| **149** | (2*R*)-3-[(6-[(5-Cyclopropyl-1*H-* pyrazol-3-yl)amino]-2-{[(1*S*)-1-(4-fluorophenyl)ethyl]amino}pyrimidin-4-yl)amino]propane-1,2-diol | 0.65 (m, 2 H), 0.90 (m, 2 H), 1.45 (m, 3H), .1.80 (m, 1H), 3.10-3.50 (m, 5 H), 4.95 (br s, 1 H), 5.35 (s, 1H), 5.45 (m, 1H), 7.10 (m, 2 H), 7.35 (m, 2 H), 8.10 (s, 1H), 8.70 (s, 1H), 10.20 (br s, 1H), 11.45 (br s, 1H) | Example 38 |
| **150** | (2*R*)-3-({2-{[(1*S*)-1-(4-Fluorophenyl)ethyl]amino}-6-[(5-methyl-1*H*-pyrazol-3-yl)amino]pyrimidin-4-yl}amino)propane-1,2-diol | 1.40 (m, 3 H), 2.15 (s, 3 H), 3.10-3.90 (m, 5 H), 4.95 (br s, 1H), 5.35 (s, 1 H), 5.65 (m, 1 H), 7.10 (m, 2 H), 7.35 (m, 2 H), 8.10 (s, 1H), 8.78 (s, 1 H), 10.20 (br s, 1 H), 11.55 (br s, 1 H) | Example 190 |
| **151** | 2-[(6-[(5-Cyclopropyl-1*H* pyrazol-3-yl)amino]-2-{[(1*S*)-1-(4-fluorophenyl)ethyl]amino}pyrimidin-4-yl)amino]ethanol | 0.65 (m, 2 H), 0.85 (m, 2 H), 1.45 (m, 3 H), 1.80 (m, 1 H), 3.10-3.50 (m, 4 H), 4.95 (br s, 1H), 5.35. (s, 1H), 5.55 (m, 1 H), 7.10 (m, 2 H), 7.35 (m, 2 H), 8.10 (s, 1H), 8.72 (s, 1H), 10.20 (br s, 1H), 11.45 (br s, 1H). | Example 38 |
| **152** | 2-({2-{[(1*S*)-1-(4-Fluorophenyl) ethyl]amino}-6-[(5-methyl-1*H* pyrazol-3-yl)amino]pyrimidin-4-yl}amino)ethanol | 1.40 (m, 3 H), 2.20 (s, 3 H), 3.10 - 3.50 (m, 4 H), 4.90 (s, 1H), 5.35 (s, 1 H), 5.71 (m, 1H), 7.10 (m, 2 H), 7.35 (m, 2 H), 8.10 (s, 1H), 8.78 (s, 1H), 10.22 (br s, 1H), 11.50 (br s, 1H) | Example 190 |
| **153** | 5-Chloro-N⁴-(5-cyclopropyl-1H-pyrazol-3-yl)-N²-[(1S)-(4-fluoro-phenyl)-ethyl]-pyrimidine-2,4,6-triamine | 0.60 (m, 2 H), 0.82 (m, 2 H), 1.41 (d, J = 7.2 Hz, 3 H), 1.83 (m, 1 H), 4.99 (m, 1H), 5.67 (br s, 1H), 6.11-6.29 (m, 2.5 H), 6.81 (br s, 0.5 H), 7.11 (m, 2 H), 7.38 (m, 2.5 H), 7.56 (br s, 0.5 H), 8.97 (br s, 0.5 H), 11.90 (s, 0.5 H), 12.40 (br s, 0.5 H) | Example 39 |
| **154** | 5-Chloro-N⁴-(5-cyclopropyl-1H-pyrazol-3-yl)-N²-[(1S)-(4-fluoro-phenyl)-ethyl]-6-(4-methyl-piperazin-1-yl)-pyrimidine-2,4-diamine | 0.71 (m, 2 H), 0.91 (m, 2 H), 1.51 (d, J = 6.4 Hz, 3 H), 1.86 (m, 1H), 2.31 (s, 3 H), 2.46 (m, 4 H), 3.46 (m, 4 H), 4.96 (m, 1 H), 5.08 (d, J = 6.4 Hz, 1 H), 5.83 (br, 1H), 7.01 (m, 2 H), 7.29 (m, 3 H), | Example 39 |
| **155** | 1-Amino-3-[(5-chloro-6-[(3-cyclopropyl-1H-pyrazol-5-yl)amino]-2-{[(1S)-1-(4-fluorophenyl)ethyl]amino}pyrimidin-4-yl)amino]propan-2-ol | 0.63 (m, 2 H), 090 (m, 2 H), 1.41 (d, 3 H), 1.85 (m, 1 H), 2.82 (m, 2 H), 3.51 (m, 2 H), 3.83 (m, 1 H), 4.95 (m, 1 H), 5.58 (br s, 1H), 5.90 (s, 1 H), 6.94 (s, 1H), 7.11 (m, 2 H), 7.28 (s, 1 H), 7.39 (m, 2 H), 7.72 (m, 2 H) | Example 39 |
| **156** | (2R)-2-[(5-Chloro-4-[(3-cyclopropyl-1H-pyrazol-5-yl)amino]-6-{[2-(dimethylamino)ethyl]amino} pyrimidin-2-yl)amino]-2-(4-fluorophenyl)ethanol | 0.67 (m, 2 H), 0.92 (m, 2 H),1.87 (m, 1 H), 2.59 (s, 3 H), 2.75 (s, 3 H), 2.83 - 3.57 (m, 4 H), 3.62 (m, 2 H), 4.90 (m, 1H), 5.92 (s, 1H), 7.13 (m, 2 H), 7.37 (m, 2 H), 7.93 (br s, 1H), 9.16 (m, 2 H) | Example 84 |
| **157** | (2R)-2-({5-Chloro-4-[(3-cyclopropyl-1H-pyrazol-5-yl)amino]-6-[(3-pyrrolidin-1-ylpropyl)amino]pyrimidin-2-yl}amino)-2-(4-fluorophenyl) ethanol | 0.67 (m, 2 H), 0.95 (m, 2 H), 1.83 - 1.97 (m, 7 H), 2.85 - 3.64 (m, 10 H), 4.92 (m, 1H), 5.89 (s, 1H), 7.15 (m, 2 H), 7.38 (m, 2 H), 8.26 (br s, 1 H), 9.41 (br s, 1H), 9.56 (br s, 1H) | Example 84 |
| **158** | 2-[(5-Chloro-6-[(3-cyclopropyl-1H-pyrazol-5-yl)amino]-2-{[(1S)-1-(4-fluorophenyl)ethyl]amino}pyrimidin-4-yl)amino] ethanol | 0.68 (m, 2 H), 0.96 (m, 2 H), 1.45 (d, 3 H), 1.90 (m, 1H), 3.37 - 3.54 (m, 4 H), 4.97 (m, 1H), 5.85 (s, 1 H), 7.14 (m, 2 H), 7.39 (m, 2 H), 7.47 (br s, 1 H), 8.59 (br s, 1H), 9.72 (br s, 1H) | Example 39 |
| **159** | 2-[(5-Chloro-6-[(3-cyclopropyl-1H-pyrazol-5-yl)amino]-2-{[(1S)-1-(4-fluorophenyl)ethyl]amino}pyrimidin-4-yl)amino]propane-1,3-diol | 0.68 (m, 2 H), 0.96 (m, 2 H), 1.45 (d, 3 H), 1.91 (m, 1H), 3.33 - 3.56 (m, 4 H), 4.04 (m, 1H), 4.98 (m, 1H), 5.86 (s, 1H), 6.68 (br s, 1 H), 7.14 (m, 2 H), 7.42 (m, 2 H), 8.54 (br s, 1H), 9.73 (br s, 1H) | Example 39 |
| **160** | (2R)-2-{[5-Chloro-4-[(3-cyclopropyl-1H-pyrazol-5-yl)amino]-6-(dimethylamino)pyrimidin-2-yl]amino}-2-(4-fluorophenyl)ethanol | 0.68 (m, 2 H), 0.96 (m, 2 H), 1.90 (m, 1 H), 3.00 (s, 6 H), 3.61 (m, 2 H), 4.88 (m, 1H), 5.94 (s, 1H), 7.13 (m, 2 H), 7.37 (m, 2 H), 8.19 (br s, 1H), 9.47 (br s, 1H). | Example 84 |
| **161** | 1-Amino-3-[(5-chloro-6-[(3-cyclopropyl-1H-pyrazol-5-yl)amino]-2-{[(1R)-1-(4-fluorophenyl)-2-hydroxyethyl]amino}pyrimidin-4-yl)amino]propan-2-ol | 0.69 (m, 2 H), 0.96 (m, 2 H), 1.92 (m, 1 H), 2.82 (m, 2 H), 3.24 - 3.65 (m, 5 H), 4.96 (m, 1H), 5.89 (s, 1H), 7.14 (m, 2 H), 7.41 (m, 2 H), 7.76 (b, 2 H), 8.43 (br s, 1H), 9.62 (br s, 1H) | Example 84 |
| **162** | 2-[(5-Chloro-6-[(5-cyclopropyl-1H-pyrazol-3-yl)amino]-2-{[(1R)-1-(4-fluorophenyl)-2-hydroxyethyl]amino]pyrimidin-4-yl)amino]propane-1,3-diol | 0.69 (m, 2 H), 0.97 (m, 2 H), 1.92 (m, 1 H), 3.28 - 3.66 (m, 5 H), 3.98 (m, 2 H), 4.93 (m, 1H), 5.87 (s, 1H), 6.62 (br s, 1H), 7.13 (m, 2 H), 7.39 (m, 2 H), 8.54 (br s, 1H), 9.64 (br s, 1H) | Example 84 |
| **163** | (2R)-2-[(5-Chloro-4-[(3-cyclopropyl-1H-pyrazol-5-yl)amino]-6-{[2-(2-hydroxyethoxy)ethyl]amino}pyrimidin-2-yl)amino]-2-(4-fluorophenyl) ethanol | 0.69 (m, 2 H), 0.97 (m, 2 H), 1.92 (m, 1 H), 3.41 - 3.64 (m, 10 H), 4.89 (m, 1 H), 5.87 (s, 1 H), 7.14 (m, 2 H), 7.35 (m, 2 H), 7.50 (br s, 1H), 8.57 (br s, 1 H), 9.67 (br s, 1 H) | Example 84 |
| **164** | (2R)-3-[(5-Chloro-6-[(3-cyclopropyl-1H-pyrazol-5-yl)amino]-2-{[(1S)-1-(4-fluorophenyl)ethyl]amino}pyrimidin-4-yl)amino]propane-1,2-diol | 0.69 (m, 2 H), 0.98 (m, 2 H), 1.45 (d, 3 H), 1.93 (m, 1 H), 3.30 - 3.54 (m, 5 H), 5.02 (m, 1 H), 5.86 (s, 1 H), 7.14 (m, 2 H), 7.42 (m, 2 H), 8.67 (br s, 1 H), 9.81 (br s, 1 H) | Example 39 |
| **165** | (2R)-2-{[5-Chloro-4-[(3-cyclopropyl-1H-pyrazol-5-yl)amino]-6-(ethylamino) pyrimidin-2-yl]amino}-2-(4-fluorophenyl)ethanol | 0.69 (m, 2 H), 0.98 (m, 5 H), 1.93 (m, 1 H), 3.20 - 3.64 (m, 4 H), 4.91 (m, 1 H), 5.86 (s, 1 H), 7.14 (m, 2 H), 7.36 (m, 2 H), 7.72 (br s, 1 H), 8.70 (br s, 1 H), 9.73 (br s, 1 H) | Example 84 |
| **166** | (2S)-3-[(5-Chloro-6-[(3-cyclopropyl-1H-pyrazol-5-yl)amino]-2-{[(1R)-1-(4-fluorophenyl)-2-hydroxyethyl]amino}pyrimidin-4-yl)amino]propane-1,2-diol | 0.69 (m, 2 H), 0.99 (m, 2 H), 1.93 (m, 1 H), 3.10 (m, 1 H), 3.32 (m, 2 H), 3.45 (m, 2 H), 3.65 (m, 2 H), 4.96 (m, 1 H), 5.86 (s, 1 H), 7.13 (m, 2 H), 7.39 (m, 2 H), 7.47 (br s, 1 H), 8.73 (br s, 1 H), 9.78 (br s, 1 H) | Example 84 |
| **167** | (2R)-3-[(5-Chloro-6-[(3-cyclopropyl-1H-pyrazol-5-yl)amino]-2-{[(1R)-1-(4-fluorophenyl)-2-hydroxyethyl]amino}pyrimidin-4-yl)amino]propane-1,2-diol | 0.70 (m, 2 H), 0.97 (m, 2 H), 1.91 (m, 1 H), 3.54 (m, 7 H), 4.97 (m, 1 H), 5.86 (s, 1 H), 7.13 (m, 2 H), 7.38 (m, 2 H), 7.47 (br s, 1 H), 8.73 (br s, 1 H), 9.80 (br s, 1 H) | Example 84 |
| **168** | (2R)-2-({5-Chloro-4-[(3-cyclopropyl-1H-pyrazol-5-yl)amino]-6-[(2-hydroxyethyl)amino]pyrimidin-2-yl}amino)-2-(4-fluorophenyl)ethanol | 0.70 (m, 2 H), 0.97 (m, 2 H), 1.93 (m, 1 H), 3.33 (m, 4 H), 3.65 (m, 2 H), 4.92 (m, 1 H), 5.86 (s, 1 H), 7.14 (m, 2 H), 7.37 (m, 2 H), 7.57 (br s, 1 H), 8.72 (br s, 1H), 9.78 (br s, 1 H) | Example 84 |
| **169** | (2R)-2-{[5-Chloro-4-[(3-cyclopropyl-1H-pyrazol-5-yl)amino]-6-(methylamino)pyrimidin-2-yl]amino}-2-(4-fluorophenyl)ethanol | 0.70 (m, 2 H), 0.97 (m, 2 H), 1.94 (m, 1 H), 2.78 (s, 3 H), 3.67 (m, 2 H), 4.97 (m, 1 H), 5.85 (s, 1H), 7.15 (m, 2 H), 7.38 (m, 2 H), 7.72 (br s, 1 H), 8.80 (br s, 1 H), 9.82 (br s, 1 H) | Example 84 |
| **170** | (2S)-1-[(5-Chloro-6-[(3-cyclopropyl-1H-pyrazol-5-yl)amino]-2-{[(1S)-1-(4-fluorophenyl)ethyl]amino} pyrimidin-4-yl)amino]propan-2-ol | 0.69 (m, 2 H), 0.96 (m, 5 H), 1.45 (d, 3 H), 1.94 (m, 1 H), 3.11 (m, 1 H), 3.29 (m, 1 H), 3.54 (m, 1 H), 4.99 (m, 1 H), 5.86 (s, 1H), 7.14 (m, 2 H), 7.38 (m, 2 H), 7.55 (br s, 1 H), 8.71 (br s, 1 H), 9.89 (br s, 1H) | Example 39 |
| **171** | 3-[(5-Chloro-6-[(3-cyclopropyl-1H-pyrazol-5-yl)amino]-2-{[(1S)-1-(4-fluorophenyl)ethyl]amino}pyrimidin-4-yl)amino]-1,1,1-trifluoropropan-2-ol | 0.69 (m, 2H), 0.96 (m, 2 H), 1.44 (d, 3 H), 1.93 (m, 1 H), 3.27 (m, 1 H), 3.60 (m, 1 H), 4.20 (m, 1 H), 4.96 (m, 1 H), 5.89 (s, 1 H), 6.40 (br s, 1 H), 7.11 (m, 2 H), 7.34 (m, 2 H), 7.50 (br s, 1 H), 8.44 (br s, 1 H), 9.67 (br s, 1 H) | Example 39 |
| **172** | 3-[(5-Chloro-6-[(3-cyclopropyl-1H-pyrazol-5-yl)amino]-2-{[(1S)-1-(4-fluorophenyl)ethyl]amino}pyrimidin-4-yl)(methyl)amino]propane-1,2-diol | 0.67 (m, 2 H), 0.92 (m, 2 H), 1.42 (d, 3 H), 1.87 (m, 1 H), 3.15 (s, 3 H), 3.29 - 3.98 (m, 5 H), 4.95 (m, 1 H), 5.94 (s, 1 H), 7.12 (m, 2 H), 7.38 (m, 2 H) | Example 39 |
| **173** | 5-Chloro-N⁴-(5-cyclopropyl-1H-pyrazol-3-yl)-N²-[(1S)-1-(4-fluorophenyl)ethyl]-6-morpholin-4-ylpyrimidine-2,4-diamine | 0.70 (m, 2 H), 0.96 (m, 2 H), 1.42 (d, 3 H), 1.92 (m, 1H), 3.42 (m, 4 H), 3.58 (m, 4 H), 4.92 (m, 1 H), 6.00 (s, 1 H), 7.12 (m, 2 H), 7.37 (m, 2 H), 8.07 (br s, 1 H), 9.59 (br s, 1 H) | Example 39 |
| **174** | (2R)-2-({5-Chloro-4-[(5-cyclopropyl-1H-pyrazol-3-yl)amino]-6-morpholin-4-ylpyrimidin-2-yl}amino)-2-(4-fluorophenyl)ethanol | 0.70 (m, 2 H), 0.95 (m, 2 H), 1.91 (m, 1 H), 3.38 (m, 4 H), 3.62 (m, 6 H), 4.86 (m, 1 H), 6.00 (s, 1 H), 7.12 (m, 2 H), 7.35 (m, 2 H), 8.01 (br s, 1 H), 9.45 (br s, 1 H) | Example 84 |
| **175** | (2R)-2-{[5-Chloro-4-[(5-cyclopropyl-1H-pyrazol-3-yl)amino]-6-(4-methylpiperazin-1-yl)pyrimidin-2-yl]amino}-2-(4-fluorophenyl)ethanol | 0.69 (m, 2 H), 0.92 (m, 2 H), 1.88 (m, 1 H), 2.77 (m, 4 H), 3.10 (s, 3 H), 3.40 (m, 2 H), 3.62 (m; 2 H), 3.99 (m, 2 H), 4.85 (m, 1 H), 6.08 (s, 1 H), 7.11 (m, 2 H), 7.36 (m, 2 H), 7.72 (br s, 1H), 9.12 (br s, 1 H), 9.98 (br s, 1 H) | Example 84 |
| **176** | 5-Chloro-N⁴-(5-cyclopropyl-1H-pyrazol-3-yl)-N²-[(1S)-1-(4-fluorophenyl)ethyl]-6-pyrrolidin-1-ylpyrimidine-2,4-diamine | 0.69 (m, 2 H), 0.98 (m, 2 H), 1.44 (d, 3 H), 1.82 (m, 4 H), 1.93 (m, 1 H), 3.59 (m, 2 H), 3.71 (m, 2 H), 4.96 (m, 1 H), 5.90 (s, 1 H), 7.15 (m, 2 H), 7.39 (m, 2 H), 8.55 (br s, 1 H), 9.79 (br s, 1 H) | Example 39 |

### Example 177

### (2R)-3-[(5-Chloro-6-[(3-ethoxy-1H-pyrazol-5-yl)amino]-2-{[(1S)-1-(4-fluorophenyl)ethyl]amino}pyrimidin-4-yl)amino]propane-1,2-diol

A mixture of 5,6-dichloro-*N*⁴-(5-ethoxy-1*H*-pyrazol-3-yl)-*N*²-[(1*S*)-1-(4-fluorophenyl)ethyl]pyrimidine-2,4-diamine (Example 191; 188 mg, 0.46 mmol), (2R)-3-aminopropane-1,2-diol (96 mg, 1.05 mmol) in *n*-butanol (2.5 ml) was heated at 112°C for 2 days. The mixture was concentrated. Reverse phase HPLC (Gilson) purification gave the title compound (18 mg). ¹H NMR (CDCl₃): δ 1.29 (t, 3H), 1.40 (d, 3 H), 3.18 - 3.61 (m, 5 H), 4.08 (q, 2 H), 4.93 (m, 1H), 5.44 (s, 1 H), 6.59 (br s, 1H), 7.11 (m, 2 H), 7.38 (m, 2H), 7.86 (br s, 1 H), 9.27 (br s, 1H).

### Example 178-181

Following a similar procedure to Example 177, the following compounds were synthesized via reaction of a suitable pyrimidine (method of production of which is also listed) and a suitable amine.

| **Ex.** | **Compound** | **¹H NMR** | **SM** |
|---|---|---|---|
| **178** | (2R)-3-({5-Chloro-2-{[(1S)-1-(4-fluorophenyl)ethyl]amino}-6-[(3-isopropoxy-1H-pyrazol-5-yl)amino]pyrimidin-4-yl}amino)propane-1,2-diol | 1.27 (d, 6 H), 1.41 (d, 3 H), 3.19 - 3.51 (m, 5 H), 4.60 (m, 1 H), 4.94 (m, 1H), 5.44 (s, 1 H), 6.69 (br s, 1 H), 7.11 (m, 2 H), 7.39 (m, 2 H), 7.96 (br s, 1 H), 9.34 (br s, 1 H) | Example 192 |
| **179** | (2R)-3-({5-Chloro-2-{[(1R)-1-(4-fluorophenyl)-2-hydroxyethyl]amino}-6-[(3-isopropoxy-1H-pyrazol-5-yl)amino]pyrimidin-4-yl}amino)propane-1,2-diol | 1.27 (d, 6 H), 3.17-3.49 (m, 5 H), 3.62 (m, 2 H), 4.62 (m, 1 H), 4.91 1 (m, 1 H), 5.45 (s, 1 H), 6.67 (br s, 1 H), 7.10 (m, 2 H), 7.36 (m, 2 H), 7.99 (br s, 1 H), 9.32 (br s, 1 H) | Example 194 |
| **180** | 2-({5-Chloro-2-{[(1S)-1-(4-fluorophenyl)ethyl]amino}-6-[(5-isopropoxy-1H-pyrazol-3-yl)amino]pyrimidin-4-yl}amino)propane-1,3-diol | 1.30 (d, 6 H), 1.46 (d, 3 H), 3.33 - 3.55 (m, 4 H), 4.04 (m, 1 H), 4.55 (m, 1 H), 4.98 (m, 1 H), 5.63 (s, 1 H), 6.70 (br s, 1H), 7.13 (m, 2 H), 7.40 (m, 2 H), 8.81 (br s, 1 H), 9.97 (br s, 1 H) | Example 192 |
| **181** | 2-({5-Chloro-2-{[(1R)-1-(4-fluorophenyl)-2-hydroxyethyl] amino}-6-[(5-isopropoxy-1H-pyrazol-3-yl)amino]pyrimidin-4-yl}amino)propane-1,3-diol | 1.27 (d, 6 H), 3.27 (m, 1H), 3.38 (m, 1 H), 3.51 (m, 2 H), 3.61 (m, 2 H), 3.93 (m, 1 H), 4.61 (m, 1 H), 4.88 (m, 1 H), 5.42 (s, 1 H), 5.90 (bs, 1 H), 7.10 (m, 2 H), 7.37 (m, 2 H), 7.87 (br s, 1 H), 9.24 (br s, 1 H), 11.95 (br s, 1 H) | Example 193 |

| | | | |
|---|---|---|---|
| "R" stands for Reference Example. | | | |

### Example 182

### N⁴-(5-Cyclopropyl-2H-pyrazol-3-yl)-N²-[(S)-1-(4-fluorophenyl)-ethyl]-5,6,7,8-tetrahydropyrido[4,3-d]pyrimidine-2,4-diamine

4-(5-Cyclopropyl-1*H*-pyrazol-3-ylamino)-2-[(*S*)-1-(4-fluoro-phenyl)-ethylamino]-7,8-dihydro-5*H*-pyrido[4,3-*d*]pyrimidine-6-carboxylic acid benzyl ester (Example 188; 0.06 g, 0.11 mmol) was dissolved in absolute EtOH (4 ml), to which was added Pd/C (0.012 g, 0.005 mmol). The reaction mixture was then purged with N₂, evacuated, purged with H₂, and stirred under H₂ at atmospheric pressure for 15 hours. The Pd/C was removed by filtration and washed with MeOH (2 x 2 ml). The filtrate was concentrated under reduced pressure and purified by column chromatography (DCM: MeOH = 20: 1) to give the title compound as a white solid (0.02 g, 44%). ¹H NMR (400 MHz, CDCl₃) δ 0.62 - 0.66 (m, 2 H), 0.87 - 0.91 (m, 2 H), 1.47 (d, J= 6.4 Hz, 3 H), 1.73 - 1.81 (m, 1 H), 2.51 - 2.59 (m, 2 H), 2.99 - 3.09 (m, 2 H), 3.58 - 3.67 (m, 2 H), 4.99 - 5.06 (m, 1 H), 5.19 (br s, 2 H), 5.89 (br s, 1 H), 6.74 (br s, 1 H), 6.94 - 6.98 (m, 2 H), 7.29 - 7.33 (m, 2 H). MS: Calcd.: 393; Found: [M+H]⁺394.

### Example 183

### N⁴-(5-Gyclopropyl-1H-pyrazol-3-yl)-N²-[(S)-1-(4-fluoro-phenyl)-ethyl]-5,6,7,8-tetrahydropyrido[2,3-d]pyrimidine-2,4-diamine

To an EtOH (20 ml) solution of N⁴-(5-cyclopropyl-1*H*-pyrazol-3-yl)-N²-[(1*S*)-1-(4-fluorophenyl)ethyl]pyrido[2,3-d]pyrimidine-2,4-diamine (Example 65; 0.3 g, 0.77 mmol) was added platinum oxide (0.017 g, 0.077 mmol). The reaction was then purged with N₂, evacuated, and then purged with H₂, and stirred for 15 hours. The reaction was then diluted with MeOH (20 ml) and filtered to remove platinum. The filtrate was concentrated under reduced pressure and purified by column chromatography (DCM: MeOH = 40: 1) to give the title compound as a solid (0.25 g, 83%). ¹H NMR (400 MHz, CDCl₃) δ 0.62 - 0.66 (m, 2 H), 0.84 - 0.87 (m, 2 H), 1.44 (d, *J*= 6.4 Hz, 3 H), 1.79 - 1.84 (m, 1 H), 1.85 - 1.91 (m,2 H), 2.30 (t, *J*= 6.2 Hz, 2 H), 3.24 - 3.26 (m, 2 H), 4.79 (br s, 1 H), 4.98 (br s, 2 H), 6.54 (br s, 1H), 6.93 - 6.97 (m, 2 H), 7.29 - 7.33 (m, 2 H). MS: Calcd.: 393; Found: [M+H]⁺ 394.

### Example 184

### N⁴-(5-Cyclopropyl-1H-pyrazol-3-yl)-N²-[(S)-1-(4-fluoro-phenyl)-ethyl]-5,6,7,8-tetrahydropyrido[3,4-d]pyrimidine-2,4-diamine

4-(5-Cyclopropyl-1*H*-pyrazol-3-ylamino)-2-[(*S*)-1-(4-fluoro-phenyl)-ethylamino]-5,8-dihydro-6*H*-pyrido[3,4-*d*]pyrimidine-7-carboxylic acid benzyl ester (Example 189; 0.7 g, 1.3 mmol) was dissolved in absolute EtOH (20 ml), and Pd/C (0.28 g, 0.13 mmol) was added. The reaction was then purged with N₂, evacuated, purged with H₂, and stirred for 15 hours. The reaction was then filtered to remove the palladium, washed with MeOH, concentrated, and purified by column chromatography (DCM: MeOH = 20: 1) to give the title compound (0.50 g, 95%). ¹H NMR (400 MHz, CDCl₃) δ 0.67 - 0.69 (m, 2 H), 0.92 - 0.95 (m, 2 H), 1.51 1 (d, *J*= 6.8 Hz, 3H), 1.80 - 1.88 (m, 1H), 2.32 - 2.37 (m, 2 H), 3.12 - 3.15 (m, 2H), 3.70 - 3.78 (m, 2H), 5.03 - 5.10 (m, 1H), 5.19 - 5.21 (m, 1H), 5.91 (br s, 1H), 6.73 (br s, 1 H), 6.98 - 7.02 (m, 2H), 7.34 - 7.38 (m, 2 H). MS: Calcd.: 393; Found: [M+H]⁺394.

### Example 185

### 1-{4-(5-Cyclopropyl-1H-pyrazol-3-ylamino)-2-[(S)-1-(4-fluoro-phenyl)-ethylamino]-7,8-dihydro-5H-pyrido[4,3-d]pyrimidin-6-yl}-ethanone

A solution of *N*⁴-(5-cyclopropyl-2*H*-pyrazol-3-yl)-*N*²-[(*S*)-1-(4-fluoro-phenyl)-ethyl]-5,6,7,8-tetrahydro-pyrido[4,3-*d*]pyrimidine-2,4-diamine (Example 182, 0.03 g, 0.08 mmol) in DCM-THF (3 ml, 1 : 1, v/v) was agitated together with acetic acid loaded TFP (tetrafluorophenyl) resin (1.4 mmol/g, 1.0 eq.) for 40 minutes. The resin was filtered and washed with DCM (2 x 5 ml). The combined organic was concentrated and purified by prep. TLC (DCM: MeOH = 15: 1) to give the title compound (0.027 g, 81%). ¹H NMR (400 MHz, CDCl₃) δ 0.71 - 0.72 (m, 2 H), 0.90 - 0.92 (m, 2 H), 1.47 (d, *J*= 6.4 Hz, 3 H), 1.84 - 1.89 (m, 1 H), 2.20 (s, 3 H), 2.63 - 2.73 (m, 2 H), 3.56 - 3.77 (m, 2 H), 4.43 (d, J = 15.8 Hz, 1 H), 4.70 (d, J = 15.8 Hz, 1 H), 5.07 - 5.15 (m, 2 H), 5.99 (br s, 1 H), 6.94 - 6.99 (m, 2 H), 7.24 - 7.32 (m, 2 H), 9.28 (br s, 1 H). MS: Calcd.: 435; Found: [M+H]⁺436.

### Example 186

### 1-{4-(5-Cyclopropyl-1H-pyrazol-3-ylamino)-2-[(S)-1-(4-fluoro-phenyl)-ethylaminol-5,8-dihydro-6H-pyrido[3,4-d]pyrimidin-7-yl}-ethanone

A solution of *N*⁴-(5-cyclopropyl-1*H*-pyrazol-3-yl)- *N*²-[(*S*)-1-(4-fluoro-phenyl)-ethyl]-5,6,7,8-tetrahydro-pyrido[2,3-*d*]pyrimidine-2,4-diamine (Example 184; 0.05 g, 0.13 mmol) in DCM: THF (3 ml, 1 : 1, v/v) was agitated together with acetic acid loaded TFP resin (1.4 mmol/g, 1.0 eq.) for 40 minutes. The resin was filtered and washed with DCM (2 x 5 ml). The combined organic was concentrated and purified by prep. TLC (DCM: MeOH = 13: 1) to give the title compound (0.023 g, 41%). ¹H NMR (400 MHz, CDCl₃) δ 0.68 - 0.70 (m, 2 H), 0.93 - 0.95 (m, 2H), 1.52 (d, J= 6.2 Hz, 3 H), 1.82 - 1.85 (m, 1H), 2.14 (s, 3H), 2.36 - 2.46 (m, 2 H), 3.65 - 3.90 (m, 2H), 4.31 - 4.49 (m, 2H), 5.07 - 5.17 (m, 2H), 6.05 (br s, 1H), 6.96 - 7.01 (m, 2 H), 7.30 - 7.36 (m, 2 H). MS: Calcd.: 435; Found: [M+H]⁺ 436.

### Example 187

### 5-Chloro-N⁴-(5-cyclopropyl-1H-pyrazol-3-yl)-N²-[2,2,2-trifluoro-1-(4-fluorophenyl)ethyl] pyrimidine-2,4-diamine

The title compound was synthesized in a similar fashion to Example 1 using [2,2,2-trifluoro-1-(4-fluorophenyl)ethyl]amine (synthesized following the procedure of Tetrahedron Asymmetry 2002, 13, 2335-44). ¹H NMR: δ 0.73 (m, 2 H), 0.94 (m, 2 H), 1.92 (m, 1 H), 5.96 (m, 1H), 6.16 (m, 2 H), 7.24 (m, 2 H), 7.63 (m, 2 H), 8.01 (s, 1 H), 8.16 (br s, 1 H), 8.80 (br s, 1H).

### Example 188

### 4-(5-Cyclopropyl-1H-pyrazol-3-ylamino)-2-[(S)-1-(4-fluoro-phenyl)-ethylaminol-7,8-dihydro-5H-pyrido[4,3-d]pyrimidine-6-carboxylic acid benzyl ester

A solution of 4-(5-cyclopropyl-1*H*-pyrazol-3-ylamino)-2-methanesulfonyl-7,8-dihydro-5*H*-pyrido[4,3-*d*]pyrimidine-6-carboxylic acid benzyl ester (Method 144; 0.10 g, 0.21 mmol), (*S*)-1-(4-fluoro-phenyl)ethylamine (0.30 g, 2.1 mmol), and DIPEA (0.27 g, 2.1 mmol) in *n*-BuOH (3 ml) was heated to 110 °C in a sealed tube for 48 hours. The reaction was cooled to 25 °C, concentrated under reduced pressure, acidified with 0.5 N HCl (50 ml), and extracted with DCM (3 x 50 ml). The combined organic layer was dried over MgSO₄, concentrated, and purified by column chromatography (DCM: MeOH = 80: 1) to give the title compound (0.6 g, 54%). MS: Calcd.: 527; Found: [M+H]⁺ 528.

### Example 189

### 4-(5-Cyclopropyl-1H-pyrazol-3-ylamino)-2-[(S)-1-(4-fluoro-phenyl)-ethylamino]-5,8-dihydro-6H-pyrido[3,4-d]pyrimidine-7-carboxylic acid benzyl ester

A mixture of 4-(5-cyclopropyl-1*H-*pyrazol-3-ylamino)-2-methanesulfonyl-5,8-dihydro-6*H-*pyrido[3,4-*d*]pyrimidine-7-carboxylic acid benzyl ester (Method 149; 2.0 g, 4.3 mmol), (*S*)-1-(4-fluoro-phenyl)-ethylamine (0.30 g, 2.1 mmol), and DIEA (5.5 g, 42.6 mmol) *n*-BuOH (15 ml) was heated to 110 °C in a sealed tube for 48 hours, cooled to 25 °C, concentrated, acidified with 0.5 N HCl (100 ml), and extracted with DCM (3 x 150 ml). The combined organic layer was dried over MgSO₄, concentrated, and purified by column chromatography (DCM: MeOH = 80: 1) to give the title compound(0.7 g, 31%). MS: Calcd.: 527; Found: [M+H]⁺ 528.

### Example 190

### 6-Chloro-N²-[(1S)-1-(4-fluorophenyl)ethyl]-N⁴-(5-methyl-1H-pyrazol-3-yl)pyrimidine-2,4-diamine R

This title compound was prepared in a similar way to the preparation of Example 1 using 2,6-dichloro-*N*-(5-methyl-1*H*-pyrazol-3-yl)pyrimidin-4-amine (Method 150) and [(1*S*)-1-(4-fluorophenyl)ethyl]amine. ¹H NMR: δ 1.45 (s, 3 H), 2.20 (m, 3 H), 5.10 (m, 1 H), 5.85 - 6.10 (m, 2 H), 7.10 (m, 2 H), 7.40 (m, 2 H), 7.80 (m, 1 H), 8.60 (m, 1 H).

### Example 191

### 5,6-Dichloro-N⁴-(5-ethoxy-1H-pyrazol-3-yl)-N²-[(1S)-1-(4-fluorophenyl)ethyl]pyrimidine-2,4-diamine

A mixture of 2,5,6-trichloro-*N*-(3-ethoxy-1*H*-pyrazol-5-yl)pyrimidin-4-amine (Method 40; 300 mg, 0.98 mmol), [(1*S*)-1-(4-fluorophenyl)ethyl]amine (163 mg, 1.2 mmol) and triethylamine (0.16 ml) in n-butanol (2 ml) was heated at 106°C for 3 days. The mixture was concentrated. Reverse phase HPLC (Gilson) purification gave the title compound (198 mg). ¹HNMR (CDCl₃): δ 1.40 (m, 3 H), 1.52 (m, 3 H), 4.18 (m, 2 H), 4.93 - 5.50 (m, 2 H), 7.11 (m, 2 H), 7.38 (m, 2 H).

### Example 192-193

Following a similar procedure to Example 1, the following compounds were synthesized via reaction of a suitable pyrimidine (method of production of which is also listed) and a suitable amine.

| **Ex.** | **Compound** | **NMR** | **SM** |
|---|---|---|---|
| **192** | 5,6-Dichloro-*N*²-[(1*S*)-1-(4-fluorophenyl)ethyl]-*N*⁴-(3-isopropoxy-1*H*-pyrazol-5-yl)pyrimidine-2,4-diamine | 1.26 (m, 6 H), 1.41 (m, 1 H), 4.66 (m, 1 H), 4.95 (m, 1 H), 5.55 (m, 1 H), 7.14 (m, 2 H), 7.40 (m, 2 H), 8.18 (m, 1H), 9.86 (br s, 1 H), 11.89 (br s, 1 H) | Method 38 |
| **193** | (2*R*)-2-({4,5-Dichloro-6-[(3-isopropoxy-1*H*-pyrazol-5-yl)amino]pyrimidin-2-yl}amino)-2-(4-fluorophenyl)ethanol | 1.26 (m, 6H), 3.61 (m, 2 H), 4.66 (m, 1 H), 4.95 (m, 1 H), 5.00 (m, 1 H), 5.54 (m, 1 H), 7.14 (m, 2 H), 7.40 (m, 2 H), 8.18 (m, 1 H), 9.85 (br s, 1H), 11.99 (br s, 1 H) | Method 38 |

### Preparation of starting materials:

The starting materials for the Examples contained herein are either commercially available or are readily prepared by standard methods from known materials. For example the following reactions are illustrations but not limitations of the preparation of some of the starting materials and examples used herein.

### Method 1

### 2,5-Dichloro-4-(5-cyclopropyl-1H-pyrazole-3-ylamino)pyrimidine

A solution of 2,4,5-trichloropyrimidine (533 mg, 2.93 mmol), 3-amino-5-cyclopropyl-1*H*-pyrazole (360 mg, 2.93 mmol) and triethylamine (0.49 ml) in EtOH (5 ml) was stirred at room temperature for 10 hours. Solvent was removed and EtOAc was added. The solution was washed with water and dried over anhydrous sodium sulfate and was concentrated to give title compound as a white solid (546 mg, 69%). The compound was carried to the next step without further purification. ¹H NMR δ 0.92 (m, 2 H), 1.20 (m, 2H), 2.18 (m, 1 H), 6.40 (s, 1 H), 8.60 (s, 1 H), 9.90 (s, 1H),12.60 (s, 1 H).

### Method 2-19

The following compounds were prepared by the procedure of Method 1 using the appropriate starting materials.

| **Method** | **Compound** | **Pyrimidine** | **Amine** |
|---|---|---|---|
| **2** | 5-Bromo-2-chloro-*N*-(3-ethyl-1*H*-pyrazol-5-yl)pyrimidin-4-amine | 5-bromo-2,4-dichloropyrimidine | 3-amino-5-ethyl-1*H*-pyrazole |
| **3** | *N*-(3-*tert*-Butyl-1*H-*pyrazol-5-yl)-2,5-dichloropyrimidin-4-amine | 2,4,5-trichloropyrimidine | 3-amino-5-tert-butyl-1*H*-pyrazole |
| **4^{a}** | 2-Chloro-*N*-(3-cyclopropyl-1*H*-pyrazol-5-yl)-5-(trifluoromethyl)pyrimidin-4-amine | 5-trifluro-2,4-dichloropyrimidine | 3-amino-5-cyclopropyl-1*H*-pyrazole |
| **5** | 5-Bromo-2-chloro-N-(3-cyclopropyl-1*H*-pyrazol-5-yl)pyrimidin-4-amine | 5-bromo-2,4-dichloropyrimidine | 3-amino-5-cyclopropyl-1*H*-pyrazole |
| **6** | 5-Bromo-*N*-(5-*tert*-butyl-1*H*-pyrazol-3-yl)-2-chloropyrimidin-4-amine | 5-bromo-2,4-dichloropyrimidine | 3-amino-5-tert-butyl-1*H*-pyrazole |
| **7** | 5-Bromo-2-chloro-*N*-(5-cyclopropyl-1*H*-pyrazol-3-yl)-6-methylpyrimidin-4-amine | 5-bromo-6-methyl-2,4-dichloropyrimidine | 3-amino-5-cyclopropyl-1*H*-pyrazole |
| **8^{b}** | 2-Chloro-*N*-(3-cyclopropyl-1*H*-pyrazol-5-yl)pyrimidin-4-amine | 2,4-dichloropyrimidine | 3-amino-5-cyclopropyl-1*H*-pyrazole |
| **9^{b}** | 2-Chloro-*N*-(3-cyclopropyl-1*H*-pyrazol-5-yl)-5-methylpyrimidin-4-amine | 5-methyl-2,4-dichloropyrimidine | 3-amino-5-cyclopropyl-1*H*-pyrazole |
| **10** | Methyl 2-chloro-6-[(3-cyclopropyl-1*H*-pyrazol-5-yl)amino]pyrimidine-4-carboxylate | 6-methoxy carbonyl-2,4-dichloropyrimidine | 3-amino-5-cyclopropyl-1*H*-pyrazole |
| **11** | 5-Bromo-2-chloro-*N*-(5-methyl-1*H*-pyrazol-3-yl)pyrimidin-4-amine | 5-bromo-2,4-dichloropyrimidine | 3-amino-5-methyl-1*H*-pyrazole |
| **12** | 2,5-Dichloro-*N*-(5-methyl-1*H*-pyrazol-3-yl)pyrimidin-4-amine | 2,4,5-trichloro pyrimidine | 3-amino-5-methyl-1*H*-pyrazole |
| **13** | 2-Chloro-*N*-(3-cyclopropyl-1*H*-pyrazol-5-yl)-5-fluoropyrimidin-4-amine | 5-fluoro-2,4-dichloropyrimidine | 3-amino-5-cyclopropyl-1*H*-pyrazole |
| **14^{b}** | 2-Chloro-*N*-(3-cyclopropyl-1*H*-pyrazol-5-yl)-6-methylpyrimidin-4-amine | 6-methyl-2,4-dichloropyrimidine | 3-amino-5-cyclopropyl-1*H*-pyrazole |
| **15** | 2,6-Dichloro-*N*-(3-cyclopropyl-1*H*-pyrazol-5-yl)pyrimidin-4-amine | 2,4,6-trichloro pyrimidine | 3-amino-5-cyclopropyl-1*H*-pyrazole |
| **16** | 2,5,6-Trichloro-*N*-(3-cyclopropyl-1*H*-pyrazol-5-yl)pyrimidin-4-amine | 2,4,5,6-tetrachloro pyrimidine | 3-amino-5-cyclopropyl-1*H*-pyrazole |
| **17^{c}** | Ethyl 2-chloro-6-[(3-cyclopropyl-1*H*-pyrazol-5-yl)amino]pyrimidine-4-carboxylate | 6-methyoxy carbonyl-2,4-dichloropyrimidine | 3-amino-5-cyclopropyl-1*H*-pyrazole |
| **18** | 2-chloro-*N*-(5-cyclopropyl-1*H*-pyrazol-3-yl)-5-nitropyrimidin-4-amine | 2,4-dichloro-5-nitropyrimidine | 3-amino-5-cyclopropyl-1*H*-pyrazole |
| **19** | 2,5-dichloro-*N*-[3-(trifluoromethyl)-1*H-*pyrazol-5-yl]pyrimidin-4-amine | 2,4,5-trichloropyrimidine | 3-(trifluoromethyl)-1*H*-pyrazol-5-amine (Method 22) |

| | | | |
|---|---|---|---|
| ^{a} The reaction is similar to Method 1 except that the reaction was performed at -20 °C ^{b} The reaction is similar to Method 1 except that the reaction was performed at 70°C ^{c} The ethoxy group resulted from transesterification with solvent | | | |

### Method 20

### N-Methyl-1-pyridin-2-ylethanamine

To 1-pyridin-2-ylethyl methanesulfonate (Tetrahedron Asymmetry 1994, 5, 1973-78; 800 mg, 4 mmol) was added methylamine (2.0 M in THF, 10 ml, 20 mmol) and the reaction mixture was stirred at 50°C overnight. The solvent was removed to give the desired product as an oil (544 mg, quantitative yield). ¹H NMR (CDCl₃): δ 1.40 (m, 3 H), 2.35 (s, 3 H), 3.75 (m, 1 H), 7.15 (m, 1 H), 7.25 (m, 1 H), 7.63 (m, 1 H), 8.56 (m, 1 H).

### Method 21

### 1-Amino-1-phenylpropan-2-ol

The diasteroisomeric mixture was prepared according in a similar fashion to a known procedure (J. Org. Chem. 1991, 56, 6939-6942).

### Method 22

### 3-(Trifluoromethyl)-1H-pyrazol-5-amine

A solution of 4,4,4-trifluoro-3-oxo-butyronitrile (Method 23; 11.0 g, 0.080 mol) and hydrazine monohydrate (3.89 ml, 0.080 mol) in EtOH (400 ml) was heated at reflux for 5 hours. After cooling to 25 °C, the solvent was removed under reduced pressure. The resulted residue was dissolved in DCM (500 ml), washed with brine (2 x 200 ml), and dried over Na₂SO₄. After evaporation of the solvent, the resulted residue was purified by column chromatography (hexane: EtOAc =1: 1) to give the title compound as a pale yellow solid (1.93 g, 16%). ¹H NMR (400 MHz): δ 5.35 (s, 2 H), 5.56 (s, 1 H), 12.10 (br s, 1 H).

### Method 23

### 4,4,4-Trifluoro-3-oxo-butyronitrile

60% NaH (9.6 g, 0.24 mol) was suspended in dioxane (400 ml), to which was added acetonitrile (12.62 ml, 0.24 mol) dropwise. The reaction mixture was stirred at 25 °C for 30 minutes, followed by addition of ethyl trifloroacetate (23.8 ml, 0.2 mol). The reaction mixture was heated to reflux for 3 hours, cooled to 25 °C, and quenched with water (400 ml). The unreacted starting material was extracted with DCM (100 ml). The aqueous layer was acidified with 10% HCl to pH 3 and extracted with DCM (100 ml). The organic layer was dried over Na₂SO₄, concentrated under reduced pressure, and purified by chromatography (hexane: EtOAc = 5: 1) to give the title compound as a white solid (11.0 g, 40%). ¹H NMR (400 MHz, CDCl₃) δ 4.72 (s, 1 H).

### Method 24

### (1-Pyridin-2-ylethyl)amine

To a mixture of 1-pyridin-2-yl-ethanone (0.20 g, 1.65 mmol) and ammonium choride (0.88 g, 16.5 mmol) in MeOH (5 ml) was added sodium cyanoborohydride (0.125 g, 1.98 mmol). The reaction was stirred at 25 °C for 64 hours and quenched by addition macroporous polystyrene sulfonic acid (MP-TsOH) (4.08 g, 16.5 mmol) resin. The suspension was agitated for 1 hour. The resin was collected by filtration and washed with MeOH (3 x 20 ml, 20 minute shaking per wash). The resulted resin was then treated with NH₃/MeOH solution (7 M, 15 ml) for 20 minutes, filtered, and washed with MeOH (2 x 15 ml). The combined filtrate was concentrated until the crude weighed 200 mg. The crude product contained about 30% desired product, 35% alcohol, and 35% dimmer as indicated by LC/MS, and was directly used without further purification.

### Method 25

### (5-benzyl-2H-pyrazol-3-yl)-(2,5-dichloro-pyrimidin-4-yl)-amine

The solution of 2,4,5-trichloropyrimidine (0.150 g, 0.82 mmol), 5-benzyl-2*H*-pyrazol-3-ylamine (Method 27; 0.129 g, 0.74 mmol), and triethylamine (0.155 ml, 1.12 mmol) in EtOH (5 ml) was heated to 55 °C for 5 hours and then stirred at room temperature overnight. The reaction solution was concentrated under reduced pressure. The resulted yellow solid was stirred in hexanes: ether solution (1: 1), collected by filtration, and then recrystallized from DCM to give the title compound (0.212 g, 89%). ¹H NMR (400 MHz, CDCl₃) δ 4.10 (d, *J*= 15.2 Hz, 2 H), 6.89 (s, 1 H), 7.27 -7.37 (m, 5 H), 8.28 (s, 1 H). MS: Calcd.: 319; Found: [M+H]⁺ 320.

### Method 26

### 3-Oxo-4-phenyl butyronitrile

NaH (60% in mineral oil, 0.48 g, 12.0 mmol) of) was suspended in dioxane (20 ml), to which was added acetonitrile (0.63 ml, 12 mmol) dropwise. The reaction was stirred for 20 minutes, followed by addition of ethyl phenylacetate (10 mmol) solution in dioxane (8 ml). The mixture was refluxed for 4 hours, cooled to 25 °C, and quenched with H₂O (40 ml). The unreacted starting material was extracted with DCM (40 ml). The aqueous layer was acidified with 1 N HCl to pH 5 and extracted with DCM (40 ml). The organic layer was dried over anhydrous Na₂SO₄, concentrated, and purification by column chromatography (hexane: EtOAc = 3: 2) to afford the title compound (0.58 g, 36%). ¹H NMR (400 MHz, CDCl₃) δ 3.45 (s, 2 H), 3.85 (s, 2 H), 7.2 - 7.28 (m, 5 H). MS: Calcd.: 159; Found: [M-H]⁻ 158.

### Method 27

### 5-Benzyl-2H-pyrazol-3-ylamine

A solution of 3-oxo-4-phenyl butyronitrile (Method 26; 0.58 g, 3.64 mmol) and hydrazine monohydrate (0.177 ml, 3.64 mmol) in EtOH (16 ml) was heated to reflux for 3 hours. After cooling to 25 °C, the reaction was concentrated under reduced pressure, extracted with DCM (15 ml), and washed twice with brine. The organic layer was dried over anhydrous Na₂SO₄ and concentrated to give an orange solid. The resulted solid was triturated with hexanes: ether (1:1) solution and collected by filtration to afford the title compound as a yellow solid (0.38 g, 60%). ¹H NMR (400 MHz, CDCl₃) δ 3.9 (s, 2 H), 4.89 (br s, 1 H), 5.44 (s, 1 H), 7.19 - 7.34 (m, 5H). MS: Calcd.: 173; Found: [M+H]⁺ 174.

### Method 28

### (2,5-Dichloro-pyrimidin-4-yl)-(5-isopropyl-2H-pyrazol-3-yl)-amine

A solution of 5-isopropyl-2H-pyrazol-3-ylamine (Method 29; 0.093 g, 0.74 mmol), 2,4,5-trichloropyrimidine (0.150 g, 0.82 mmol), and triethylamine (0.36 ml, 2.6 mmol) in EtOH (5 ml) was heated to 55°C for 5 hours and stirred at 25 °C overnight. The reaction mixture was concentrated under reduced pressure, triturated with DCM to afford the title compound (0.192, 95%). ¹H NMR (400 MHz, CDCl₃) δ 1.34 - 1.37 (d, *J*= 7.0 Hz, 6 H), 3.07 - 3.14 (q, 1 H), 6.72 (s, 1 H), 8.20 (s, 1 H). MS: Calcd.: 271; Found: [M+H]⁺ 272.

### Method 29

### 5-Isopropyl-2H-pyrazol-3-ylamine

A solution of 4-methyl-3-oxo-pentanenitrile (Method 30; 0.42 g, 3.78 mmol) and hydrazine monohydrate (0.183 ml, 3.78 mmol) in EtOH (20 ml) was heated to reflux for 1 hour, cooled, and then concentrated under reduced pressure. The resulted oil was dissolved with DCM, washed with brine, dried over anhydrous Na₂SO₄, concentrated, and purification by column chromatography (EtOAc: MeOH = 20: 1) to the title compound as an orange solid (0.26 g, 56%). ¹H NMR (400 MHz, CDCl₃) δ 1.21 - 1.23 (d, *J*= 7.0 Hz, 6 H), 2.82 - 2.89 (q, 1 H), 5.42 (s, 1 H). MS: Calcd.: 125; Found: [M+H]⁺ 126.

### Method 30

### 4-Methyl-3-oxo-pentanenitrile

NaH (60% in mineral oil, 0.72 g, 18 mmol) was suspended in dioxane (20 ml), to which was carefully added ethyl isobutyrate (2.0 ml, 15 mmol) followed by addition of acetonitrile (0.95 ml, 18 mmol). The reaction mixture was refluxed for 5 hours, cooled, and quenched with water (40 ml). The unreacted starting material was extracted with DCM. The aqueous layer was acidified with 1 N HCl to pH 5 and extracted with DCM. The organic layer was dried over Na₂SO₄, concentrated, and purification by column chromatography (hexane: EtOAc = 3: 2) to give the title compound (0.43 g, 26%). ¹H NMR (400 MHz, CDCl₃) δ 1.13 - 1.15 (d, *J*= 7.0, 6 H), 2.73 - 2.79 (q, 1H), 3.52 (s, 2 H). MS: Calcd.: 111; Found: [M-H]⁻ 110.

### Method 31

### 5-Cyclopropylmethyl-2H-pyrazol-3-yl)-(2,5-dichloro-pyrimidin-4-yl)-amine

A solution of 2,4,5-trichloropyrimidine (0.2 g, 1.1 mmol), 5-cyclopropylmethyl-2*H-*pyrazol-3-ylamine (Method 32; 0.14 g, 0.99 mmol), and triethylamine (0.15 g, 1.5 mmol) in EtOH (5 ml) was heated to 55 °C overnight. The solvent was removed under reduced pressure and the resulted solid was triturated with DCM to afford the title compound as a white solid (0.206 g, 73%). ¹H NMR (400 MHz, CDCl₃) δ 0.008 (m, 2 H), 0.363 (m, 2 H), 0.801 (m, 1 H), 2.36 - 2.38 (d, *J*= 7.0 Hz, 2 H), 6.49 (s, 1H), 7.93 (s, 1 H). MS: Calcd.: 283; Found: [M+H]⁺ 284.

### Method 32

### 5-Cyclopropylmethyl-2H-pyrazol-3-ylamine

A solution of 4-cyclopyropyl-3-oxo-butyronitrile (Method 33; 1.0 g, 8.1 mmol) and hydrazine monohydrate (0.4 g, 8.1 mmol) in EtOH (35 ml) was heated to reflux for 2 hours, cooled, and concentrated under reduced pressure. The resulted residue was taken up in DCM, washed with brine, dried over anhydrous sodium sulfate, concentrated, and purified by column chromatography (EtOAc: MeOH: Et₃N = 94 : 5 : 1) to afford the title compound (0.464 g, 42%). ¹H NMR (400 MHz, CDCl₃) δ 0.005 (m, 2 H), 0.370 (m, 2 H), 0.78 (m, 1 H), 2.27 - 2.29 (d, *J*= 7.0 Hz, 2 H), 5.07 (br s, 1H), 5.32 (s, 1H). MS: Calcd.: 137; Found: [M+H]⁺ 138.

### Method 33

### 4-Cyclopropyl-3-oxo-butyronitrile

Cyanoacetic acid (8.5 g, 100 mmol) solution in EtOAc (200 ml) was treated with anhydrous MgSO₄ and stirred for 20 minutes. MgSO₄ was removed by filtration and the filtrate was concentrated under reduced pressure. The resulted white solid was dissolved in THF (166 ml) and 5 mg of 2,2'-bipyridine was added as an indicator. The reaction solution was cooled to -78°C, to which was added *n*-butyllithium solution (80 ml, 199 mmol). The reaction mixture was allowed to warm to 0 °C gradually then cooled to -78°C again, to which was added cyclopropyl-acetyl chloride (Method 34; 5.9 g, 50 mmol) in DCM (80 ml) via an addition funnel. The reaction mixture was then stirred at 25°C for 1 hour, quenched with 2 N HCl and extracted with chloroform. The organic layer was washed with a saturated sodium bicarbonate solution, brine, dried with MgSO₄, concentrated, and purified by column chromatography (EtOAc: MeOH = 20: 1) to give the title compound (1.0 g, 16% yield). ¹H NMR (400 MHz, CDCl₃) δ 0.019 (m, 2 H), 0.472 (m, 2 H), 0.832 (m, 1H), 2.29 - 2.32 (d, *J*= 7.0 Hz, 2 H), 3.38 (s, 2 H).

### Method 34

### Cyclopropyl-acetyl chloride

To the DCM (30 ml) solution of cyclopropyl acetic acid (5.0 g, 49.9 mmol) with 6 drops DMF, was slowly added the solution of oxalyl chloride (5.2 ml, 59.9 mmol) in DCM (5 ml). After completion of the addition, the reaction solution was stirred at 25 °C for 4 hours. Evaporation of the solvent under reduced pressure gave the title compound as a bright yellow solid (5.9 g, 99.6%). ¹H NMR (400 MHz, CDCl₃) δ 0.018 (m, 2 H), 0.421 (m, 2 H), 0.896 (m, 1 H), 2.53 - 2.55 (d, *J*= 5.85 Hz, 2 H).

### Method 35

### (5-Cyclopropylmethoxy-1H-pyrazol-3-yl)-(2,5-dichloro-pyrimidin-4-yl)-amine

A solution of 2,4,5-trichloropyrimidine (0.30 g, 1.6 mmol), 5-cyclopropylmethoxy-1*H*-pyrazol-3-ylamine (Method 44; 0.23 g, 1.5 mmol), and triethylamine (0.23 ml, 2.2 mmol) in EtOH (8 ml) was heated to 55 °C overnight. The reaction was concentrated under reduced pressure and purification by column chromatography (hexane: EtOAc =1 : 1) to give the title compound (0.20 g, 6%). ¹H NMR (400 MHz): d 0.321 (m, 2 H), 0.552 (m, 2 H), 0.863 (m, 1 H), 3.90 (d, *J*= 7.0 Hz, 2 H), 5.8 (s, 1H), 8.41 (s, 1H). MS: Calcd.: 299; Found: [M+H]⁺ 5 300.

### Methods 36-43

The following compounds were prepared by the procedure of Method 35 using the appropriate starting materials.

| **Method** | **Compound** | **pyrimidine** | **Pyrazole** |
|---|---|---|---|
| **36** | *N*-(5-*sec*-Butoxy-1*H*-pyrazol-3-yl)-2,5-dichloropyrimidin-4-amine | 2,4,5-trichloropyrimidine | 5-*sec*-butoxy-1*H*-pyrazol-3-amine (Method 45) |
| **37** | *N*-(5-Propoxy-1*H*-pyrazol-3-yl)-2,5-dichloropyrimidin-4-amine | 2,4,5-trichloropyrimidine | 5-propoxy-1*H* pyrazol-3-amine (Method 46) |
| **38** | *N*-(5-Isopropoxy-1*H*-pyrazol-3-yl)-2,5-dichloropyrimidin-4-amine | 2,4,5-trichloropyrimidine | 5-isopropoxy-1*H*-pyrazol-3-amine (Method 47) |
| **39** | *N*-(5-Ethoxy-1*H*-pyrazol-3-yl)-2,5-dichloropyrimidin-4-amine | 2,4,5-trichloropyrimidine | 5-ethoxy-1*H*-pyrazol-3-amine (Method 48) |
| **40** | 2,5,6-Trichloro-*N*-(5-ethoxy-1*H*-pyrazol-3-yl)pyrimidin-4-amine | 2,4,5,6-tetrachloropyrimidine | 5-ethoxy-1*H*-pyrazol-3-amine (Method 48) |
| **41** | 2,5,6-Trichloro-*N*-(5-isopropoxy-1*H*-pyrazol-3-yl)pyrimidin-4-amine | 2,4,5,6-tetrachloropyrimidine | 5-isopropoxy-1*H*-pyrazol-3-amine (Method 47) |
| **42** | 2-Chloro-*N*-(5-ethoxy-1*H*-pyrazol-3-yl)-5-fluoropyrimidin-4-amine | 2,4-dichloro-5-fluoropyrimidine | 5-ethoxy-1*H*-pyrazol-3-amine (Method 48) |
| **43** | 2-Chloro-*N*-(5-isopropoxy-1*H*-pyrazol-3-yl)-5-fluoropyrimidin-4-amine | 2,4-dichloro-5-fluoropyrimidine | 5-isopropoxy-1*H*-pyrazol-3-amine (Method 47) |

### Method 44

### 5-Cyclopropylmethoxy-1H-pyrazol-3-ylamine

Triphenylphosphine (16.0 g, 61 mmol), 5-amino-2*H*-pyrazol-3-ol (5.5 g, 56 mmol), and cyclopropyl methanol (4.4 g, 61 mmol) were dissolved in THF (100 ml), to which was slowly added the diisopropyl azodicarboxylate (12 ml, 61 mmol) solution in THF (50 ml). The reaction mixture was stirred for 1 hour, diluted with DMF (45 ml), and allowed at 25 °C overnight. The solvent was removed under reduced pressure. The resulted residue was treated with water, extracted with EtOAc twice and DCM once. The combined organics were dried over anhydrous sodium sulfate, concentrated under reduced pressure, and purified by column chromatography (EtOAc) to give the title compound (1.3 g, 15%). MS: Calcd.: 153; Found: [M+H]⁺ 154.

### Method 45-48

The following compounds were prepared by the procedure of Method 44 using the appropriate starting materials.

| **Method** | **Compound** | **Alcohol** | **Pyrazole** |
|---|---|---|---|
| **45** | 5-*sec*-Butoxy-1*H*-pyrazol-3-amine | butan-2-ol | 5-amino-2*H*-pyrazol-3-ol |
| **46** | 5-Propoxy-1*H*-pyrazol-3-amine | propanol | 5-amino-2*H*-pyrazol-3-ol |
| **47** | 5-Isopropoxy-1*H*-pyrazol-3-amine | isopropanol | 5-amino-2*H*-pyrazol-3-ol |
| **48** | 5-Ethoxy-1*H*-pyrazol-3-amine | ethanol | 5-amino-2*H*-pyrazol-3-ol |

### Method 49

### {(1S)-1-[4-(Trifluoromethyl)-1,3-thiazol-2-yl]ethyl}amine

To a solution of (*S*)-*N*-benzyloxycarbonyl-1-(4-trifluoromethyl-thiazol-2-yl)-ethylamine (Method 50; 0.45 g, 1.36 mmol) in anhydrous CH₃CN (5 ml) was added TMS-I (0.23 ml, 1.64 mmol) at 25 °C. The reaction was stirred at room temperature for 30 minutes, quenched with cold HCl/ethyl ether solution (2 N, 10 ml), and diluted with ethyl ether (40 ml). The precipitate was quickly collected by filtration and washed with ether. The resulted solid was treated with 10 ml of saturated NaHCO₃, extracted with ethyl ether (3 x 20 ml). The combined organic was washed with brine (20 ml) and dried over Na₂SO₄. The solvent was evaporated under reduced pressure at below 20°C (Note: this product is volatile, high-vacuum should be avoided). The crude product as a light brown oil (0.24 g, 90%) was used without further purification.

### Method 50

### (S)-N-Benzyloxycarbonyl-1-(4-trifluoromethyl-thiazol-2-yl)-ethylamine

*N*^{α}-Benzyloxycarbonyl-L-alanine thiamide (Method 51; 1.0 g, 4.2 mmol) and 3-bromo-1,1,1-trifluoro-propan-2-one (0.52 ml, 5.0 mmol) were dissolved in acetone (10 ml) and heated to reflux for 6 hours. The reaction was cooled to 25 °C and the solvent was removed under reduced pressure. The reaction resultant was treated with saturated NaHCO₃ solution (15 ml), extracted with EtOAc (15 ml), washed with H₂O (2 x 15 ml), brine (15 ml), and dried over Na₂SO₄. After evaporation of the solvent, the crude material was purified by silica-gel chromatography (hexane: EtOAc = 2: 1) to give the title compound as a yellowish oil (1.1 g, 79%). ¹H NMR (400 MHz, CDCl₃) δ 1.65 (d, *J*= 6.4 Hz, 3 H), 5.15 (m, 3 H), 5.31 1 (br s, 1H), 7.30 (m, 5H), 7.68 (s, 1H), MS: Calcd.: 330; Found: [M+H]⁺ 331.

### Method 51

### N^{α}-Benzyloxycarbonyl-L-alanine thiamide

*N*^{α}-Benzyloxycarbonyl-L-alanine (10.0 g, 44.8 mmol) in THF (150 ml) was treated with 1,1'-carbonyldiimidazole (CDI) (21.79 g, 134.4 mmol) at 25 °C for 4 hours. With ice-H₂O cooling, NH₃ was bubbled through for 1 hour. The reaction mixture was allowed to stir at 25 °C overnight. THF was removed at reduced pressure. The resulted residue was extracted with EtOAc (200 ml), washed with H₂O (2 x 100 ml), brine (200 ml), and dried over Na₂SO₄. After evaporation of the solvent, the crude material was passed through a silica-gel column eluted with EtOAc. The desired *N*^{α}-benzyloxycarbonyl-L-alanine amide was obtained as a white solid (8.10 g, 81% yield). This amide derivative (5.0 g, 22.5 mmol) was dissolved in THF (50 ml) and treated with Lawesson's reagent (5.46 g, 13.5 mmol) at 25 °C for 4 hours. After evaporation of THF, the resultant mixture was directly loaded for column purification eluted with EtOAc to give the title compound as a white solid (4.70 g, 87%). ¹H NMR (400 MHz, CDCl₃) δ 1.39 (d, *J*= 6.8 Hz, 3H), 4.28 (m, 1H), 5.11 (s, 2 H), 5.50 (d, *J*= 6.8 Hz, 1 H), 5.76 (br s, 1 H), 6.19 (br s, 1 H), 7.33 (m, 5 H). MS: Calcd.: 238; Found: [M+H]⁺239.

### Method 52

### [(1S)-1-(1,3-Thiazol-2-yl)ethyl]amine

To a solution of (*S*)-*N*-benzyloxycarbonyl-1-thiazol-2-yl-ethylamine (Method 53; 0.50 g, 1.51 mmol) in anhydrous CH₃CN (5 ml) was added TMS-I (0.26 ml, 1.82 mmol) at 25 °C. The reaction was stirred at room temperature for 30 minutes, then quenched by addition of cold HCl/ethyl ether solution (2 N, 10 ml) and diluted with 40 ml of ethyl ether. The precipitate was quickly collected by filtration and washed with ether. The resultant solid was suspended in DCM (1 ml) and treated with 25% of NaOMe/MeOH solution (0.363 ml, 1.59 mmol). The reaction mixture was stirred at 25 °C for 30 minutes, then to which was added 10 ml DCM. The reaction mixture was stirred at 25 °C for 1 hour. The white solid was removed by filtration and the filtrated was concentrated under reduced pressure at below 20 °C to give the crude product as light-brown oil (0.25 g, 84%). (Note: this product is volatile, high-vacuum should be avoided). The crude product was used without further purification.

### Method 53

### (S)-N-Benzyloxycarbonyl-1-thiazol-2-yl-ethylamine

*N*^{α}-Benzyloxycarbonyl-L-alanine thiamide (Method 51; 2.1 g, 8.8 mmol) and bromoacetaldehyde dimethyl acetal (2.08 ml, 17.6 mmol) were dissolved in acetone (20 ml), to which was added HCl/dioxane solution (4 N, 0.1 ml, 0.44 mmol). The resultant solution was heated to reflux for 6 hours. The reaction was cooled to 25 °C and the solvent was removed under reduced pressure. The reaction resultant was treated saturated NaHCO₃ solution (30 ml) and extracted with EtOAc (30 ml). The organic layer was washed with H₂O (2 x 30 ml), brine (30 ml), and dried over Na₂SO₄. After evaporation of the solvent, the crude material was purified by silica-gel chromatography (hexane: EtOAc = 2: 1) to give the title compound as a light brown oil (1.7 g, 74%). ¹H NMR (400 MHz, CDCl₃) δ 1.63 (d, *J*= 6.8 Hz, 3 H), 5.01 - 5.22 (m, 3 H), 5.54 (br s, 1 H), 7.26 (d, *J*= 2.8 Hz, 1 H), 7.33 (m, 5 H), 7.71 (d, *J*=2.8 Hz, 1 H): MS: Calcd.: 262; Found: [M+H]⁺263.

### Method 54

### 2,4-Dichloroquinazoline

A solution of quinazoline-2,4(1*H*, 3*H*)-dione (1.0 g, 6.17 mmol) and POCl₃ (20 ml) in DMF (96 ml) was heated at 110°C for 17 hours. The resulting solution was cooled down and poured onto ice with stirring. Once the ice melted, the solid material was filtered and dissolved in DCM (100 ml). The solution was washed with water once and concentrated to give product as a white solid (970 mg, 79%). ¹H NMR δ 7.90 (m, 1 H), 8.03 (m, 1 H), 8.815 (m, 1 H), 8.28 (m, 1 H).

### Method 55

### 2-Chloro-N-(5-cyclopropyl-1H-pyrazol-3-yl)quinazolin-4-amine

A solution of 2,4-dichloroquinazoline (Method 54; 404 mg, 2.0 mmol), 3-amino-5-cyclopropyl-1H-pyrazole (250 mg, 2.0 mmol) and triethylamine (0.34 ml, 0.24 mmol) in THF (6.0 ml) was stirred at room temperature for 10 hrs. The solid formed was filtered and washed with EtOH (5 ml) and MeOH (5 ml). The solid was dried to give desired product as a white solid (100 mg, 69%. The compound was carried to the next step without further purification. ¹H NMR δ 0.9 (m, 2 H), 1.1 (m, 2 H), 1.8 -2.1 (m, 1 H), 6.6 (m, 1 H), 7.75 (m, 1 H), 7.85 (m, 1 H), 8.0 (m, 1 H), 9.9 (br s, 1 H), 11.0 (br s, 1 H).

### Method 56-57

The following compounds were prepared by the procedure of Methods 54-55 using the appropriate starting materials.

| **Method** | **Compound** | **Quinazoline** | **Amine** |
|---|---|---|---|
| **56** | *N*-(5-*tert*-Butyl-1*H*- pyrazol-3-yl)-2-chloroquinazolin-4-amine | 2,4-dichloroquinazoline Method 54 | 3-amino-5-tert-butyl-1*H*-pyrazole |
| **57** | 2-Chloro-*N*-(5-methyl-1*H*-pyrazol-3-yl)quinazolin-4-amine | 2,4-dichloroquinazoline Method 54 | 3-amino-5-methyl-1*H-* pyrazole |

### Method 58

### Methyl 3-[(aminocarbonyl)aminolthiophene-2-carboxylate

To a solution of methyl 3-amino-2-thiophenecarboxylate (3.43 g, 21.8 mmol) in acetic acid (20 ml) was added a solution of potassium cyanate (3.2 g, 39.4 mmol) in water (15 ml). After stirring for 20 hrs at room temperature, the mixture was filtered and washed by water. The off white solid is the desired product (4.78 g). ¹H NMR (400 MHz): δ 3.9 (s, 3 H), 6.8 (br s, 2 H), 7.76 (d, J = 4 Hz, 1 H), 7.93 (d, J = 4 Hz, 1 H).

### Methods 59-61

The following compounds were prepared by the procedure of Method 58 using the appropriate starting materials.

| **Method** | **Compound** | **Carboxylate** |
|---|---|---|
| **59** | Methyl 2-[(aminocarbonyl)amino] thiophene-3-carboxylate | methyl 2-aminothiophene-3-carboxylate |
| **60** | Methyl 5-[(aminocarbonyl) amino]-1*H*-pyrazole-4-carboxylate | methyl 5-amino-1H-pyrazole-4-carboxylate |
| **61** | Methyl 4-[(aminocarbonyl)amino] thiophene-3-carboxylate | methyl 4-aminothiophene-3-carboxylate |

### Method 62

### Thieno[3.2-d]pyrimidine-2.4(1H,3H)-dione

To a solution of methyl 3-[(aminocarbonyl)amino]thiophene-2-carboxylate (Method 58, 4.78 g) in MeOH (50 ml) was added the solution of sodium hydroxide (1.2 g, 30 mmol) in water (15 ml). The reaction mixture was heated to reflux for 1 hr. Water was added to dissolve the solid. 50% sulfuric acid was added to adjust the pH<1. The resulting off white solid was filtered to give desired product (2.41 g, 66% for 2 steps). ¹H NMR (400 MHz): δ 6.91 (d, J = 5.2 Hz, 1 H), 8.05 (d, J = 5.2 Hz, 1 H), 11.2 (br s, 1 H), 11.5 (br s, 1 H).

### Methods 63-65

The following compounds were prepared by the procedure of Method 62 using the appropriate starting materials.

| **Method** | **Compound** | **Carboxylate** | **SM** |
|---|---|---|---|
| **63** | Thieno[2,3-d]pyrimidine-2,4(1*H*,3*H*)-dione | methyl2-[(aminocarbonyl)amino] thiophene-3-carboxylate | Method 59 |
| **64** | 1*H*-Pyrazolo[3,4-d] pyrimidine-4,6(5*H*,7*H*)-dione | methyl 5-[(aminocarbonyl)amino]-1*H-*pyrazole-4-carboxylate | Method 60 |
| **65** | Thieno[3,4-*d*]pyrimidine-2,4(1*H*,3*H*)-dione | methyl4-[(aminocarbonyl)amino] thiophene-3-carboxylate | Method 61 |

### Method 66

### 2,4-Dichlorothieno[3,2-d]pyrimidine

A mixture of thieno[3,2-d]pyrimidine-2,4(1*H*,3*H*)-dione (Method 62, 380 mg, 2.3 mmol), phosphorus oxychloride(10 ml) and diethylaniline (1 ml) was heated at 105°C for 16 hrs. Solvent was then removed and ice was added to the mixture. The solid was filtered and gave pink colour solid product (432.4 mg, 92%). ¹H NMR (400 MHz): δ 7.98 (d, J = 5.6 Hz, 1 H), 8.94 (d, J = 5.6 Hz, 1 H).

### Methods 67-69

The following compounds were prepared by the procedure of Method 66 using the appropriate starting materials.

| **Method** | **Compound** | **Starting Material** |
|---|---|---|
| **67** | 2,4-Dichlorothieno[2,3-d]pyrimidine | Method 63 |
| **68** | 4,6-Dichloro-1*H*-pyrazolo[3,4-d]pyrimidine | Method 64 |
| **69** | 2,4-Dichlorothieno[3,4-d]pyrimidine | Method 65 |

### Method 70

### 2-Chloro-N-(5-cyclopropyl-1Hpyrazol-3-yl)thieno[3,2-d]pyrimidin-4-amine

A mixture of 2,4-dichlorothieno[3,2-d]pyrimidine (Method 66, 100 mg, 0.49 mmol), 3-cyclopropyl-1*H*pyrazol-5-amine (150 mg, 1.22 mmol) and triethylamine (0.15 ml, 1.1 mmol) was stirred at room temperature for 24 hrs. Water was added and the solid was filtered to give off white solid product (98 mg, 69%). ¹H NMR (400 MHz): δ 0.5 (m, 2 H), 0.8 (m, 2 H), 1.8 (m, 1 H), 6.0 (br s, 1 H), 7.1 (d, J = 6 Hz, 1 H), 8.0 (d, J = 6 Hz, 1 H), 10.2 (br s, 1 H), 12.2 (br s, 1 H).

### Methods 71-73

The following compounds were prepared by the procedure of Method 70 using the appropriate starting materials.

| **Method** | **Compound** | **Fused pyrimidine** | **Amine** | **SM** |
|---|---|---|---|---|
| **71** | 2-Chloro-*N*-(5-cyclopropyl-1*H*-pyrazol-3-yl)thieno[2,3-*d*]pyrimidin-4-amine | 2,4-dichlorothieno [2,3-d]pyrimidine | 3-amino-5-cyclopropyl-1*H*-pyrazole | Method 67 |
| **72** | 6-Chloro-*N*-(5-cyclopropyl-1*H-* pyrazol-3-yl)-1*H*-pyrazolo[3,4-d]pyrimidin-4-amine | 4,6-dichloro-1H pyrazolo[3,4-d]pyrimidine | 3-amino-5-cyclopropyl-1H-pyrazole | Method 68 |
| **73** | 2-Chloro-*N*-(5-cyclopropyl-1*H*-pyrazol-3-yl)thieno[3,4-d]pyrimidin-4-amine | 2,4-dichlorothieno [3,4-d]pyrimidine | 3-amino-5-cyclopropyl-1*H*-pyrazole | Method 69 |

### Method 74

### Pyrido[2,3-d]pyrimidine-2,4(1H,3H)-dione

A finely powdered mixture of 2-aminonicotinic acid (7.0 g, 50.7 mmol) and urea (13.0 g, 216 mmol) was heated to 180-190°C, and maintained for 15 minutes at the same temperature. The temperature was gradually raised to 200°C and the clear melt started to solidify. The temperature was raised to 210°C and the heating was discontinued. The mixture was cooled to room temperature and 2 N NaOH solution (70 ml) was added. It was heated at 50 - 55°C to get a clear solution. This warm solution was saturated with carbon dioxide, cooled and filtered and the solid was washed with water (2 x 25 ml) to give desired product (7.71 g, 76%). ¹H NMR δ 7.16 (m, 1 H), 8.22 (d, J = 6.4 Hz, 1 H), 8.53 (m, 1 H).

### Methods 75-81

The following compounds were prepared by the procedure of Method 74 using the appropriate starting materials.

| **Method** | **Compound** | **Amine** |
|---|---|---|
| **75** | 7-Methylquinazoline-2,4(1H,3H)-dione | 2-amino-4-methylbenzoic acid |
| **76** | 6-Methylquinazoline-2,4(1*H*,3*H*)-dione | 2-amino-5-methylbenzoic acid |
| **77** | 6-Methoxyquinazoline-2,4(1H,3H)-dione | 2-amino-5-methoxybenzoic acid |
| **78** | 7-Chloroquinazoline-2,4(1*H*,3*H*)-dione | 2-amino-4-chlorobenzoic acid |
| **79** | 6-Chloroquinazoline-2,4(1*H*,3*H*)-dione | 2-amino-5-chlorobenzoic acid |
| **80** | 8-Methoxyquinazoline-2,4(1H,3H)-dione | 2-amino-3-methoxybenzoic acid |
| **81** | 8-Chloroquinazoline-2,4(1H,3H)-dione | 2-amino-3-chlorobenzoic acid |

### Method 82

### 2,4-Dichloropyrido[2,3-d]pyrimidine

A suspension of pyrido[2,3-*d*]pyrimidine-2,4(1*H*,3*H*)-dione (Method 74, 6.52 g, 40 mmol) in POCl₃ (70 ml) was heated under reflux for 18 hours. The black coloured solution was concentrated to dryness under vacuum. The residue was treated with ice (100 g) and quickly extracted with chloroform (3 x 150 ml). Combined organic layer was washed with water (100 ml) and dried over MgSO₄, filtered and evaporated to dryness. The solid obtained was triturated with ether (50 ml), filtered and dried to give desired product (3.2 g, 40%). ¹H NMR (CDCl₃) δ 7.74 (m, 1 H), 8.66 (d, J = 2.9 Hz, 1 H), 9.33 (m, 1 H). MS: m/z 202 (M+3), 200 (M+1).

### Methods 83-89

The following compounds were prepared by the procedure of Method 82 using the appropriate starting materials.

| **Method** | **Compound** | **Starting Material** |
|---|---|---|
| **83** | 7-Methyl-2,4-dichloroquinazoline | Method 75 |
| **84** | 6-Methyl-2,4-dichloroquinazoline | Method 76 |
| **85** | 6-Methoxy-2,4-dichloroquinazoline | Method 77 |
| **86** | 2,4,7-Trichloroquinazoline | Method 78 |
| **87** | 2,4,6-Trichloroquinazoline | Method 79 |
| **88** | 8-Methoxy-2,4-dichloroquinazoline | Method 80 |
| **89** | 2,4,8-Trichloroquinazoline | Method 81 |

### Method 90

### (2-Chloro-pyrido[2,3-d]pyrimidin-4yl)-(5-cyclopropyl-2H-pyrazol-3-yl)-amine

To a solution of 3-amino-5-cyclopropyl-1H-pyrazole (800 mg, 6.5 mmol) and diisopropyl ethyl amine (2.51 g, 3.4 ml, 19.5 mmol) in EtOH (20 ml) was added 2,4-dichloropyrido[2,3-d]pyrimidine (Method 82,1.3 g, 6.5 mmol). The mixture was stirred for 15 minutes and the separated solid was filtered, washed with EtOH (5 ml). The solid was taken in a mixture of CHCl₃ (10 ml) and THF (5 ml) and refluxed for 15 minutes. The residue was filtered and dried to give the desired product (800 mg, 46%). ¹H NMR δ 0.73 (m, 2 H), 0.98 (m, 2 H), 1.96 (m, 1 H), 6.51 (s, 1 H), 7.61 (m, 1 H), 9.02 (m, 1 H), 9.03 (m, 1 H). MS: m/z 289 (M+3), 287 (M+1).

### Methods 91-97

The following compounds were prepared by the procedure of Method 90 using the appropriate starting materials.

| **Method** | **Compounds** | **Quinazolines** | **Amine** | **SM** |
|---|---|---|---|---|
| **91** | 2-Chloro-*N*-(5-cyclopropyl-1*H-*pyrazol-3-yl)-7-methylquinazolin-4-amine | 7-methyl,-2,4-dichloroquinazoline | 3-amino-5-cyclopropyl-1*H*-pyrazole | Method 83 |
| **92** | 2-Chloro-*N*-(5-cyclopropyl-1*H*-pyrazol-3-yl)-6-methylquinazolin-4-amine | 6-methyl-2,4-dichloroquinazoline | 3-amino-5-cyclopropyl-1*H*-pyrazole | Method 84 |
| **93** | 2-Chloro-*N*-(5-cyclopropyl-1*H*-pyrazol-3-yl)-6-methoxyquinazolin-4-amine | 6-methoxy-2,4-dichloroquinazoline | 3-amino-5-cyclopropyl-1*H*-pyrazole | Method 85 |
| **94** | 2,7-Dichloro-*N*-(5-cyclopropyl-1*H*-pyrazol-3-yl)quinazolin-4-amine | 2,4,7-trichloroquinazoline | 3-amino-5-cyclopropyl-1*H*-pyrazole | Method 86 |
| **95** | 2,6-Dichloro-*N*-(5-cyclopropyl-1*H*-pyrazol-3-yl)quinazolin-4-amine | 2,4,6-trichloroquinazoline | 3-amino-5-cyclopropyl-1*H*-pyrazole | Method 87 |
| **96** | 2-Chloro-*N*-(5-cyclopropyl-1*H*-pyrazol-3-yl)-8-methoxyquinazolin-4-amine | 8-methoxy-2,4-dichloroquinazoline | 3-amino-5-cyclopropyl-1*H*-pyrazole | Method 88 |
| **97** | 2,8-Dichloro-*N*-(5-cyclopropyl-1*H*-pyrazol-3-yl)quinazolin-4-amine | 2,4,8-trichloroquinazoline | 3-amino-5-cyclopropyl-1*H*-pyrazole | Method 89 |

### Method 98

### 7-Fluoro-2,4-dioxo (1H, 3H) quinazoline

A finely powdered mixture of 4-fluoroanthranilic acid (24.0 g,154.8 mmol) and urea (27.0 g, 450 mmol) was heated at 220°C for 1 hour. During the heating a clear solution was obtained at 190°C and solidified on continued heating. The mixture was cooled to 25°C and water (500 ml) was added to it and boiled for 1 hour and cooled to 25°C. The residue was filtered and dried to give the desired product (21.55 g, 77%). ¹H NMR δ 6.88 (m, 1 H), 6.91 (m, 1 H), 7.94 (m, 1 H).

### Method 99

### 6-Fluoro-2,4-dioxo (1H, 3H) quinazoline

The title compound was prepared in a similar way to Method 98 from 3-fluoro anthranilic acid.

### Method 100

### 7-Fluoro-2.4-dichloroquinazoline

A suspension of 7-fluoro-2,4-dioxo (1*H*, 3*H*) quinazoline (Method 98, 1.8 g, 10 mmol) in POCl₃ (30 ml) was heated under reflux for 72 hours. The brown coloured solution was concentrated to dryness under vacuum. The residue was treated with ice water (50 ml) and filtered. The residue was washed with ice-cold water (10 ml) and dried to give the desired product (1.7 g, 78%). ¹H NMR (CDCl₃) δ 7.92 (m, 1 H), 7.95 (d, J = 2.9 Hz, 1 H), 8.42 (m, 1 H). MS: m/z 219 (M+3), 217 (M+1).

### Method 101

### 6-Fluoro-2,4-dichloroquinazoline

The title compound was prepared in a similar way to Method 100 from 6-fluoro-2,4-dioxo (1*H*, 3*H*) quinazoline (Method 99).

### Method 102

### (2-Chloro-7-fluoro-quinazolin-4yl)-(5-cyclopropyl-2H-pyrazol-3-yl)-amine

To a solution of 3-amino-5-cyclopropyl-1*H*-pyrazole(1.23g, 10 mmol) and diisopropyl ethyl amine (3.87 g, 5.2 ml, 30 mmol) in EtOH (40 ml) was added 7-fluoro-2,4-dichloroquinazoline (Method 100,1.7 g, 7.87 mmol). The mixture was stirred for 45 minutes and the separated solid was filtered, washed with EtOH (5 ml). The solid was taken in a mixture of CHCl₃ (10 ml) and THF (5 ml) and refluxed for 15 minutes. The residue was filtered and dried to give the desired product (1.5 g, 65%). ¹H NMR δ 0.73 (m, 2 H), 0.97 (m, 2 H), 1.94 (m, 1 H), 6.47 (s; 1 H), 7.52 (m, 2 H), 8.74 (m, 1 H), 10.89 (s, 1 H), 12.40 (br s, 1 H). MS: m/z 306 (M+3), 304 (M+1).

### Method 103

### (2-Chloro-6-fluoro-quinazolin-4yl)-(5-cyclopropyl-2H-pyrazol-3-yl)-amine

The title compound was prepared in a similar way to Method 102from 6-fluoro-2,4-dichloroquinazoline (Method 101) and 3-amino-5-cyclopropyl-1H pyrazole.

### Method 104

### (2R)-2-Amino-2-(4-fluorophenyl)ethanol

To a stirred solution of lithium aluminium hydride (1.0 M in THF, 11.9 ml, 11.9 mmol) at 0°C was added 4-(R)-fluorophenylglycine (1.0 g, 5.9 mmol) portion wise over 1 hour. The mixture was stirred at room temperature for 16 hours. Water (0.45 ml), 4 N NaOH (0.45 ml) and water (1.34 ml) was added to the mixture and the mixture was stirred for 10 minutes before being filtered. The filtrate was concentrated to give a yellow residue. Flash chromatography (CombiFlash^{®}, CH₂Cl₂/MeOH/NH₃ = 90/10/1) gave product as a white solid (800 mg, 88%). ¹H NMR δ 3.25 (m, 1 H), 3.30 (m, 1 H), 3.40 (m, 1 H), 3.83 (m, 1H), 4.77 (brs, 1H), 7.06 (m, 2 H), 7.37 (m, 2 H).

### Methods 105-111

The following compounds were prepared by the procedure of Method 104 using the appropriate starting materials.

| **Method** | **Compound** | **Starting material** |
|---|---|---|
| **105** | (2*S*)-2-Amino-2-(4-fluorophenyl)ethanol | 4-(*S*)-fluorophenylglycine |
| **106** | 2-Amino-2-(4-fluorophenyl)ethanol | 4-fluorophenylglycine |
| **107** | (3R)-3-Amino-3-(4-fluorophenyl)propanol | (3*S*)-3-amino-3-(4-fluorophenyl)propanoic acid |
| **108** | 2-Amino-2-(2-thienyl)ethanol | amino(2-thienyl)acetic acid |
| **109** | 3-Amino-2-methyl-3-phenylpropan-1-ol | 3-amino-2-methyl-3-phenylpropanoic acid |
| **110** | tert-Butyl [1-(4-fluorophenyl)-2-hydroxyethyl]carbamate | [(tert-butoxycarbonyl)amino](4-fluorophenyl)acetic acid |
| **111** | tert-Butyl [(1*S*)-1-(4-fluorophenyl)-3-hydroxypropyl]carbamate | (3*S*)-3-[(tert-butoxycarbonyl)amino]-3-(4-fluorophenyl)propanoic acid |
| **112** | tert-Butyl [(1*R*)-1-(4-fluorophenyl)-3-hydroxyethyl]carbamate | (3*R*)-3-[(tert-butoxycarbonyl)amino]-3-(4-fluorophenyl)acetic acid |

### Method 113

### (R)-1-(4-Fluorophenyl)-2-(methoxy)ethylamine

The title compound was prepared according to the procedure of Russell, M. G. N et al J. Med. Chem. 1999, 42, 4981-5001.

### Method 114

### 1-(4-Fluorophenyl)-2-morpholin-4-ylethanamine

To a solution of *tert*-butyl [1-(4-fluorophenyl)-2-hydroxyethyl]carbamate (Method 110; 1.42 g, 5.58 mmol) in DCM (20 ml) was added triethylamine (1.2 ml) and methanesulfonyl chloride (0.52 ml, 6.69 mmol) and the reaction mixture was stirred at room temperature for 2.5 hours. The reaction mixture was poured into water and extracted with DCM. The combined organic layers were dried and concentrated. Column chromatography (25 %EtOAc to 50% EtOAc in hexanes) gave the desired product as a white solid (1.61 g). To a solution of 2-[(*tert*-butoxycarbonyl)amino]-2-(4-fluorophenyl)ethyl methanesulfonate obtained above (307 mg, 0.92 mmol) in THF (3 ml) was added morpholine (0.45 ml, 5.2 mmol) and the reaction mixture was heated at 70°C for 14 hours (precipitation was observed during this period). The mixture was concentrated and column chromatography (25% EtOAc to 75% EtOAc in hexanes) gave the desired product as an oil (93.4 mg, 31%). To a solution of this oil (93.4 mg, 0.29 mmol) in DCM (5 ml) was added trifluoroacetic acid (5 ml) and the reaction mixture was stirred at room temperature for 4 hours. Solvent was removed and water was added to the mixture. The solution was basified and extracted with chloroform/2-propanol (3/1). The organic layer was dried (Na₂SO₄) and concentrated to give a yellow oil (75 mg, quantitative yield). ¹H NMR (CDCl₃): δ 2.25-2.50 (m, 6 H), 3.70-3.75 (m, 4 H), 4.10 (m, 1H), 6.90 (m, 2 H), 7.25 (m, 2 H).

### Method 115

### 5-Bromo-2-chloro-N-(5-methoxy-1H-pyrazol-3-yl)pyrimidin-4-amine

3-Methoxy-3-(methylthio)acrylonitrile (Method 116; 353 mg, 2.7 mmol) in hydrazine monohydrate (1.5 ml) was heated at 120°C for 1 hour. Hydrazine was removed under vacuum. NMR indicated the major product was 3-methoxy-1*H*-pyrazol-5-amine.¹ To the crude product was added EtOH (5 ml), 5-bromo-2,4-dichloropyrimidine (1.9 g, 8 mmol) and triethylamine (1.2 ml, 8 mmol) and the reaction stirred at room temperature overnight. Solvent was removed and the residue partitioned between EtOAc and H₂O. The organic layer was purified by column chromatography (ISCO system, 20%-50% EtOAc in hexanes) to give the title compound as a solid (94 mg, 11% for two steps).¹H NMR (CD₃OD): δ 3.87 (s, 3 H), 5.84 (s, 1 H), 8.38 (s, 1 H).
¹JP 01047769

### Method 116

### 3-Methoxy-3-(methylthio)acrylonitrile¹

To a solution of *n*-BuLi (2.5 M in Hexane, 11.0 ml, 0.0275 mol) in THF (17.5 ml) at - 78°C was added a solution of CH₃CN (1.0 g, 1.3 ml, 0.025 mol) in THF (25 ml). After stirring at -78°C for 1 hour, O,S dimethyl dithiocarbonate (Method 117; 3.05 g, 0.025 mol) in THF (5 ml) was added, and after a further 1 hour at -78°C the reaction mixture was warmed to room temperature and the solvent removed by evaporation. The residue was triturated with hexane and dried under high vacuum to give a pale yellow foam.

To a solution of the crude lithium salt (0.025 mol) in EtOH (8 ml) was added CH₃I (3.9 g, 1.71 ml, 0.0275 mol) and the reaction mixture then stirred at room temperature overnight. Solvent was removed and the residue partitioned between Et₂O and H₂O. The organic layer was washed with brine, dried (Na₂SO₄) and concentrated, and the residue purified by vacuum distillation to give the title compound as a mixture of E and Z isomers (890 mg, 28 % for two steps). ¹H NMR (CDCl₃): δ 2.37 (s, 3 H), 3.80 (s, 3 H), 4.45 (s, 1 H); the other isomer: δ 2.33 (s, 3 H), 4.02 (s, 3 H), 4.28 (s, 1 H).
¹Liebigs Ann. Chem. 1973, 1637-1643

### Method 117

### O,S-Dimethyl dithiocarbonate¹

To a suspension of granulated KOH (9.3 g, 0.166 mol) in dry Et₂O and benzene (1:1, 70 ml), was added MeOH (5.3 g, 6.7 ml, 0.166 mol). The reaction mixture was cooled to 6°C and a solution of CS₂ (12.6 g, 10.0 ml, 0.166 mol) in benzene (7.5 ml) was added dropwise. After 5 hrs at 6°C, Me₂SO₄ (20.9 g, 15.7 ml, 0.166 mol) was added and the reaction mixture then stirred at room temperature for 20 hours. The organic layer was decanted, washed sequentially with 0.1 N HCl, saturated NaCl and half saturated NaCl. Solvent was removed and the residue was purified by vacuum distillation to give the title compound (11.5 g, contaminated with about 15 % S,S-dimethyl dithiocarbonate, 57 %).¹H NMR: δ 2.55 (s, 3 H), 4.14 (s, 3 H).
1J. Org. Chem 1996, 4175

### Method 118

### 2.5-Dichloro-N-[5-(methylthio)-1H-pyrazol-3-yl]pyrimidin-4-amine

3,3-Bis(methylthio)acrylonitrile (Method 119; 300 mg, 2 mmol) in H₂NNH₂.H₂O(1 ml) was heated at 120°C for 1 hour. Hydrazine was removed under vacuum to give a pale green oil (267 mg). NMR indicated the major product was 3-(methylthio)-1*H*-pyrazol-5-amine. EtOH (3 ml), 2,4,5-trichloropyrimidine (757 mg, 4 mmol), and Et₃N (0.86 ml, 6 mmol) were added to the crude product and the mixture was stirred at room temperature overnight. Solvent was removed and the residue partitioned between EtOAc and H₂O. The organic layer was purified by column chromatography (ISCO system, 20%-50% EtOAc in hexanes) to give the title compound as a white solid (272 mg, 48% for two steps). ¹H NMR (CD₃OD): δ 2.49 (s, 3 H), 6.72 (s, 1 H), 8.24 (s, 1 H).

### Method 119

### 3,3-Bis(methylthio)acrylonitrile¹

n-Butyllithium (2.5 M in hexane, 20.0 ml, 0.05 mol) was added dropwise to a solution of N, N-diisopropylamine (7.07 ml, 0.05 mol) in Et₂O (32 ml) at -10°C. After stirring for 10 min, the solution was cooled to -70°C and CH₃CN (1.3 ml, 0.025 mol) in Et₂O (3.8 ml) was added dropwise, maintaining the temperature below -65°C. CS₂ (1.5 ml, 0.025 mol) in Et₂O (3.8 ml) was added dropwise. The reaction mixture turned yellow, with precipitation, and was then stirred at 0°C for 1 hour. The yellow solid was filtered under nitrogen, washed with ether, and dried under vacuum to give 3.67 g orange solid.

To a solution of crude dilithium salt (0.025 mol) in DMF (37 ml) at 0°C was added a solution of CH₃I (4.78 ml, 0.076 mol) in DMF (11 ml) maintaining the temperature below 20°C, and the reaction mixture then stirred at RT for 1 hour. The reaction was diluted with water, extracted with ether and the organic layer washed with water and brine, dried (Na₂SO₄) and concentrated. The crude product was purified by column chromatography (ISCO system, 0%-30% EtOAc in hexanes) to give the title compound as a pale yellow solid (2.1 g, 58% for two steps). ¹H NMR: δ2.47 (s, 3 H), 2.54 (s, 3 H), 5.46 (s, 1 H).
¹Syn. Comm. 1988, 1103-1110

### Method 120

### 2,5-Dichloro-N-(1H-pyrazol-5-yl)pyrimidin-4-amine

The title compound was prepared by the procedure of Method 1 using the appropriate starting materials.

### Method 121

### N³-(2,5-Dichloropyrimidin-4-yl)-N⁵,N⁵-dimethyl-1H-pyrazole-3.5-diamine

EtOH (3 ml), 2,4,5-trichloropyrimidine (141 mg, 0.77 mmol), and Et₃N (0.13 ml, 0.9 mmol) were added to *N*⁵,*N*⁵-dimethyl-1*H*-pyrazole-3,5-diamine (Method 122; 97 mg, 0.77 mol) and the reaction mixture was stirred at room temperature overnight. Solvent was removed and the residue partitioned between EtOAc and H₂O . The organic layer was purified by column chromatography (ISCO system, 15%-80% EtOAc in hexane) to give the title compound as a pale yellow solid (76 mg, 36%). ¹H NMR (CD₃OD): δ 2.86 (s, 6 H), 5.86 (s, 1 H), 8.24 (s, 1 H).

### Method 122

### N⁵,N⁵-Dimethyl-1H-pyrazole-3,5-diamine¹

(Z)-3-(Dimethylamino)-3-(methylthio)acrylonitrile (Method 123; 150 mg, 1.0 mmol) and hydrazine (0.5 ml) in EtOH (5 ml) were refluxed overnight. Solvent was removed and the residue was purified by column chromatography (ISCO system, 3-25% MeOH in CH₂Cl₂) to give the title compound (97 mg, 71 %). ¹H NMR (CD₂Cl₂): δ 2.79 (s, 6 H), 4.78 (s, 1 H), 6.99 (br s, 2 H).
¹WO03045379

### Method 123

### (Z)-3-(Dimethylamino)-3-(methylthio)acrylonitrile¹

To a solution of 2-cyano-3,3-bis(methylthio)acrylic acid (Method 124, 422 mg, 2.2 mmol) in MeOH (5 ml) was added dimethylamine (2.0 M in THF, 2.0 ml, 4 mmol) and Et₃N (31 µl, 2.2 mmol). The reaction mixture was stirred at 26°C overnight. Solvent was removed and the residue was purified by column chromatography (ISCO system, 0-20% MeOH in CH₂Cl₂) to give the title compound (160 mg, 50%). ¹H NMR (CDCl₃): δ 2.39 (s, 3 H), 3.03 (s, 6 H), 4.08 (s, 1 H).
¹WO03045379

### Method 124

### 2-Cyano-3.3-bis(methylthio)acrylic acid¹

To a solution of ethyl cyanoacetate (13.3 ml, 0.125 mol) and CS₂ (7.5 ml, 0.125 mol) in EtOH (150 ml) at 0°C was added a solution of NaOH (10 g, 0.25 mol) in water (10 ml) at a rate that the temperature did not exceed 10°C. The reaction mixture was warmed to room temperature for 10 min, and cooled to 5°C. The resulting precipitate was filtered, washed with EtOH (30 ml) and H₂O (100 ml) and dried under high vacuum to afford the disodium salt (29 g).

The disodium salt (13 g, 0.05 mol) was added to a solution of NaOH (3.22 g, 0.08 mol) in water (23 ml) and the reaction mixture stirred at 40°C for 5 hours. After cooling to room temperature, dry EtOH (41 ml) was added, and the mixture stirred at room temperature for 5 minutes. The layers were separated and the lower layer diluted with water to a total volume of 80 ml. The solution was cooled to 5°C and dimethyl sulfate (7.4 ml, 0.078 mol) added at a rate that maintained the temperature at 5-15°C. After stirring at RT for 1 hour, the solution was cooled to 15°C and filtered. The filtrate was acidified to pH 1.5-2 with 4 N HCl, and the resulting solid filtered, washed with water, and dried under high vacuum to give the title compound (439 mg, 44% over 3 steps). ¹H NMR: δ 2.58 (s, 3 H), 2.69 (s, 3 H), 13.43 (br s, 1H).
¹Acta Chem. Scand. 1996, 432

### Method 125

### [(1S)-1-(4-Fluorophenyl)-3-morpholin-4-ylpropyl]amine

To a solution of tert-butyl [(1S)-1-(4-fluorophenyl)-3-oxopropyl]carbamate (Method 126; 199 mg, 0.744 mmol) in DCM (10 ml) was added morpholine (0.1 ml, 1.14 mmol). To the mixture was added sodium triacetoxyborohydride (250 mg, 1.18 mmol) and the reaction mixture was stirred at room temperature for overnight. The mixture was poured into water and was extracted with DCM. The combined organic layers were washed with brine, dried (Na₂SO₄) and concentrated. Column chromatography (25% EtOAc in hexanes to 100% EtOAc) gave the desired product as an oil (183 mg, 73%). To a solution of this oil (183 mg, 0.54 mmol) in DCM (5 ml) was added trifluoroacetic acid (5 ml) and the reaction mixture was stirred at room temperature for 4 hrs. Solvent was removed and water was added to the mixture. The solution was basified and extracted with chloroform/2-propanol (3/1). The organic layer was dried (Na₂SO₄) and concentrated to give a yellow oil (178 mg, quantitative yield). ¹H NMR (CDCl₃): δ 1.50 (m, 4 H), 2.10-2.40 (m, 6 H), 3.55 (m, 4 H), 3.85 (m,1H), 6.85(m,2H),7.15(m,2H).

### Method 126

### tert-Butyl [(1S)-1-(4-fluorophenyl)-3-oxopropyl]carbamate

Oxalyl chloride (0.16 ml,1.84 mmol) was added to anhydrous DCM (10 ml) and the mixture was cooled to -78°C. DMSO (0.29 ml, 4.09 mmol) was added followed by slow addition of *tert*-butyl [(1*S*)-1-(4-fluorophenyl)-3-hydroxypropyl]carbamate (Method 111; 447 mg, 1.66 mmol) in DCM (5 ml) to the mixture. The reaction mixture was stirred at -78°C for 15 min and to it was added diisopropylethylamine (1.44 ml, 8.3 mmol). The mixture was further stirred for 6 hours. The mixture was poured into water and extracted with DCM. The combined organic layers were washed with brine and dried (Na₂SO₄) and concentrated. Column chromatography (25% EtOAc to 50% EtOAc in hexanes) gave the desired aldehyde as an oil (397 mg, 90%). ¹H NMR (CDCl₃): δ 1.30 (s, 9 H), 2.80 (m, 2 H), 4.90 (m, 1H), 5.05 (m, 1 H), 6.90 (m, 2 H), 7.20 (m, 2 H), 9.60 (s, 1 H).

### Method 127-132

Following a similar procedure to Method 125, the following compounds were synthesized via reaction of tert-butyl [(1*S*)-1-(4-fluorophenyl)-3-oxopropyl]carbamate (Method 126) and a suitable amine.

| **Method** | **Compound** | **Amine** |
|---|---|---|
| **127** | [(1S)-1-(4-Fluorophenyl)-3-pyrrolidin-1-ylpropyl]amine | pyrrolidine |
| **128** | (1S)-N³,N³-Diethyl-1-(4-fluorophenyl)propane-1,3-diamine | diethylamine |
| **129** | [(1S)-1-(4-Fluorophenyl)-3-(4-methylpiperazin-1-yl)propyl]amine | 1-methylpiperazine |
| **130** | (1S)-1-(4-Fluorophenyl)-N³-(2-methoxyethyl)-N³-methylpropane-1,3-diamine | (2-methoxyethyl)methylamine |
| **131** | 2-{[(3S)-3-Amino-3-(4-fluorophenyl) propyl](methyl)amino}ethanol | 2-(methylamino)ethanol |
| **132** | (1*S*)-1-(4-Fluorophenyl)-*N*³,*N*³-dimethylpropane-1,3-diamine | Dimethylamine |

### Method 133

### (3S)-3-Amino-3-(4-fluorophenyl)-N,N-dimethylpropananiide

A mixture of (3*S*)-3-[(*tert*-butoxycarbonyl)amino]-3-(4-fluorophenyl)propanoic acid (200 mg, 0.74 mmol), EDCI (184 mg, 0.96 mmol), HOBT (160 mg, 1.2 mmol) and dimethylamine (2.0 M in THF, 0.48 ml, 0.96 mmol) was stirred at room temperature for 48 hours. To the mixture was added water and the mixture was extracted with DCM. The combined organic layers were dried and concentrated. Reverse phase HPLC (Gilson) purification (monitored at 220 nm) gave the desired product as a white solid (506 mg, 90%). ¹HNMR (CDCl₃): δ1.50 (s, 9 H), 2.90 (m, 2 H), 3.10 (m, 6 H), 5.15 (m, 1 H), 7.10 (m, 2 H), 7.40 (m, 2 H).

To a solution of above solid in DCM (5 ml) was added trifluoroacetic acid (5 ml) and the reaction mixture was stirred for 2 hours. Solvent was removed and the residue was basified with K₂CO₃/H₂O. The mixture was extracted with CHCl₃/2-propanol (3:1). The combined organic layers were concentrated to give a yellow oil (130 mg, 93%). ¹H NMR (CDCl₃): δ 1.80 (s, 2 H), 2.50 (m, 2 H), 2.90 (m, 6 H), 4.45 (m, 1 H), 6.90 (m, 2 H), 7.30 (m, 2H).

### Method 134-5

Following a similar procedure to Method 133, the following compounds were synthesized via reaction of a suitable acid and a suitable amine.

| **Method** | **Compound** | **Acid** | **Amine** |
|---|---|---|---|
| **134** | (3*S*)-3-Amino-3-(4-fluorophenyl)-*N-* methylpropanamide | (3*S*)-3-[(*tert*- butoxycarbonyl)amino]-3-(4-fluorophenyl)propanoic acid | methylamine |
| **135** | (3*S*)-3-Amino-3-(4-fluorophenyl)-*N*-(2-hydroxyethyl)propanamide | (3*S*)-3-[(*tert*- butoxycarbonyl)amino]-3-(4-fluorophenyl)propanoic acid | 2-aminoethanol |

### Method 136

### tert-Butyl [(2R)-2-amino-2-(4-fluorophenyl)ethyl]carbamate

To a solution of 2-[(1R)-2-amino-1-(4-fluorophenyl)ethyl]-1H isoindole-1,3(2H)-dione (Method 138; 774 mg, 2.4 mmol) in THF (10 ml) was added di-tert-butyl dicarbonate (948 mg, 4.35 mmol) and Et₃N (1.1 ml, 7.9 mmol) and the mixture was stirred at room temperature overnight. Water was added and the mixture was extracted with EtOAc. The combined organic layers were dried and concentrated to give a colorless oil (1.02 g). To a solution of this oil (720 mg, 1.87 mmol) in EtOH (3 ml) was added 33% MeNH₂ in EtOH (6 ml). The reaction mixture was stirred at room temperature for 5 minutes and then was heated at 80°C for 2 hours. Solvent was removed and reverse phase HPLC (Gilson) gave the desired product as a colorless oil (178 mg, 37%). ¹H NMR (CDCl₃): δ 1.80 (s, 9 H), 3.10 - 3.25 (m, 2 H), 4.00 (m, 1 H), 4.25 (m, 1 H), 4.80 (m, 1 H), 6.80 (m, 2 H), 7.20 (m, 2 H).

### Method 137

### N-[(2R)-2-Amino-2-(4-fluorophenyl)ethyl]acetamide

The title compound was synthesized in a similar way to Method 136 except that acetyl chloride was used instead of di-tert-butyl dicarbonate.

### Method 138

### 2-[(1R)-2-Amino-1-(4-fluorophenyl)ethyl]-1H-isoindole-1,3(2H)-dione

To a solution of (3*S*)-3-(1,3-dioxo-1,3-dihydro-2*H*-isoindol-2-yl)-3-(4-fluorophenyl)propanoic acid (Method 139; 1.73 g, 5.5 mmol) in THF (10 ml) was added ethylchloroformate (0.53 ml, 5.5 mmol) and Et₃N (0.77 ml, 5.5 mmol) at 0°C. The solution became cloudy and the stirring was continued for 1 hour. A solution of NaN₃ (729 mg, 11.2 mmol) in water (5 ml) was added and the mixture was stirrd for 1 hour. The mixture was poured into water and was extracted with EtOAc and dried over anhydrous Na₂SO₄ to give a yellow oil (1.567 g). This oil was dissolved in toluene (20 ml) and was heated at 110°C for 1.5 hours. Evaporation of solvent gave a brown oil (1.555 g, 91%). To a solution of the brown oil (1.26 g, 4.1 mmol) in dioxane (10 ml) and water (2 ml) was added concentrated HCl (0.2 ml) at 0°C. The reaction mixture was stirred at room temperature for 2 hours. Solvent was removed and ether was added. The solid was then filtered to give the desired product as a white solid (1.2 g, 92%). The product was used without further purification.

### Method 139

### (3S)-3-(1,3-Dioxo-1,3-dihydro-2H-isoindol-2-yl)-3-(4-fluorophenyl)propanoic acid

A mixture of phthalic anhydride (890 mg, 6.0 mmol) and (3*S*)-3-amino-3-(4-fluorophenyl)propanoic acid (1.1 g, 6.0 mmol) in DMF (5 ml) was heated at 135 °C overnight. Water was added and the white solid was filtered and the solid was washed with water and dried to give the title compound (1.73 g, 93%). ¹H NMR: δ 3.20 (m, 1 H), 3.30 (m, 1 H), 5.50 (m, 1 H), 7.05 (m, 2 H), 7.30 (m, 2 H), 7.80 (m, 4 H).

### Method 140

### 4-Oxo-piperidine-1,3-dicarboxylic acid 1-benzyl ester 3-ethyl ester

A mixture of 1-benzyl-3-carbethoxy-4-piperidone hydrochloride (89.0 g, 298.9 mmol) and palladium hydroxide (4.2 g, 5.9 mmol) in MeOH (700 ml) was degassed and shaken under 40 psi of H₂ overnight. The reaction mixture was filtered through a pad of celite, and concentrated. The resulted solid was dissolved in DCM (800 ml), to which was added triethyl amine (90.7 g, 897 mmol). The solution was cooled to 0 °C followed by addition of benzyl chloroformate (56.1 g, 329 mmol). The reaction mixture was stirred at 25 °C for 15 hours, quenched with water (500 ml), extracted with DCM (2 x 400 ml), dried over MgSO₄, and concentrated. The crude material was purified by silica-gel chromatography (hexanes: EtOAc = 5: 1) to give the title compound (72 g, 78%) and used without further analysis.

### Method 141

### 4-Hydroxy-2-methylsulfanyl-7,8-dihydro-5H-pyrido[4,3-d]pyrimidine-6-carboxylic acid benzyl ester

Sodium metal (1.36 g, 59 mmol) was added to MeOH (250 ml) and the mixture was stirred until the metal sodium completely disappeared. This was added to a solution of 4-oxo-piperidine-1,3-dicarboxylic acid 1-benzyl ester 3-ethyl ester (Method 140; 10.0 g, 32.8 mmol) in MeOH (50 ml), followed by addition of 2-methyl-2-thiopseudourea sulfate (8.2 g, 29.5 mmol). The reaction was stirred at 25 °C for 15 hours and concentrated under reduced pressure. The resulted residue was treated with water (200 ml), extracted with EtOAc (3 x 200 ml), washed with brine (200 ml), dried over MgSO₄, and concentrated to give the title compound (7.0 g, 64%) and used without further analysis.

### Method 142

### 4-Chloro-2-methylsulfanyl-7,8-dihydro-5H-pyrido[4 3-d]pyrimidine-6-carboxylic acid benzyl ester

4-Hydroxy-2-methylsulfanyl-7,8-dihydro-5*H*-pyrido[4,3-*d*]pyrimidine-6-carboxylic acid benzyl ester (Method 141; 7.0 g, 21 mmol) was dissolved in chloroform (100 ml), to which was added POCl₃ (17.8 g, 116 mmol) slowly. The reaction was refluxed for 15 hours, cooled to 25 °C, quenched by pouring onto ice, and extracted with DCM (3 x 200 ml). The combined organic layer was washed with aqueous NaOH solution (1 N, 100 ml), dried over MgSO₄, and concentrated. The resulted residue was then purified by silica-gel column chromatography (DCM: MeOH = 50: 1) to give the title compound (4.0 g, 54%) and used without further analysis.

### Method 143

### 4-(5-Cyclopropyl-1H-pyrazol-3-ylamino)-2-methylsulfanyl-7,8-dihydro-5H-pyrido[4,3-d]pyrimidine-6-carboxylic acid benzyl ester

A solution of 4-chloro-2-methylsulfanyl-7,8-dihydro-5*H*-pyrido[4,3-*d*]pyridine-6-carboxylic acid benzyl ester (Method 142; 2.0 g, 5.7 mmol), 5-cyclopropyl-1*H*-pyrazol-3-ylamine (0.7 g, 5.7 mmol) and triethylamine (1.7 g, 17 mmol) in NMP (15 ml) was heated to 110 °C for 48 hours, cooled to 25 °C, quenched with H₂O (30 ml), and extracted with methyl tert-butyl ether (4 x 50 ml). The combined organic layer was dried over MgSO₄ concentrated under reduced pressure, and purified by column chromatography (DCM: MeOH = 100: 1) to give the title compound (1.4 g, 56%). MS: Calcd.: 436; Found: [M+H]⁺437.

### Method 144

### 4-(5-Cyclopropyl-1H-pyrazol-3-ylamino)-2-methanesulfonyl-7,8-dihydro-5H-pyrido[4,3-d]pyrimidine-6-carboxylic acid benzyl ester

4-(5-Cyclopropyl-1*H*-pyrazol-3-ylamino)-2-methylsulfanyl-7,8-dihydro-5*H-*pyrido[4,3-*d*]pyrimidine-6-carboxylic acid benzyl ester (Method 143; 0.35 g, 0.80 mmol) was dissolved in DCM (50 ml) and cooled to 0 °C, to which was added a solution of meta-chloroperoxybenzoic acid (0.99 g, 4.0 mmol) in DCM (30 ml) over 30 minutes. The reaction was then stirred for an additional 2 hours at 0 °C, quenched with 10% Na₂S₂O₃ (100 ml) washed with saturated NaHCO₃ (100 ml), and extracted with DCM (2 x 100 ml). The combined organic layer was dried over Na₂SO₄, concentrated, and purified by column chromatography (DCM: MeOH = 100 : 1) to give the title compound (0.19 g, 5 %). MS: Calcd.: 468; Found: [M+H]⁺469.

### Method 145

### 3-Oxo-piperidine-1,4-dicarboxylic acid 1-benzyl ester 4-ethyl ester

Ethyl *N*-benzyl-3-oxo-4-piperidine-carboxylate hydrochloride (75.0 g, 251.9 mmol) was dissolved in 700 ml of MeOH and placed in a Parr container. Palladium hydroxide (4.2 g, 5.9 mmol) was then added. The reaction was shaken under 40 psi of H₂ overnight. The reaction mixture was then filtered through a pad of celite and concentrated under reduced pressure. The resulted solid together with triethyl amine (72.6 g, 717 mmol) was dissolved in DCM (800 ml) and cooled to 0 °C, to which was benzyl chloroformate (53.0 g, 311 mmol). The reaction mixture was stirred at 25 °C for 15 hours, diluted with H₂O (500 ml), and extracted with DCM (2 x 400 ml). The combined organic layer was dried over MgSO₄, concentrated, and purified by silica-gel chromatography (hexane: EtOAc =5: 1) to give the title compound (73 g, 97%) and used without further analysis.

### Method 146

### 4-Hydroxy-2-methylsulfanyl-5,8-dihydro-6H-pyrido[3,4-d]pyrimidine-7-carboxylic acid benzyl ester

Sodium metal (1.36 g, 59 mmol) was added to MeOH (250 ml) and stirred until the sodium completely disappeared, to which was added the MeOH (50 ml) solution of 3-oxo-piperidine-1,4-dicarboxylic acid 1-benzyl ester 4-ethyl ester (Method 145; 10.0 g, 32.8 mmol) followed by addition of 2-methyl-2-thiopseudourea sulfate (8.2 g, 29.5 mmol). The reaction was stirred at 25 °C for 15 hours and concentrated under reduced pressure. The resulted residue was dissolved in H₂O (200 ml) and extracted with EtOAc (3 x 200 ml). The combined organic layer was dried over MgSO₄ and concentrated to give the title compound (8.0 g, 74%) and used without further analysis.

### Method 147

### 4-Chloro-2-methylsulfanyl-5,8-dihydro-6H-pyrido[3,4-d]pyrimidine-7-carboxylic acid benzyl ester

POCl₃ (22.9 g, 149 mmol) was slowly added to the chloroform (100 ml) solution of 4-hydroxy-2-methylsulfanyl-5,8-dihydro-6*H*-pyrido[3,4-*d*]pyrimidine-7-carboxylic acid benzyl ester (Method 146; 9.0 g, 27 mmol). The reaction was heated to reflux for 15 hours, cooled to 25 °C, quenched by pouring onto ice, and extracted with DCM (3 x 200 ml). The combined organic layer was washed with aqueous NaOH solution (1 N, 100 ml), dried over MgSO₄, and purified by column chromatography (DCM: MeOH = 100: 1) to give the title compound (7.0 g, 73%) and used without further analysis.

### Method 148

### 4-(5-Cyclovropyl-1H-pyrazol-3-ylamino)-2-methylsulfanyl-5,8-dihydro-6H-pyrido[3,4-d]pyrimidine-7-carboxylic acid benzyl ester

A solution of 4-chloro-2-methylsulfanyl-5,8-dihydro-6*H*-pyrido[3,4-*d*]pyrimidine-7-carboxylic acid benzyl ester (Method 147; 5.0 g, 14.0 mmol), triethylamine (4.3 g, 43 mmol), and 5-cyclopropyl-1*H-*pyrazol-3-ylamine (2.6 g, 21 mmol) in NMP (30 ml) was heated at 110 °C for 48 hours. After cooling to 25 °C, the reaction was diluted with H₂O (30 ml), extracted with MTBE (4 x 50 ml). The combined organic layer was dried over MgSO₄, concentrated, and purified by column chromatography (DCM: MeOH = 50: 1) to give the title compound (2.5 g, 40%). MS: Calcd.: 436; Found: [M+H]⁺ 437.

### Method 149

### 4-(5-Cyclopropyl-1H-pyrazol-3-ylamino)-2-methanesulfonyl-5,8-dihydro-6H-pyrido[3,4-d]pyrimidine-7-carboxylic acid benzyl ester

4-(5-Cyclopropyl-1*H*-pyrazol-3-ylamino)-2-methylsulfanyl-5,8-dihydro-6*H-*pyrido[3,4-*d*]pyrimidine-7-carboxylic acid benzyl ester (Method 148; 2.5 g, 5.75 mmol) was dissolved in DCM (200 ml) and cooled to 0 °C, to which was added a DCM (50 ml) solution of MCPBA (5.0 g, 20.0 mmol) over a period of 1 hour. The reaction was then stirred for an additional 2 hrs at 0 °C, quenched with 10% Na₂S₂O₃ (200 ml), washed with saturated NaHCO₃ (200 ml), and extracted with DCM (2 x 200 ml). The combined organic layer was dried over Na₂SO₄ concentrated, and purified by column chromatography (DCM: MeOH = 100: 1) to give the title compound (2.0 g, 74%). MS: Calcd.: 468; Found: [M+H]⁺ 469.

### Method 150

### 2,6-Dichloro-N-(5-methyl-1H-pyrazol-3-yl)pyrimidin-4-amine

A mixture of 2,4,6-trichloropyrimidine (1.00 g, 5.46 mmol), 3-amino-5-methyl-1*H-*pyrazole (530 mg, 5.46 mmol) and triethylamine (1.1 ml, 8.2 mmol) in EtOH (10 ml) was stirred at room temperature for 1 day. Solvent was removed and the mixture was partitioned between EtOAc and water. The organic layer was concentrated to give the desired product (1.34 g, quantitative). ¹H NMR (CDCl₃): δ 2.30 (m, 3 H), 5.90 (m, 1 H), 7.85 (m, 1H), 8.50 (br s, 1 H).

### Method 151

### [(1R)-1-(4-Fluorophenyl)-2-morpholin-4-ylethyl]amine

The compound was synthesized following Method 114 using *tert*-butyl [(1*R*)-1-(4-fluorophenyl)-2-hydroxyethyl]carbamate (Method 112) as starting material.

### Utility

The compounds of the present invention have utility for the treatment of cancer by inhibiting the tyrosine kinases, particularly the Trks and more particularly Trk A and B. Methods of treatment target tyrosine kinase activity, particularly the Trk activity and more particularly Trk A and B activity, which is involved in a variety of cancer related processes. Thus, inhibitors of tyrosine kinase, particularly the Trks and more particularly Trk A and B, are expected to be active against neoplastic disease such as carcinoma of the breast, ovary, lung, colon, prostate or other tissues, as well as leukemias and lymphomas, tumours of the central and peripheral nervous system, and other tumour types such as melanoma, fibrosarcoma and osteosarcoma. Tyrosine kinase inhibitors, particularly the Trk inhibitors and more particularly Trk A and B inhibitors are also expected to be useful for the treatment other proliferative diseases including but not limited to autoimmune, inflammatory, neurological, and cardiovascular diseases.

Compounds of the present invention have been shown to inhibit tyrosine kinases, particularly the Trks and more particularly Trk A and B, as determined by the Trk B Assay described herein.

Compounds provided by this invention should also be useful as standards and reagents in determining the ability of a potential pharmaceutical to inhibit tyrosine kinases, particularly the Trks and more particularly Trk A and B. These would be provided in commercial kits comprising a compound of this invention

### TrkB Assay Format

TrkB kinase activity is being measured against its ability to phosphorylate synthetic tyrosine residues within a generic polypeptide substrate using homogenous time-resolved fluorescence (HTRF) technology. The intracellular domain of a HIS-tagged human TrkB kinase was expressed in SF9 cells and purified using standard nickel column chromatography. After the kinase is incubated with a biotinylated substrate and adenosine triphosphate (ATP) for 50 minutes at room temperature, the kinase reaction is stopped by the addition of 60 mM ethylenediaminetetraacetic acid (EDTA). The reaction is performed in 384 well microtitre plates and reaction products are detected with the addition of strepavidin-linked and phosphotyrosine-specific antibodies using the Tecan Ultra Evolution Microplate Fluometer after an additional 3-hour incubation at room temperature.

| | |
|---|---|
| Peptide substrate | PolyEAY-biotin (PGAT-bio.) |
| ATP Km | 60 uM |
| Assay conditions | 400 ng/ml TrkB, 10 mM HEPES, 0.005% BR SA, 20 mM MnCl₂, 100 nM PGAT-bio, 120 nM ATP |
| Incubation | 50 minutes, room temperature |
| Termination/Detection conditions | 50 mM HEPES, 60 mM EDTA, 0.03% BR SA, 5.9 nM p-Tyr LANCE Ab, 45 nM XL-665 Ab |
| Detection incubation | 3 hours, room temperature |
| Fluometer settings | Excitation = 340 nM |
| | Emission 1 = 612 nM |
| | Emission 2 = 670 nM |
| | Flash = 10 |
| | Integration = 200 us |
| | Lad = 50 us |

Although the pharmacological properties of the compounds of the formula (I) vary with structural change, in general activity possessed by compounds of the formula **(I)** may be demonstrated at IC₅₀ concentrations (concentrations to achieve 50% inhibition) or doses in the range of (0.01 µM to 10 µM).

When tested in the above in-vitro assay the Trk inhibitory activity of the following examples was measured at the following IC₅₀s.

| Ex | IC₅₀ |
|---|---|
| 42 | 0.067 µM |
| 64 | 0.059 µM |
| 80 | 0.087 µ M |

## Claims

1. A compound of formula (I): 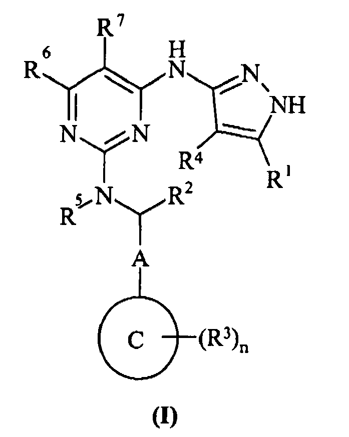 wherein:
**A** is a direct bond;
**Ring C** is carbocyclyl or heterocyclyl;
**R¹** and **R⁴** are independently selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N-*(C₁₋₆alkyl)amino, *N,N*-(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N*,*N*-(C₁₋₆alkyl)₂sulphamoyl, C₁₋₆alkylsulphonylamino, carbocyclyl or heterocyclyl; wherein R¹ and R⁴ independently of each other may be optionally substituted on carbon by one or more R⁸; and wherein if said heterocyclyl contains an -NH- moiety that nitrogen may be optionally substituted by a group selected from R⁹;
**R²** is selected from C₁₋₆alkyl; wherein R² may be optionally substituted on carbon by one or more R¹⁰;
**R¹⁰** is selected from halo, hydroxy, carboxy, amino, C₁₋₆alkoxy, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbamoyl or heterocyclyl; wherein R¹⁰ may be optionally substituted on carbon by one or more R^{19a} ; and wherein if said heterocyclyl contains an -NH- moiety that nitrogen may be optionally substituted by a group selected from R^{20a};
**R^{19a}** is selected from hydroxy or C₁₋₆alkoxy;
**R^{20a}** is selected from C₁₋₆alkyl;
**R³** is fluoro;
**R⁵** is hydrogen or optionally substituted C₁₋₆alkyl; wherein said optional substituents are selected from one or more R¹⁴;
**R⁶** and **R⁷** are independently selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N*,*N*-(C₁₋₆alkyl)₂sulphamoyl, C₁₋₆alkylsulphonylamino, carbocyclyl or heterocyclyl; wherein R⁶ and R⁷ independently of each other may be optionally substituted on carbon by one or more R¹⁵ ; and wherein if said heterocyclyl contains an -NH- moiety that nitrogen may be optionally substituted by a group selected from R¹⁶;
or **R⁶** and **R⁷** together with the pyrimidine bond to which they are attached form a 5 or 6 membered carbocyclic ring or a 5 or 6 membered heterocyclic ring wherein said ring is fused to the pyrimidine of formula **(I);** wherein the double bonds of the resulting bicyclic ring may be further delocalised across the whole of the bicyclic ring; and wherein said carbocyclic ring or heterocyclic ring may be optionally substituted on carbon by one or more R¹⁷ ; and wherein if said heterocyclic ring contains an -NH- moiety that nitrogen may be optionally substituted by a group selected from R¹⁸;
n=1;
**R⁸, R¹⁴, R¹⁵** and **R¹⁷** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl) amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂Carbamoyl, C₁₋₆alkylS(O)ₐ wherein a is 0 to 2, C₁₋₆alkoxycarbonyl, *N*-(C₁₋₆alkyl)sulphamoyl, *N*,*N*-(C₁₋₆alkyl)₂sulphamoyl, C₁₋₆alkylsulphonylamino, carbocyclyl or heterocyclyl; wherein R⁸, R¹⁴, R¹⁵ and R¹⁷ independently of each other may be optionally substituted on carbon by one or more R¹⁹; and wherein if said heterocyclyl contains an -NH- moiety that nitrogen may be optionally substituted by a group selected from R²⁰;
**R⁹, R¹⁶, R¹⁸** and **R²⁰** are independently selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N*,*N*-(C₁₋₆alkyl)₂carbamoyl, benzyl, benzyloxycarbonyl, benzoyl and phenylsulphonyl; wherein R⁹, R¹⁶, R¹⁸ and R²⁰ independently of each other may be optionally substituted on carbon by on or more R²¹;
**R¹⁹** and **R²¹** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N*,*N*-(C₁₋₆alkyl)₂sulphamoyl, C₁₋₆alkylsulphonylamino, carbocyclyl or heterocyclyl; wherein R¹⁹ and R²¹ independently of each other may be optionally substituted on carbon by one or more R²³; and wherein if said heterocyclyl contains an -NH- moiety that nitrogen may be optionally substituted by a group selected from R²⁴;
**R²³** is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N*,*N*-dimethylcarbamoyl, *N*,*N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N*,*N*-dimethylsulphamoyl, *N,N*-diethylsulphamoyl or *N*-methyl-*N*-ethylsulphamoyl; and
**R²⁴** is selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N*,*N*-(C₁₋₆alkyl)₂carbamoyl, benzyl, benzyloxycarbonyl, benzoyl and phenylsulphonyl;
wherein a "carbocyclyl" is a saturated, partially saturated or unsaturated, mono or bicyclic carbon ring that contains 3-12 atoms; wherein a -CH₂- group can optionally be replaced by a -C(O)-;
wherein a "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked,
wherein a -CH₂- group can optionally be replaced by a -C(O)-, and a ring sulphur atom may be optionally oxidised to form the S-oxides;
wherein when "R⁶ and R⁷ together with the bond to which they are attached form a 5 or 6 membered heterocyclic ring" said ring is a partially saturated or unsaturated, mono or bicyclic carbon ring that contains 5 or 6 atoms two atoms of which are shared with the pyrimidine ring of formula **(I);** of which at least one atom is chosen from nitrogen, sulphur or oxygen; wherein a -CH₂- group can optionally be replaced by a -C(O)-, and a ring sulphur atom may be optionally oxidized to form the S-oxides; and
wherein where "R⁶ and R⁷ together with the bond to which they are attached form a 5 or 6 membered carbocyclic ring" said ring is a partially saturated or unsaturated, mono or bicyclic carbon ring that contains 5 or 6 atoms two atoms of which are shared with the pyrimidine ring of formula **(I);** wherein a -CH₂- group can optionally be replaced by a -C(O)-;or a pharmaceutically acceptable salt thereof.

2. A compound of formula **(I),** or a pharmaceutically acceptable salt thereof, according to claim 1 wherein Ring C is phenyl, thienyl, pyridyl,; thiazolyl.

3. A compound of formula **(I),** or a pharmaceutically acceptable salt thereof, according to either claim 1 or claim 2 wherein R¹ is selected from hydrogen, C₁₋₆alkyl, C₁₋₆alkoxy, *N*,*N*-(C₁₋₆alkyl)₂amino, C₁₋₆alkylS(O)ₐ wherein a is 0 or carbocyclyl; wherein R¹ may be optionally substituted on carbon by one or more R⁸; wherein R⁸ is selected from halo or carbocyclyl.

4. A compound of formula **(I),** or a pharmaceutically acceptable salt thereof, according to any one of claims 1-3 wherein R⁴ is hydrogen.

5. A compound of formula **(I),** or a pharmaceutically acceptable salt thereof, according to any one of claims 1-4 wherein R⁵ is hydrogen or optionally substituted C₁₋₆alkyl; wherein said optional substituents are selected from one or more R¹⁴ ; and R¹⁴ is selected from hydroxy.

6. A compound of formula **(I),** or a pharmaceutically acceptable salt thereof, according to any one of claims 1-5 wherein:
R⁶ and R⁷ are independently selected from hydrogen, halo, nitro, cyano, amino, C₁-₆alkyl, *N*-(C₁₋₆alkyl)amino, *N*,*N*-(C₁₋₆alkyl)₂amino, *N*-(C₁₋₆alkyl)carbamoyl, C₁₋₆alkoxycarbonyl or heterocyclyl; wherein R⁶ and R⁷ independently of each other may be optionally substituted on carbon by one or more R¹⁵ ; and wherein if said heterocyclyl contains an -NH- moiety that nitrogen may be optionally substituted by a group selected from R¹⁶;
or R⁶ and R⁷ together with the pyrimidine bond to which they are attached form a 6 membered carbocyclic ring or a 5 or 6 membered heterocyclic ring wherein said ring is fused to the pyrimidine of formula (I); wherein the double bonds of the resulting bicyclic ring may be further delocalised across the whole of the bicyclic ring; and wherein said carbocyclic ring or heterocyclic ring may be optionally substituted on carbon by one or more R¹⁷; and wherein if said heterocyclic ring contains an -NH- moiety that nitrogen may be optionally substituted by a group selected from R¹⁸;
R¹⁵ is selected from halo, hydroxy, amino, C₁₋₆alkoxy, *N*,*N*-(C₁₋₆alkyl)₂amino, carbocyclyl or heterocyclyl; wherein R¹⁵ may be optionally substituted on carbon by one or more R¹⁹ ; and wherein if said heterocyclyl contains an -NH- moiety that nitrogen may be optionally substituted by a group selected from R²⁰;
R¹⁷ is selected from halo, C₁₋₆alkyl or C₁₋₆alkoxy; wherein R¹⁷ may be optionally substituted on carbon by one or more R¹⁹;
R¹⁶ is selected from C₁₋₆alkyl;
R¹⁸ is selected from C₁₋₆alkanoyl;
R¹⁹ is selected from halo, hydroxy, C₁₋₆alkoxy or heterocyclyl; and wherein if said heterocyclyl contains an -NH- moiety that nitrogen may be optionally substituted by a group selected from R²⁴;
R²⁰ is selected from C₁₋₆alkyl; and
R²⁴ is selected from C₁₋₆alkyl.

7. A compound of formula **(I)**, as claimed in claim 1, wherein:
A is a direct bond;
Ring C is phenyl, thienyl, pyridyl, thiazolyl;
R¹ is selected from hydrogen, methyl, ethyl, isopropyl, t-butyl, trifluoromethyl, cyclopropylmethyl, benzyl, methoxy, ethoxy, propoxy, isopropoxy, sec-butoxy, dimethylamino, methylthio or cyclopropyl;
R² is selected from methyl, ethyl, trifluoromethyl, hydroxymethyl, carboxymethyl, aminomethyl, methoxymethyl, morpholinomethyl, 1-hydroxyethyl, 2-hydroxyethyl, 1-carboxyethyl, 2-dimethylaminoethyl, 2-diethylaminoethyl, acetamidomethyl, 2-[*N*-methyl-*N-*(2-methoxyethyl)amino]ethyl, 2-[*N-*methyl-*N-*(2-hydroxyethyl)amino]ethyl, 2-(*N-*methylcarbamoyl)ethyl, 2-[*N-*(2-hydroxyethyl)carbamoyl]ethyl, 2-(*N,N-*dimethylcarbamoyl)ethyl, 2-morpholinoethyl, 2-pyrrolidin-1-ylethyl or 2-(1-methylpiperazin-4-yl)ethyl, 1-methyl-2-hydroxyethyl;
R³ is selected from fluoro;
R⁴ is hydrogen;
R⁵ is hydrogen, methyl or 2-hydroxyethyl;
R⁶ and R⁷ are independently selected from hydrogen, fluoro, chloro, bromo, nitro, cyano, amino, methyl, methylamino, ethylamino, propylamino, isopropylamino, dimethylamino, *N*-methyl-*N*-propylamino, *N*-ethylcarbamoyl, methoxycarbonyl, ethoxycarbonyl, butoxycarbonyl, morpholino, pyrrolidinyl or piperazinyl; wherein R⁶ and R⁷ independently of each other may be optionally substituted on carbon by one or more R¹⁵; and wherein said piperazinyl may be optionally substituted on nitrogen by a group selected from R¹⁶;
or R⁶ and R⁷ together with the pyrimidine to which they are attached form a bicyclic ring selected from quinazolinyl, thieno[3,2-d]pyrimidinyl, thieno[2,3-d]pyrimidinyl, 1*H-*pyrazolo[3,4-d]pyrimidinyl, thieno[3,4-d]pyrimidinyl, pyrido[2,3-d]pyrimidinyl, 5,6,7,8-tetrahydro-pyrido[4,3-d]pyrimidinyl, 5,6,7,8-tetrahydro-pyrido[2,3-d]pyrimidinyl or 5,6,7,8-tetrahydro-pyrido[3,4-d]pyrimidinyl; and wherein said bicyclic ring may be optionally substituted on carbon by one or more R¹⁷; and wherein said 5,6,7,8-tetrahydro-pyrido[4,3-d]pyrimidinyl, 5,6,7,8-tetrahydro-pyrido[2,3-d]pyrimidinyl or 5,6,7,8-tetrahydro-pyrido[3,4-d]pyrimidinyl may be optionally substituted on nitrogen by a group selected from R¹⁸;
R¹⁵ is selected from fluoro, hydroxy, amino, ethoxy, dimethylamino, phenyl, pyrrolidinyl, piperazinyl or morpholino; wherein R¹⁵ may be optionally substituted on carbon by one or more R¹⁹; and wherein said piperazinyl may be optionally substituted on nitrogen by a group selected from R²⁰;
R¹⁶ is selected from methyl;
R¹⁷ is selected from fluoro, chloro, methyl, methoxy, ethoxy or propoxy; wherein R¹⁷ may be optionally substituted on carbon by one or more R¹⁹;
R¹⁸ is selected from acetyl;
R¹⁹ is selected from fluoro, hydroxy, methoxy, piperazinyl, pyrrolidinyl or morpholino; and wherein said piperazinyl may be optionally substituted on nitrogen by a group selected from R²⁴;
R²⁰ is selected from methyl;
R²⁴ is selected from methyl;
n= 1;
or a pharmaceutically acceptable salt thereof.

8. A compound of formula **(I)**, as claimed in claim 1, selected from:
(2*R*)-2-({4-[(5-cyclopropyl-1*H-*pyrazol-3-yl)amino]-5-fluoropyrimidin-2-yl}amino)-2-(4-fluorophenyl)ethanol;
5-bromo-N⁴-(3-cyclopropyl-1*H*-pyrazol-5-yl)-N²-[(1*S*)-1-(4-fluorophenyl)ethyl]pyrimidine-2,4-diamine;
(2*R*)-2-({5-chloro-4-[(3-cyclopropyl-1*H-*pyrazol-5-yl)amino]pyrimidin-2-yl}amino)-2-(4-fluorophenyl)ethanol;
(2R)-2-({5-chloro-4-[(3-isopropoxy-1*H-*pyrazol-5-yl)amino]pyrimidin-2-yl}amino)-2-(4-fluorophenyl)ethanol;
(3*S*)-3-({5-chloro-4-[(5-cyclopropyl-1*H-*pyrazol-3-yl)amino]pyrimidin-2-yl}amino)-3-(4-fluorophenyl)-*N-*methylpropanamide;
2-({5-chloro-2-{[(1S)-1-(4-fluorophenyl)ethyl]amino}-6-[(5-isopropoxy-1H-pyrazol-3-yl)amino]pyrimidin-4-yl}amino)propane-1,3-diol;
2-[(5-chloro-6-[(3-cyclopropyl-1H-pyrazol-5-yl)amino]-2-{[(1S)-1-(4-fluorophenyl)ethyl] amino}pyrimidin-4-yl)amino]propane-1,3-diol;
5-chloro-N⁴-(5-cyclopropyl-1H-pyrazol-3-yl)-N²-[(1S)-(4-fluoro-phenyl)-ethyl]-6-(4-methyl-piperazin-1-yl)-pyrimidine-2,4-diamine;
(2R)-2-({4-[(5-cyclopropyl-1*H-*pyrazol-3-yl)amino]-7-fluoroquinazolin-2-yl}amino)-2-(4-fluorophenyl)ethanol; and
2-[(5-chloro-6-[(5-cyclopropyl-1H-pyrazol-3-yl)amino]-2-{[(1R)-1-(4-fluorophenyl)-2-hydroxyethyl]amino}pyrimidin-4-yl)amino]propane-1,3-diol;
or a pharmaceutically acceptable salt thereof.

9. A process for preparing a compound of formula **(I)** or a pharmaceutically acceptable salt thereof, as claimed in any one of claims 1-8, which process comprises of:
*Process a*) reaction of a pyrimidine of formula **(II)**: 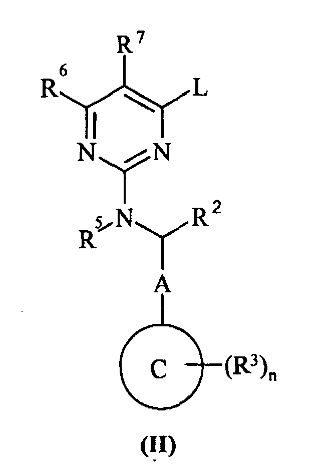 wherein L is a displaceable group; with an pyrazole amine of formula **(III):** 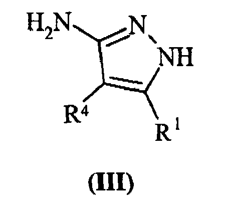 or
*Process b*) reacting a pyrimidine of formula (IV): 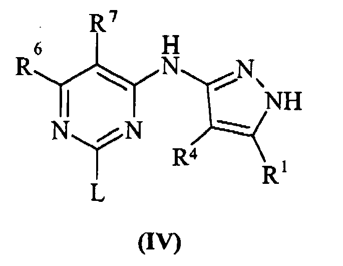 wherein L is a displaceable group; with a compound of formula **(V)**: 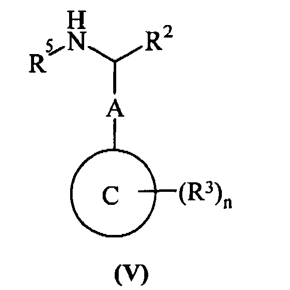
*Process c*) reacting a compound of formula **(VI)**: 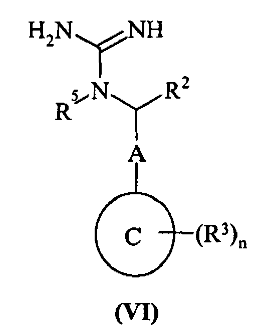 with a compound of formula **(VII):** 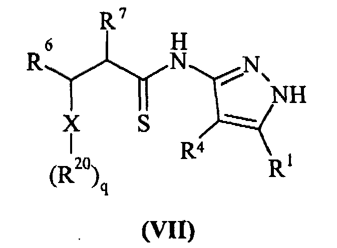 wherein X is an oxygen atom and q is 1; or X is a nitrogen atom and q is 2; and wherein each R²⁰ independently represents a C₁₋₆alkyl group; or
*Process d*) reacting a compound of formula **(VIII):** 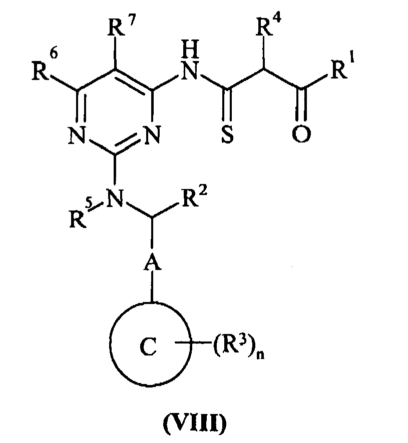 with hydrazine; or
and thereafter if necessary:
i) converting a compound of the formula **(I)** into another compound of the formula **(I);**
ii) removing any protecting groups;
iii) forming a pharmaceutically acceptable salt.

10. A compound of formula **(I),** or a pharmaceutically acceptable salt thereof, as claimed in any one of claims 1-8, for use as a medicament.

11. The use of a compound of formula **(I),** or a pharmaceutically acceptable salt thereof, as claimed in any one of claims 1-8, in the manufacture of a medicament for the inhibition of Trk activity.

12. The use of a compound of formula **(I),** or a pharmaceutically acceptable salt thereof, as claimed in any one of claims 1-8, in the manufacture of a medicament for the treatment or prophylaxis of cancer.

13. The use of a compound of formula **(I),** or a pharmaceutically acceptable salt thereof, as claimed in any one of claims 1-8, in the manufacture of a medicament for the production of an anti-proliferative effect.

14. A pharmaceutical composition comprising a compound of formula **(I)**, or a pharmaceutically acceptable salt thereof, as claimed in any one of claims 1-8, together with at least one pharmaceutically acceptable carrier, diluent or excipient.

15. The use according to claim 12 wherein said cancer is selected from oesophageal cancer, myeloma, hepatocellular, pancreatic, cervical cancer, ewings tumour, neuroblastoma, kaposis sarcoma, ovarian cancer, breast cancer, colorectal cancer, prostate cancer, bladder cancer, melanoma, lung cancer - non small cell lung cancer (NSCLC), small cell lung cancer (SCLC), gastric cancer, head and neck cancer, renal cancer, lymphoma, leukaemia, tumours of the central and peripheral nervous system, melanoma, fibrosarcoma and osteosarcoma.

## Patentansprüche

1. Verbindung der Formel (I): 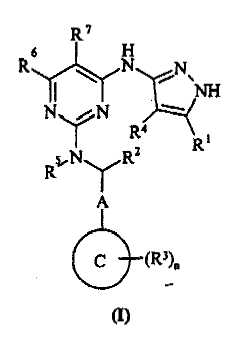 worin:
A für eine direkte Bindung steht;
Ring C für Carbocyclyl oder Heterocylyl steht;
R¹ und R⁴ unabhängig voneinander unter Wasserstoff, Halogen, Nitro, Cyano, Hydroxy, Trifluormethoxy, Amino, Carboxy, Carbamoyl, Mercapto, Sulfamoyl, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*-(C₁₋₆-Alkyl) amino, *N*,*N*-(C₁₋₆-Alkyl)₂amino, C₁₋₆-Alkanoylamino, *N*-(C₁₋₆-Alkyl) carbamoyl , *N*,*N*-(C₁₋₆-Alkyl)₂carbamoyl, C₁₋₆-Alkyl-S(O)ₐ, worin a für 0 bis 2 steht, C₁₋₆-Alkoxycarbonyl, *N*-(C₁₋₆-Alkyl) sulfamoyl, *N*,*N*-(C₁₋₆-Alkyl)₂sulfamoyl, C₁₋₆-Alkylsulfonylamino, Carbocyclyl oder Heterocyclyl ausgewählt sind; worin R¹ und R⁴ unabhängig voneinander gegebenenfalls an Kohlenstoff durch eine oder mehrere Gruppen R⁸ substituiert sein können und worin dann, wenn das Heterocyclyl eine -NH-Gruppierung enthält, dieser Stickstoff gegebenenfalls durch eine unter R⁹ ausgewählte Gruppe substituiert sein kann;
R² unter C₁₋₆-Alkyl ausgewählt ist; worin R² gegebenenfalls an Kohlenstoff durch eine oder mehrere Gruppen R¹⁰ substituiert sein kann;
R¹⁰ unter Halogen, Hydroxy, Carboxy, Amino, C₁₋₆-Alkoxy, *N*,*N*-(C₁₋₆-Alkyl)₂amino, C₁₋₆-Alkanoylamino, *N*-(C₁₋₆-Alkyl) carbamoyl, *N*,*N*-(C₁₋₆-Alkyl)₂carbamoyl oder Heterocyclyl ausgewählt ist; worin R¹⁰ gegebenenfalls an Kohlenstoff durch eine oder mehrere Gruppen R^{19a} substituiert sein kann und worin dann, wenn das Heterocyclyl eine -NH-Gruppierung enthält, dieser Stickstoff gegebenenfalls durch eine unter R^{20a} ausgewählte Gruppe substituiert sein kann;
R^{19a} unter Hydroxy oder C₁₋₆-Alkoxy ausgewählt ist;
R^{20a} unter C₁₋₆-Alkyl ausgewählt ist;
R³ für Fluor steht;
R⁵ für Wasserstoff oder gegebenenfalls substituiertes C₁₋₆-Alkyl steht; worin die fakultativen Substituenten unter einer oder mehreren Gruppen R¹⁴ ausgewählt sind;
R⁶ und R⁷ unabhängig voneinander unter Wasserstoff, Halogen, Nitro, Cyano, Hydroxy, Trifluormethoxy, Amino, Carboxy, Carbamoyl, Mercapto, Sulfamoyl, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*-(C₁₋₆-Alkyl) amino, *N*,*N*-(C₁₋₆-Alkyl)₂amino, C₁₋₆-Alkanoylamino, *N*-(C₁₋₆-Akyl) carbamoyl, *N*,*N*-(C₁₋₆-Alkyl)₂carbamoyl, C₁₋₆-Alkyl-S(O)ₐ, worin a für 0 bis 2 steht, C₁₋₆-Alkoxycarbonyl , *N*-(C₁₋₆-Alkyl)sulfamoyl, *N,N-*(C₁₋₆-Alkyl)₂sulfamoyl, C₁₋₆-Alkylsulfonylamino, Carbocyclyl oder Heterocyclyl ausgewählt sind; worin R⁶ und R⁷ unabhängig voneinander gegebenenfalls an Kohlenstoff durch eine oder mehrere Gruppen R¹⁵ substituiert sein können und worin dann, wenn das Heterocyclyl eine -NH-Gruppierung enthält, dieser Stickstoff gegebenenfalls durch eine unter R¹⁶ ausgewählte Gruppe substituiert sein kann;
oder R⁶ und R⁷ gemeinsam mit der Pyrimidinbindung, an die sie gebunden sind, einen 5- oder 6-gliedrigen carbocyclischen Ring oder einen 5- oder 6-gliedrigen heterocyclischen Ring bilden, worin der Ring an das Pyrimidin der Formel (I) anelliert ist; worin die Doppelbindungen des resultierenden bicyclischen Rings ferner über den gesamten bicyclischen Ring delokalisiert sein können und worin der carbocyclische Ring oder heterocyclische Ring gegebenenfalls an Kohlenstoff durch eine oder mehrere Gruppen R¹⁷ substituiert sein kann und worin dann, wenn das Heterocyclyl eine -NH-Gruppierung enthält, dieser Stickstoff gegebenenfalls durch eine unter R¹⁸ ausgewählte Gruppe substituiert sein kann;
n = 1;
R⁸, R¹⁴, R¹⁵ und R¹⁷ unabhängig voneinander unter Halogen, Nitro, Cyano, Hydroxy, Trifluormethoxy, Amino, Carboxy, Carbamoyl, Mercapto, Sulfamoyl, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*- (C₁₋₆-Alkyl) amino, *N*,*N*-(C₁₋₆-Alkyl)₂amino, C₁₋₆-Alkanoylamino, *N*-(C₁₋₆-Alkyl) carbamoyl , *N*,*N*-(C₁₋₆-Alkyl)₂carbamoyl, C₁₋₆-Alkyl-S(O)ₐ, worin a für 0 bis 2 steht, C₁₋₆-Alkoxycarbonyl, *N*-(C₁₋₆-Alkyl) sulfamoyl , *N,N-*(C₁₋₆-Alkyl)₂sulfamoyl, C₁₋₆-Alkylsulfonylamino, Carbocyclyl oder Heterocyclyl ausgewählt sind; worin R⁸, R¹⁴, R¹⁵ und R¹⁷ unabhängig voneinander gegebenenfalls an Kohlenstoff durch eine oder mehrere Gruppen R¹⁹ substituiert sein können und worin dann, wenn das Heterocyclyl eine -NH-Gruppierung enthält, dieser Stickstoff gegebenenfalls durch eine unter R²⁰ ausgewählte Gruppe substituiert sein kann;
R⁹, R¹⁶, R¹⁸ und R²⁰ unabhängig voneinander unter C₁₋₆-Alkyl, C₁₋₆-Alkanoyl, C₁₋₆-Alkylsulfonyl, C₁₋₆-Alkoxycarbonyl, Carbamoyl, *N*-(C₁₋₆-Alkyl)carbamoyl, *N*,*N*-(C₁₋₆-Alkyl)₂carbamoyl, Benzyl, Benzyloxycarbonyl, Benzoyl und Phenylsulfonyl ausgewählt sind; worin R⁹, R¹⁶, R¹⁸ und R²⁰ unabhängig voneinander gegebenenfalls an Kohlenstoff durch eine oder mehrere Gruppen R²¹ substituiert sein können;
R¹⁹ und R²¹ unabhängig voneinander unter Halogen, Nitro, Cyano, Hydroxy, Trifluormethoxy, Amino, Carboxy, Carbamoyl, Mercapto, Sulfamoyl, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*-(C₁₋₆-Alkyl) amino, *N*,*N*-(C₁₋₆-Alkyl)₂amino, C₁₋₆-Alkanoylamino, *N*-(C₁₋₆-Alkyl)carbamoyl, *N*,*N*-(C₁₋₆-Alkyl)₂carbamoyl, C₁₋₆-Alkyl-S(O)ₐ, worin a für 0 bis 2 steht, C₁₋₆-Alkoxycarbonyl, *N*-(C₁₋₆-Alkyl)sulfamoyl, *N,N-*(C₁₋₆-Alkyl)₂sulfamoyl, C₁₋₆-Alkylsulfonylamino, Carbocyclyl oder Heterocyclyl ausgewählt sind; worin R¹⁹ und R²¹ unabhängig voneinander gegebenenfalls an Kohlenstoff durch eine oder mehrere Gruppen R²³ substituiert sein können und worin dann, wenn das Heterocyclyl eine -NH-Gruppierung enthält, dieser Stickstoff gegebenenfalls durch eine unter R²⁴ ausgewählte Gruppe substituiert sein kann;
R²³ unter Halogen, Nitro, Cyano, Hydroxy, Trifluormethoxy, Trifluormethyl, Amino, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, *N*-Methyl-*N*-ethylamino, Acetylamino, *N*-Methylcarbamoyl, *N-*Ethylcarbamoyl, *N*,*N*-Dimethylcarbamoyl, *N,N-*Diethylcarbamoyl, *N*-Methyl-*N*-ethylcarbamoyl, Methylthio, Ethylthio, Methylsulfinyl, Ethylsulfinyl, Mesyl, Ethylsulfonyl, Methoxycarbonyl, Ethoxycarbonyl, *N*-Methylsulfamoyl, *N*-Ethylsulfamoyl, *N*,*N*-Dimethylsulfamoyl, *N*,*N*-Diethylsulfamoyl oder *N*-Methyl-*N*-ethylsulfamoyl ausgewählt ist und
R²⁴ unter C₁₋₆-Alkyl, C₁₋₆-Alkanoyl, C₁₋₆-Alkylsulfonyl, C₁₋₆-Alkoxycarbonyl, Carbamoyl, *N*-(C₁₋₆-Alkyl) carbamoyl , *N*,*N*-(C₁₋₆-Alkyl)₂carbamoyl, Benzyl, Benzyloxycarbonyl, Benzoyl und Phenylsulfonyl ausgewählt ist;
worin ein "Carbocyclyl" für einen gesättigten, teilweise gesättigten oder ungesättigten, mono-oder bicyclischen Kohlenstoffring mit 3-12 Atomen steht, worin eine -CH₂-Gruppe gegebenenfalls durch ein -C(O)- ersetzt sein kann;
worin ein "Heterocyclyl" für einen gesättigten, teilweise gesättigten oder ungesättigten, mono-oder bicyclischen Ring mit 4-12 Atomen, von denen mindestens ein Atom unter Stickstoff, Schwefel oder Sauerstoff ausgewählt ist, steht, wobei der Ring über Kohlenstoff oder Stickstoff gebunden sein kann, sofern nicht anders vermerkt, worin eine -CH₂-Gruppe gegebenenfalls durch ein -C(O)-ersetzt sein kann und ein Ringschwefelatom gegebenenfalls zu den S-Oxiden oxidiert sein kann;
worin dann, wenn "R⁶ und R⁷ gemeinsam mit der Bindung, an die sie gebunden sind, einen 5- oder 6-gliedrigen heterocyclischen Ring bilden", der Ring für einen teilweise gesättigten oder ungesättigten, mono- oder bicyclischen Kohlenstoffring mit 5 oder 6 Atomen, von denen mindestens zwei mit dem Pyrimidinring der Formel (I) geteilt werden, wovon mindestens ein Atom unter Stickstoff, Schwefel oder Sauerstoff ausgewählt ist, steht; worin eine -CH₂-Gruppe gegebenenfalls durch ein -C(O)- ersetzt sein kann und ein Ringschwefelatom gegebenenfalls zu den S-Oxiden oxidiert sein kann; und
worin dann, wenn "R⁶ und R⁷ gemeinsam mit der Bindung, an die sie gebunden sind, einen 5- oder 6-gliedrigen carbocyclischen Ring bilden", der Ring für einen teilweise gesättigten oder ungesättigten, mono- oder bicyclischen Ring mit 5 oder 6 Atomen, von denen mindestens zwei mit dem Pyrimidinring der Formel (I) geteilt werden, steht; worin eine -CH₂-Gruppe gegebenenfalls durch ein -C(O)- ersetzt sein kann;
oder ein pharmazeutisch annehmbares Salz davon.

2. Verbindung der Formel (I) oder ein pharmazeutisch annehmbares Salz davon nach Anspruch 1, worin Ring C für Phenyl, Thienyl, Pyridyl oder Thiazolyl steht.

3. Verbindung der Formel (I) oder ein pharmazeutisch annehmbares Salz davon nach Anspruch 1 oder Anspruch 2, worin R¹ unter Wasserstoff, C₁₋₆-Alkyl, C₁₋₆-Alkoxy, *N*,*N*-(C₁₋₆-Alkyl)₂amino, C₁₋₆-Alkyl-S(O)ₐ, worin a für 0 steht, oder Carbocyclyl ausgewählt ist, worin R¹ gegebenenfalls an Kohlenstoff durch eine oder mehrere Gruppen R⁸ substituiert sein kann; worin R⁸ unter Halogen oder Carbocyclyl ausgewählt ist.

4. Verbindung der Formel (I) oder ein pharmazeutisch annehmbares Salz davon nach einem der Ansprüche 1-3, worin R⁴ für Wasserstoff steht.

5. Verbindung der Formel (I) oder ein pharmazeutisch annehmbares Salz davon nach einem der Ansprüche 1-4, worin R⁵ für Wasserstoff oder gegebenenfalls substituiertes C₁₋₆-Alkyl steht; worin die fakultativen Substituenten unter einer oder mehreren Gruppen R¹⁴ ausgewählt sind; und R¹⁴ unter Hydroxy ausgewählt ist.

6. Verbindung der Formel (I) oder ein pharmazeutisch annehmbares Salz davon nach einem der Ansprüche 1-5, worin:
R⁶ und R⁷ unabhängig voneinander unter Wasserstoff, Halogen, Nitro, Cyano, Amino, C₁₋₆-Alkyl, *N*-(C₁₋₆-Alkyl) amino, *N*,*N*-(C₁₋₆-Alkyl)₂amino, *N*-(C₁₋₆-Alkyl)-carbamoyl, C₁₋₆-Alkoxycarbonyl oder Heterocyclyl ausgewählt sind; worin R⁶ und R⁷ unabhängig voneinander gegebenenfalls an Kohlenstoff durch eine oder mehrere Gruppen R¹⁵ substituiert sein können und worin dann, wenn das Heterocyclyl eine -NH-Gruppierung enthält, dieser Stickstoff gegebenenfalls durch eine unter R¹⁶ ausgewählte Gruppe substituiert sein kann;
oder R⁶ und R⁷ gemeinsam mit der Pyrimidinbindung, an die sie gebunden sind, einen 6-gliedrigen carbocyclischen Ring oder einen 5- oder 6-gliedrigen heterocyclischen Ring bilden, worin der Ring an das Pyrimidin der Formel (1) anelliert ist; worin die Doppelbindungen des resultierenden bicyclischen Rings ferner über den gesamten bicyclischen Ring delokalisiert sein können und worin der carbocyclische Ring oder heterocyclische Ring gegebenenfalls an Kohlenstoff durch eine oder mehrere Gruppen R¹⁷ substituiert sein kann und worin dann, wenn der heterocyclische Ring eine -NH-Gruppierung enthält, dieser Stickstoff gegebenenfalls durch eine unter R¹⁸ ausgewählte Gruppe substituiert sein kann;
R¹⁵ unter Halogen, Hydroxy, Amino, C₁₋₆-Alkoxy, *N*,*N-*(C₁₋₆-Alkyl)₂amino, Carbocyclyl oder Heterocyclyl ausgewählt ist; worin R¹⁵ gegebenenfalls an Kohlenstoff durch eine oder mehrere Gruppen R¹⁹ substituiert sein kann und worin dann, wenn das Heterocyclyl eine -NH-Gruppierung enthält, dieser Stickstoff gegebenenfalls durch eine unter R²⁰ ausgewählte Gruppe substituiert sein kann;
R¹⁷ unter Halogen, C₁₋₆-Alkyl oder C₁₋₆-Alkoxy ausgewählt ist; worin R¹⁷ gegebenenfalls an Kohlenstoff durch eine oder mehrere Gruppen R¹⁹ substituiert sein kann;
R¹⁶ unter C₁₋₆-Alkyl ausgewählt ist;
R¹⁸ unter C₁₋₆-Alkanoyl ausgewählt ist;
R¹⁹ unter Halogen, Hydroxy, C₁₋₆-Alkoxy oder Heterocyclyl ausgewählt ist; und worin dann, wenn das Heterocyclyl eine -NH-Gruppierung enthält, dieser Stickstoff gegebenenfalls durch eine unter R²⁴ ausgewählte Gruppe substituiert sein kann;
R²⁰ unter C₁₋₆-Alkyl ausgewählt ist und
R²⁴ unter C₁₋₆-Alkyl ausgewählt ist.

7. Verbindung der Formel (I) nach Anspruch 1, worin:
A für eine direkte Bindung steht;
Ring C für Phenyl, Thienyl, Pyridyl oder Thiazolyl steht;
R¹ unter Wasserstoff, Methyl, Ethyl, Isopropyl, *t-*Butyl, Trifluormethyl, Cyclopropylmethyl, Benzyl, Methoxy, Ethoxy, Propoxy, Isopropoxy, sec-Butoxy, Dimethylamino, Methylthio oder Cyclopropyl ausgewählt ist;
R² unter Methyl, Ethyl, Trifluormethyl, Hydroxymethyl, Carboxymethyl, Aminomethyl, Methoxymethyl, Morpholinomethyl, 1-Hydroxyethyl, 2-Hydroxyethyl, 1-Carboxyethyl, 2-Dimethylaminoethyl, 2-Diethylaminoethyl, Acetamidomethyl, 2-[*N*-Methyl-*N*-(2-methoxyethyl)amino]ethyl, 2-[*N*-Methyl-*N*-(2-hydroxyethyl)amino]ethyl, 2-(*N*-Methylcarbamoyl)-ethyl, 2-[*N*-(2-Hydroxyethyl)carbamoyl]ethyl, 2-(*N*,*N*-Dimethylcarbamoyl)ethyl, 2-Morpholinoethyl, 2-Pyrrolidin-1-ylethyl, 2-(1-Methylpiperazin-4-yl)ethyl oder 1-Methyl-2-hydroxyethyl ausgewählt ist;
R³ unter Fluor ausgewählt ist;
R⁴ für Wasserstoff steht;
R⁵ für Wasserstoff, Methyl oder 2-Hydroxyethyl steht;
R⁶ und R⁷ unabhängig voneinander unter Wasserstoff, Fluor, Chlor, Brom, Nitro, Cyano, Amino, Methyl, Methylamino, Ethylamino, Propylamino, Isopropylamino, Dimethylamino, *N*-Methyl-*N*-propylamino, *N-*Ethylcarbamoyl, Methoxycarbonyl, Ethoxycarbonyl, Butoxycarbonyl, Morpholino, Pyrrolidinyl oder Piperazinyl ausgewählt sind; worin R⁶ und R⁷ unabhängig voneinander gegebenenfalls an Kohlenstoff durch eine oder mehrere Gruppen R¹⁵ substituiert sein können und worin das Piperazinyl gegebenenfalls an Stickstoff durch eine unter R¹⁶ ausgewählte Gruppe substituiert sein kann;
oder R⁶ und R⁷ gemeinsam mit dem Pyrimidin, an das sie gebunden sind, einen bicyclischen Ring bilden, der unter Chinazolinyl, Thieno[3,2-d]pyrimidinyl, Thieno[2,3-d]pyrimidinyl, 1*H-*Pyrazolo[3,4-d]-pyrimidinyl, Thieno[3,4-d]pyrimidinyl, Pyrido[2,3-d]pyrimidinyl, 5,6,7,8-Tetrahydropyrido[4,3-d]-pyrimidinyl, 5,6,7,8-Tetrahydropyrido[2,3-d]-pyrimidinyl oder 5,6,7,8--Tetrahydropyrido[3,4-d]-pyrimidinyl ausgewählt ist; und worin der bicyclische Ring gegebenenfalls an Kohlenstoff durch eine oder mehrere Gruppen R¹⁷ substituiert sein kann und worin 5,6,7,8-Tetrahydropyrido[4,3-d]pyrimidinyl, 5,6,7,8-Tetrahydropyrido[2,3-d]-pyrimidinyl oder 5,6,7,8-Tetrahydropyrido[3,4-d]-pyrimidinyl gegebenenfalls an Stickstoff durch eine unter R¹⁸ ausgewählte Gruppe substituiert sein kann;
R¹⁵ unter Fluor, Hydroxy, Amino, Ethoxy, Dimethylamino, Phenyl, Pyrrolidinyl, Piperazinyl oder Morpholino ausgewählt ist; worin R¹⁵ gegebenenfalls an Kohlenstoff durch eine oder mehrere Gruppen R¹⁹ substituiert sein kann; und worin das Piperazinyl gegebenenfalls an Stickstoff durch eine unter R²⁰ ausgewählte Gruppe substituiert sein kann;
R¹⁶ unter Methyl ausgewählt ist;
R¹⁷ unter Fluor, Chlor, Methyl, Methoxy, Ethoxy oder Propoxy ausgewählt ist; worin R¹⁷ gegebenenfalls an Kohlenstoff durch einen oder meherere Gruppen R¹⁹ substituiert sein kann;
R¹⁸ unter Acetyl ausgewählt ist;
R¹⁹ unter Fluor, Hydroxy, Methoxy, Piperazinyl, Pyrrolidinyl oder Morpholino ausgewählt ist; und worin das Piperazinyl gegebenenfalls an Stickstoff durch eine unter R²⁴ ausgewählte Gruppe substituiert sein kann;
R²⁰ unter Methyl ausgewählt ist;
R²⁴ unter Methyl ausgewählt ist;
n = 1;
oder ein pharmazeutisch annehmbares Salz davon.

8. Verbindung der Formel (I) nach Anspruch 1, ausgewählt unter:
(2*R*)-2-({4-[(5-Cyclopropyl-1*H-*pyrazal-3-yl)amino]-5-fluorpyrimidin-2-yl}amino)-2-(4-fluorphenyl)-ethanol;
5-Brom-N⁴-(3-cyclopropyl-1*H-*pyrazol-5-yl)-N²-[(1*S*)-1-(4-fluorphenyl)ethyl]pyrimidin-2,4-diamin;
(2*R*)-2-({5-Chlor-4-[(3-cyclopropyl-1*H-*pyrazol-5-yl)amina]pyrimidin-2-yl}amino)-2-(4-fluorphenyl)-ethanol;
(2*R*)-2-({5-Chlor-4-[(3-isopropoxy-1*H-*pyrazol-5-yl)amino]pyrimidin-2-yl}amino)-2-(4-fluorphenyl)-ethanol;
(3*S*)-3-({5-Chlor--4-[(5-cyclopropyl-1*H-*pyrazol-3-yl)amino]pyrimidin-2-yl}amino)-3-(4-fluorphenyl)-*N*-methylpropanamid;
2-({5-Chlor-2-{[(1S)-1-(4-fluorphenyl)ethyl]-amino}-6-[(5-isopropoxy-1H-pyrazol-3-yl)amino]-pyrimidin-4-yl}amino)propan-1,3-diol;
2-[(5-Chlor-6-[(3-cyclopropyl-1H-pyrazol-5-yl)-amino]-2-{[(1S)-1-(4-fluorphenyl)ethyl]amino}-pyrimidin-4-yl)amino]propan-1,3-diol;
5-Chlor-N⁴-(5-cyclopropyl-1*H-*pyrazol-3-yl)-N²-[(1*S*)-(4-fluorphenyl)ethyl]-6-(4-methylpiperazin-1-yl)pyrimidin-2,4-diamin;
(2R)-2-({4-[(5-Cyclopropyl-1*H-*pyrazol-3-yl)amino]-7-fluorchinazolin-2-yl}amino)-2-(4-fluorphenyl)-ethanol und
2-[(5-Chlor-6-[(5-cyclopropyl-1H-pyrazol-3-yl)-amino]-2-{[(1R)-1-(4-fluorphenyl)-2-hydroxyethyl]-amino}pyrimidin-4-yl)amino]propan-1,3-diol;
oder ein pharmazeutisch annehmbares Salz davon.

9. Verfahren zur Herstellung einer Verbindung der Formel (I) oder eines pharmazeutisch annehmbaren Salzes davon, bei dem man:
*Verfahren a*) eine Verbindung der Formel (II): 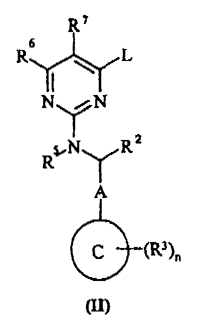 worin L für eine verdängbare Gruppe steht, mit einem Pyrazolamin der Formel (III): 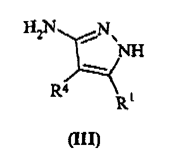 umsetzt;
*Verfahren b*) ein Pyrimidin der Formel (IV): 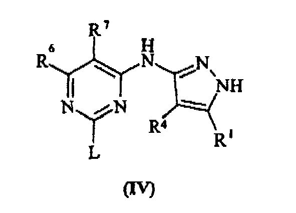 worin L für eine verdrängbare Gruppe steht, mit einer Verbindung der Formel (V): 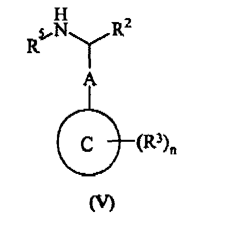 umsetzt;
*Verfahre*n *c*) eine Verbindung der Formel (VI): 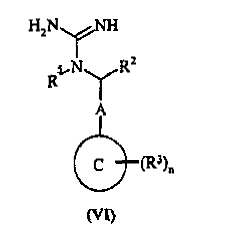 mit einer Verbindung der Formel (VII): 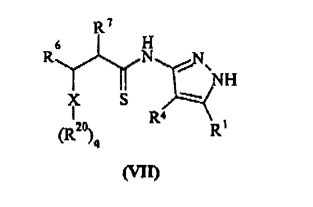 worin X für ein Sauerstoffatom steht und q für 1 steht oder X für ein Stickstoffatom steht und q für 2 steht; und worin R²⁰ jeweils unabhängig voneinander für eine C₁₋₆-Alkylgruppe steht, umsetzt; oder
*Verfahren d*) eine Verbindung der Formel (VIII): 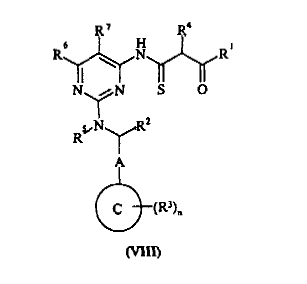 mit Hydrazin umsetzt und danach gegebenenfalls:
i) eine Verbindung der Formel (I) in eine andere Verbindung der Formel (I) umwandelt;
ii) gegebenenfalls vorhandene Schutzgruppen abspaltet;
iii) ein pharmazeutisch annehmbares Salz bildet.

10. Verbindung der Formel (I) oder ein pharmazeutisch annehmbares Salz davon nach einem der Ansprüche 1-8 zur Verwendung als Arzneimittel.

11. Verwendung einer Verbindung der Formel (I) oder eines pharmazeutisch annehmbaren Salzes davon nach einem der Ansprüche 1-8 zur Herstellung eines Arzneimittels zur Inhibierung von Trk-Aktivität.

12. Verwendung einer Verbindung der Formel (I) oder eines pharmazeutisch annehmbaren Salzes davon nach einem der Ansprüche 1-8 zur Herstellung eines Arzneimittels zur Behandlung oder Prophylaxe von Krebs.

13. Verwendung einer Verbindung der Formel (I) oder eines pharmazeutisch annehmbaren Salzes davon nach einem der Ansprüche 1-8 zur Herstellung eines Arzneimittels zur Hervorrufung einer antiproliferativen Wirkung.

14. Pharmazeutische Zusammensetzung, enthaltend eine Verbindung der Formel (I) oder ein pharmazeutisch annehmbares Salz davon nach einem der Ansprüche 1-8 zusammen mit mindestens einem pharmazeutisch annehmbaren Träger, Verdünnungsmittel oder Hilfsstoff.

15. Verwendung nach Anspruch 12, bei der der Krebs unter Speiseröhrenkrebs, Myelom, Leberzellkrebs, Bauchspeicheldrüsenkrebs, Zervixkrebs, Ewing-Sarkom, Neuroblastom, Kaposi-Sarkom, Eierstockkrebs, Brustkrebs, Kolorektalkrebs, Prostatakrebs, Blasenkrebs, Melanom, Lungenkrebs - nichtkleinzelligem Lungenkrebs (NSCLC) und kleinzelligem Lungenkrebs (SCLC), Magenkrebs, Kopf- und Halskrebs, Nierenkrebs, Lymphom, Leukämie, Tumoren des zentralen und peripheren Nervensystems, Melanom, Fibrosarkom und Osteosarkom ausgewählt ist.

## Revendications

1. Composé de formule (I) : 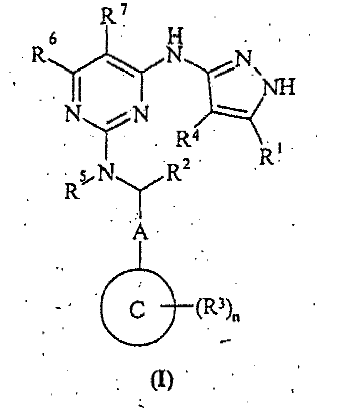 dans laquelle :
**A** est une liaison directe ;
le **cycle C** est carbocyclyle ou hétérocyclyle ;
**R¹** et **R⁴** sont choisis indépendamment parmi hydrogène, halogéno, nitro, cyano, hydroxy, trifluorométhoxy, amino, carboxy, carbamoyle, mercapto, sulfamoyle, C₁₋₆alkyle, C₂₋₆alcényle, C₂₋₆ alcynyle, C₁₋₆alcoxy, C₁₋₆alcanoyle, C₁₋₆alcanoyloxy, *N*-(C₁₋₆alkyl) amino, *N,N*-(C₁₋₆alkyl)₂amino, C₁₋₆ alcanoylamino, *N*-(C₁₋₆alkyl) carbamoyle, *N*,*N*-(C₁₋₆ alkyl)₂carbamoyle, C₁₋₆alkyl-S(O)ₐ, où a est 0 à 2, C₁₋₆alcoxycarbonyle, *N*-(C₁₋₆alkyl) sulfamoyle, *N,N-*(C₁₋₆alkyl)₂sulfamoyle, C₁₋₆alkylsulfonylamino, carbocyclyle ou hétérocyclyle, où R¹ et R⁴ indépendamment l'un de l'autre peuvent être éventuellement substitués sur le carbone par un ou plusieurs R⁸ ; et où si ledit hétérocyclyle contient un motif -NH-, cet azote peut être éventuellement substitué par un groupement choisi parmi R⁹ ;
**R²** est choisi parmi C₁₋₆alkyle ; où R² peut être éventuellement substitué sur le carbone par un ou plusieurs R¹⁰ ;
**R¹⁰** est choisi parmi halogéno, hydroxy, carboxy, amino, C₁₋₆alcoxy, *N*,*N*-(C₁₋₆alkyl)₂amino, C₁₋₆alcanoylamino, *N*-(C₁₋₆alkyl) carbamoyle, *N,N-*(C₁₋₆alkyl)₂carbamoyle ou hétérocyclyle ; où R¹⁰ peut être éventuellement substitué sur le carbone par un ou plusieurs R^{19a} ; et où si ledit hétérocyclyle contient un motif -NH-, cet azote peut être éventuellement substitué par un groupement choisi parmi R^{20a} ;
**R^{19a}** est choisi parmi hydroxy ou C₁₋₆alcoxy ;
**R^{20a}** est choisi parmi C₁₋₆alkyle ;
**R³** est fluoro ;
**R⁵** est hydrogène ou C₁₋₆alkyle éventuellement substitué ; où lesdits substituants éventuels sont choisis parmi un ou plusieurs R¹⁴ ;
**R⁶** et **R⁷** sont choisis indépendamment parmi hydrogène, halogéno, nitro, cyano, hydroxy, trifluorométhoxy, amino, carboxy, carbamoyle, mercapto, sulfamoyle, C₁₋₆alkyle, C₂₋₆alcényle, C₂₋₆alcynyle, C₁₋₆alcoxy, C₁₋₆alcanoyle, C₁₋₆alcanoyloxy, *N*-(C₁₋₆alkyl) amino, *N,N-*(C₁₋₆alkyl)₂amino, C₁₋₆alcanoylamino, *N-*(C₁₋₆alkyl) carbamoyle, *N*,*N*- (C₁₋₆alkyl)₂carbamoyle, C₁₋₆alkyl-S(O)ₐ, où a est 0 à 2, C₁₋₆alcoxycarbonyle, *N*-(C₁₋₆alkyl) sulfamoyle, *N*,*N*-(C₁₋₆alkyl)₂sulfamoyle, C₁₋₆alkylsulfonylamino, carbocyclyle ou hétérocyclyle ; où R⁶ et R⁷ indépendamment l'un de l'autre peuvent être éventuellement substitués sur carbone par un ou plusieurs R¹⁵ ; et où si ledit hétérocyclyle contient un motif -NH-, cet azote peut être éventuellement substitué par un groupement choisi parmi R¹⁶ ;
ou **R⁶** et **R⁷** ensemble avec la liaison pyrimidine à laquelle ils sont liés forment un cycle carbocyclique à 5 ou 6 chaînons ou un cycle hétérocyclique à 5 ou 6 chaînons, où ledit cycle est condensé sur la pyrimidine de formule (I) ; où les doubles liaisons du cycle bicyclique résultant peuvent être en outre délocalisées sur l'ensemble du cycle bicyclique ; et où ledit cycle carbocyclique ou cycle hétérocyclique peut être éventuellement substitué sur le carbone par un ou plusieurs R¹⁷ ; et où si ledit cycle hétérocyclique contient un motif -NH-, cet azote peut être éventuellement substitué par un groupement choisi parmi R¹⁸ ;
**n =** 1 ;
**R⁸, R¹⁴, R¹⁵** et **R¹⁷** sont choisis indépendamment parmi halogéno, nitro, cyano, hydroxy, trifluorométhoxy, amino, carboxy, carbamoyle, mercapto, sulfamoyle, C₁₋₆alkyle, C₂₋₆alcényle, C₂₋₆alcynyle, C₁₋₆alcoxy, C₁₋₆alcanoyle, C₁₋₆alcanoyloxy, *N*-(C₁₋₆alkyl) amino, *N*,*N*-(C₁₋₆alkyl) ₂amino, C₁₋₆alcanoylamino, *N-*(C₁₋₆alkyl) carbamoyle, *N*,*N*- (C₁₋₆alkyl)₂carbamoyle, C₁₋₆alkyl-S(O)ₐ, où a est 0 à 2, C₁₋₆alcoxycarbonyle, *N*-(C₁₋₆alkyl)sulfamoyle, *N*,*N*-(C₁₋₆alkyl) ₂sulfamoyle, C₁₋₆alkylsulfonylamino, carbocyclyle ou hétérocyclyle ; où R⁸, R¹⁴, R¹⁵ et R¹⁷ indépendamment les uns des autres peuvent être éventuellement substitués sur le carbone par un ou plusieurs R¹⁹ ; et où si ledit hétérocyclyle contient un motif -NH-, cet azote peut être éventuellement substitué par un groupement choisi parmi R²⁰ ;
**R⁹, R¹⁶, R¹⁸** et **R²⁰** sont choisis indépendamment parmi C₁₋₆alkyle, C₁₋₆alcanoyle, C₁₋₆alkylsulfonyle, C₁₋₆alcoxycarbonyle, carbamoyle, *N-*(C₁₋₆alkyl ) carbamoyle, *N*,*N*-(C₁₋₆alkyl)₂carbamoyle, benzyle, benzyloxycarbonyle, benzoyle et phénylsulfonyle ; où R⁹, R¹⁶, R¹⁸ et R²⁰ indépendamment les uns des autres peuvent être éventuellement substitués sur le carbone par un ou plusieurs R²¹ ;
**R¹⁹** et **R²¹** sont choisis indépendamment parmi halogéno, nitro, cyano, hydroxy, trifluorométhoxy, amino, carboxy, carbamoyle, mercapto, sulfamoyle, C₁₋₆alkyle, C₂₋₆alcényle, c₂₋₆alcynyle, C₁₋₆alcoxy, C₁₋₆alcanoyle, C₁₋₆alcanoyloxy, *N*-(C₁₋₆alkyl) amino, *N,N-*(C₁₋₆alkyl)₂amino, C₁-₆alcanoylamino, *N-*(C₁-₆alkyl) carbamoyle, *N*,*N*-(C₁-₆alkyl)₂carbamoyle, C₁₋₆alkyl-S(O) a, où a est 0 à 2, C₁₋₆alcoxycarbonyle, *N*-(C₁₋₆alkyl) sulfamoyle, *N,N-* (C₁₋₆alkyl)₂ sulfamoyle, C₁₋₆alkylsulfonylamino, carbocyclyle ou hétérocyclyle ; où R¹⁹ et R²¹ indépendamment l'un de l'autre peuvent être éventuellement substitués sur le carbone par un ou plusieurs R²³ ; et où si ledit hétérocyclyle contient un motif -NH-, cet azote peut être éventuellement substitué par un groupement choisi parmi R²⁴ _{;}
**R²³** est choisi parmi halogéno, nitro, cyano, hydroxy, trifluorométhoxy, trifluorométhyle, amino, carboxy, carbamoyle, mercapto, sulfamoyle, méthyle, éthyle, méthoxy, éthoxy, acétyle, acétoxy, méthylamino, éthylamino, diméthylamino, diéthylamino, *N*-méthyl-*N*-éthylamino, acétylamino, *N*-méthylcarbamoyle, *N*-éthylcarbamoyle, *N,N-*diméthylcarbamoyle, *N*,*N*-diéthylcarbamoyle, *N-*méthyl-*N*-éthylcarbamoyle, méthylthio, éthylthio, méthylsulfinyle, éthylsulfinyle, mésyle, éthylsulfonyle, méthoxycarbonyle, éthoxycarbonyle, *N*-méthylsulfamoyle, *N*-éthylsulfamoyle, *N,N-*diméthylsulfamoyle, *N*,*N*-diéthylsulfamoyle ou *N-*méthyl-*N*-éthylsulfamoyle et
**R²⁴** est choisi parmi C₁₋₆alkyle, C₁₋₆alcanoyle, C₁₋₆alkylsulfonyle, C₁₋₆alcoxycarbonyle, carbamoyle, *N*-(C₁₋₆alkyl) carbamoyle, *N*,*N*-(C₁₋₆alkyl)₂ carbamoyle, benzyle, benzyloxycarbonyle, benzoyle et phénylsulfonyle ;
où un "carbocyclyle" est un cycle carboné mono- ou bicyclique saturé, partiellement saturé ou insaturé qui contient de 3 à 12 atomes ; où un groupement -CH₂- peut éventuellement être remplacé par un -C(O)- ;
où un "hétérocyclyle" est un cycle mono- ou bicyclique saturé, partiellement saturé ou insaturé contenant de 4 à 12 atomes dont au moins un atome est choisi parmi azote, soufre ou oxygène, qui peut, sauf précision contraire, être lié au carbone ou à l'azote, où un groupement -CH₂- peut éventuellement être remplacé par un -C(O)-, et un atome de soufre du cycle peut être éventuellement oxydé pour former les S-oxydes ;
où lorsque "R⁶ et R⁷ ensemble avec la liaison à laquelle ils sont liés forment un cycle hétérocyclique à 5 ou 6 chaînons", ledit cycle est un cycle carboné mono- ou bicyclique partiellement saturé ou insaturé qui contient 5 ou 6 atomes dont deux atomes sont partagés avec le cycle pyrimidine de formule (I) ; dont au moins un atome est choisi parmi azote, soufre ou oxygène ; où un groupement -CH₂- peut éventuellement être remplacé par un -C(O)-, et un atome de soufre du cycle peut être éventuellement oxydé pour former les S-oxydes ; et
où lorsque " R⁶ et R⁷ ensemble avec la liaison à laquelle ils sont liés forment un cycle carbocyclique à 5 ou 6 chaînons", ledit cycle est un cycle carboné mono- ou bicyclique partiellement saturé ou insaturé qui contient 5 ou 6 atomes dont deux atomes sont partagés avec le cycle pyrimidine de formule (I) ; où un groupement -CH₂- peut éventuellement être remplacé par un -C(O)- ; ou un sel pharmaceutiquement acceptable de celui-ci.

2. Composé de formule (**I**), ou un sel pharmaceutiquement acceptable de celui-ci, selon la revendication 1, dans laquelle le cycle C est phényle, thiényle, pyridyle, thiazolyle.

3. Composé de formule **(I),** ou un sel pharmaceutiquement acceptable de celui-ci, selon l'une ou l'autre de la revendication 1 et de la revendication 2, dans laquelle R¹ est choisi parmi hydrogène, C₁₋₆alkyle, C₁₋₆alcoxy, *N,N-* C₁₋₆ alkyl)₂amino, c₁₋₆alkyl-S(O)ₐ, où a est 0, ou carbocyclyle ; où R¹ peut être éventuellement substitué sur le carbone par un ou plusieurs R⁸ ; où R⁸ est choisi parmi halogéno ou carbocyclyle.

4. Composé de formule **(I),** ou un sel pharmaceutiquement acceptable de celui-ci, selon l'une quelconque des revendications 1 à 3, dans laquelle R⁴ est hydrogène.

5. Composé de formule **(I),** ou un sel pharmaceutiquement acceptable de celui-ci, selon l'une quelconque des revendications 1 à 4, dans laquelle R⁵ est hydrogène ou C₁₋₆alkyle éventuellement substitué ; où lesdits substituants éventuels sont choisis parmi un ou plusieurs R¹⁴ ; et R¹⁴ est choisi parmi hydroxy.

6. Composé de formule **(I),** ou un sel pharmaceutiquement acceptable de celui-ci, selon l'une quelconque des revendications 1 à 5, dans laquelle :
R⁶ et R⁷ sont indépendamment choisis parmi hydrogène, halogéno, nitro, cyano, amino, C₁₋₆alkyle, *N*-(C₁₋₆alkyl)amino, *N*,*N*-(C₁₋₆alkyl)₂ amino, *N*-(C₁₋₆alkyl) carbamoyle, C₁₋₆alcoxycarbonyle ou hétérocyclyle; où R⁶ et R⁷ indépendamment l'un de l'autre peuvent être éventuellement substitués sur le carbone par un ou plusieurs R¹⁵ ; et où si ledit hétérocyclyle contient un motif -NH- cet azote peut être éventuellement substitué par un groupement choisi parmi R¹⁶ ;
ou R⁶ et R⁷ ensemble avec la liaison pyrimidine à laquelle ils sont liés forment un cycle carbocyclique à 6 chaînons ou un cycle hétérocyclique à 5 ou 6 chaînons où ledit cycle est condensé sur la pyrimidine de formule (I) ; où les doubles liaisons du cycle bicyclique résultant peuvent en outre être délocalisées sur l'ensemble du cycle bicyclique ; et où ledit cycle carbocyclique ou cycle hétérocyclique peut être éventuellement substitué sur le carbone par un ou plusieurs R¹⁷ ; et où si ledit cycle hétérocyclique contient un motif -NH- cet azote peut être éventuellement substitué par un groupement choisi parmi R¹⁸ ;
R¹⁵ est choisi parmi halogéno, hydroxy, amino, C₁₋₆alcoxy, *N*,*N*-(C₁₋₆alkyl)₂ amino, carbocyclyle ou hétérocyclyle ; où R¹⁵ peut être éventuellement substitué sur le carbone par un ou plusieurs R¹⁹ ; et où si ledit hétérocyclyle contient un motif -NH- cet azote peut être éventuellement substitué par un groupement choisi parmi R²⁰ ;
R₁₇ est choisi parmi halogéno, C₁₋₆alkyle ou C₁₋₆alcoxy ; où R¹⁷ peut être éventuellement substitué sur le carbone par un ou plusieurs R¹⁹ ;
R¹⁶ est choisi parmi C₁₋₆alkyle ;
R¹⁸ est choisi parmi C₁₋₆alcanoyle ;
R¹⁹ est choisi parmi halogéno, hydroxy, C₁₋₆alcoxy ou hétérocyclyle ; et où si ledit hétérocyclyle contient un motif -NH- cet azote peut être éventuellement substitué par un groupement choisi parmi R²⁴ ;
R²⁰ est choisi parmi C₁₋₆alkyle ; et
R²⁴ est choisi parmi C₁₋₆alkyle.

7. Composé de formule (I), selon la revendication 1, dans laquelle :
A est une liaison directe ;
le cycle C est phényle, thiényle, pyridyle, thiazolyle ;
R¹ est choisi parmi hydrogène, méthyle, éthyle, isopropyle, *t*-butyle, trifluorométhyle, cyclopropylméthyle, benzyle, méthoxy, éthoxy, propoxy, isopropoxy, sec-butoxy, diméthylamino, méthylthio ou cyclopropyle ;
R² est choisi parmi méthyle, éthyle, trifluorométhyle, hydroxyméthyle, carboxyméthyle, aminométhyle, méthoxyméthyle, morpholinométhyle, 1-hydroxyéthyle, 2-hydroxyéthyle, 1-carboxyéthyle, 2-diméthylaminoéthyle, 2-diéthylaminoéthyle, acétamidométhyle, 2-[*N*-méthyl-*N*-(2-méthoxyéthyl)amino]éthyle, 2-[*N*-méthyl-*N*-(2-hydroxyéthyl)amino]éthyle, 2-(*N-*méthylcarbamoyl)éthyle, 2-[*N*-(2-hydroxyéthyl)carbamoyl]éthyle, 2*-(N,N-*diméthylcarbamoyl)éthyle, 2-morpholinoéthyle, 2-pyrrolidin-1-yléthyle, 2-(1-méthylpipérazin-4-yl)éthyle ou 1-méthyl-2-hydroxyéthyle ;
R³ est choisi parmi fluoro ;
R⁴ est hydrogène ;
R⁵ est hydrogène, méthyle ou 2-hydroxyéthyle ;
R⁶ et R⁷ sont indépendamment choisis parmi hydrogène, fluoro, chloro, bromo, nitro, cyano, amino, méthyle, méthylamino, éthylamino, propylamino, isopropylamino, diméthylamino, *N-*méthyl-*N*-propylamino, *N*-éthylcarbamoyle, méthoxycarbonyle, éthoxycarbonyle, butoxycarbonyle, morpholino, pyrrolidinyle ou pipérazinyle ; où R⁶ et R⁷ indépendamment l'un de l'autre peuvent être éventuellement substitués sur le carbone par un ou plusieurs R¹⁵ ; et où ledit pipérazinyle peut être éventuellement substitué sur l'azote par un groupement choisi parmi R¹⁶ ;
ou R⁶ et R⁷ ensemble avec la pyrimidine à laquelle ils sont liés forment un cycle bicyclique choisi parmi quinazolinyle, thiéno[3,2-d]pyrimidinyle, thiéno[2,3-d]pyrimidinyle, 1*H*-pyrazolo[3,4-d]pyrimidinyle, thiéno [3,4-d] pyrimidinyle, pyrido[2,3-d]pyrimidinyle, 5,6,7,8-tétrahydropyrido[4,3-d]pyrimidinyle, 5,6,7,8-tétrahydropyrido[2,3-d]pyrimidinyle ou 5,6,7,8-tétrahydropyrido[3,4-d]pyrimidinyle ; et où ledit cycle bicyclique peut être éventuellement substitué sur le carbone par un ou plusieurs R¹⁷ ; et où ledit 5,6,7,8-tétrahydropyrido[4,3-d]pyrimidinyle, 5,6,7,8-tétrahydropyrido[2,3-d]pyrimidinyle ou 5,6,7,8-tétrahydropyrido[3,4-d]pyrimidinyle peut être éventuellement substitué sur l'azote par un groupement choisi parmi R¹⁸ _{;}
R¹⁵ est choisi parmi fluoro, hydroxy, amino, éthoxy, diméthylamino, phényle, pyrrolidinyle, pipérazinyle ou morpholino ; où R¹⁵ peut être éventuellement substitué sur le carbone par un ou plusieurs R¹⁹ ; et où ledit pipérazinyle peut être éventuellement substitué sur l'azote par un groupement choisi parmi R²⁰ ;
R¹⁶ est choisi parmi méthyle ;
R¹⁷ est choisi parmi fluoro, chloro, méthyle, méthoxy, éthoxy ou propoxy ; où R¹⁷ peut être éventuellement substitué sur le carbone par un ou plusieurs R¹⁹ ;
R¹⁸ est choisi parmi acétyle ;
R¹⁹ est choisi parmi fluoro, hydroxy, méthoxy, pipérazinyle, pyrrolidinyle ou morpholino ; et où ledit pipérazinyle peut être éventuellement substitué sur l'azote par un groupement choisi parmi R²⁴ ;
R²⁰ est choisi parmi méthyle ;
R²⁴ est choisi parmi méthyle ;
n = 1 ;
ou un sel pharmaceutiquement acceptable de celui-ci .

8. Composé de formule (I) selon la revendication 1, choisi parmi :
le (2*R*)-2-({4--[(5-cyclapropyl-1*H*-pyrazol-3-yl)amino]-5-fluoropyrimidin-2-yl}amino)-2-(4-fluorophényl) éthanol ;
la 5-bromo-N⁴- (3-cyclopropyl-1*H-*pyrazol-5-yl) -N²-[(1*S*)-1-(4-fluorophényl) éthyl]pyrimidine-2,4-diamine ;
le (2*R*)-2-({5-chloro-4-[(3-cyclopropyl-1*H*-pyrazol-5-yl)amino]pyrimidin-2-yl}amino)-2-(4-fluorophényl)éthanol;
le (2*R*)-2-({5-chloro-4-[(3-isopropoxy-1*H*-pyrazol-5-yl)amino]pyrimidin-2-yl}amino)-2-(4-fluorophényl)éthanol ;
le (3*S*)-3-({5-chloro-4-[(5-cyclopropyl-1*H*-pyrazol-3-yl)amino]pyrimidin-2-yl}amino)-3-(4-fluorophényl)-*N*-méthylpropanamide ;
le 2-({5-chloro-2-{[(1S)-1-(4-fluorophényl)éthyl]-amino}-6-[(5-isopropoxy-1H-pyrazol-3-yl)amino]-pyrimidin-4-yl}amino)propane-1,3-diol ;
le 2-[(5-chloro-6-[(3-cyclopropyl-1H-pyrazol-5-yl)amino]-2-{[(1)-1-(4-fluorophényl)éthyl]amino}-pyrimidin-4-yl)amino]propane-1,3-diol ;
la 5-chloro-N⁴- (5-cyclopropyl-1*H*-pyrazol-3-yl)-N²-[(1*S*)-1-(4-fluorophényl)éthyl]-6-(4-méthylpipérazin-1-yl)pyrimidine-2,4-diamine ;
le (2R)-2-({4-[(5-cycloprapyl-1*H*-pyrazol-3-yl)amino]-7-fluoroquinazolin-2-yl}amino)-2-(4-fluorophényl)éthanol ; et
le 2-[(5-chloro-6-[(5-cyclapropyl-1H-pyrazol-3-yl)amino]-2-{[(1R)-1-(4-fluorophényl)-2-hydroxyéthyl]amino}pyrimidin-4-yl)amino]propane-1,3-dol ;
ou un sel pharmaceutiquement acceptable de celui-Ci.

9. Procédé de préparation d'un composé de formule (I)
ou d'un sel pharmaceutiquement acceptable de celui-ci, selon l'une quelconque des revendications 1 à 8, lequel procédé comprend :
*Procédé a)* la réaction d'une pyrimidine de formule (**II**) : 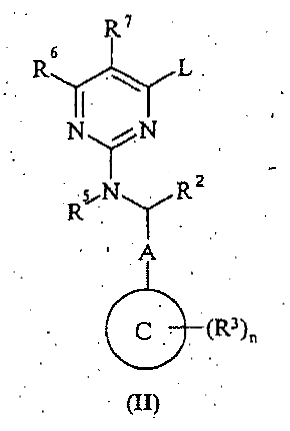 dans laquelle L est un groupement déplaçable ; avec une pyrazole-amine de formule (**III)** : 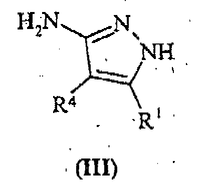 ou
*Procédé b)* la réaction d'une pyrimidine de formule (**IV)** : 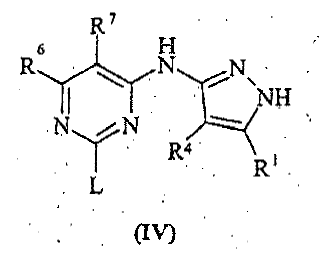 dans laquelle L est un groupement déplaçable ; avec un composé de formule **(V)** : 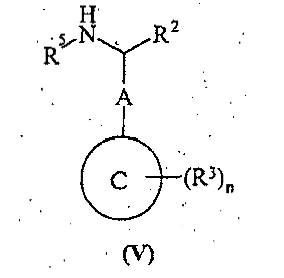
*Procédé c)* la réaction d'un composé de formule (VI) : 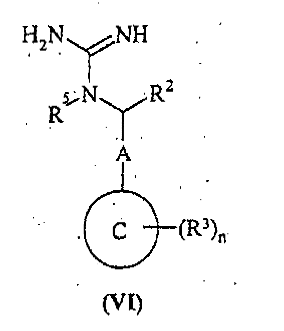 avec un composé de formule **(VII)** : 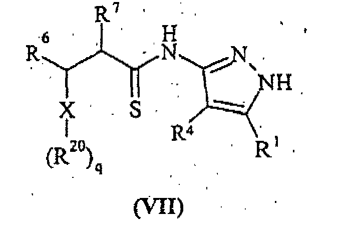 dans laquelle X est un atome d'oxygène et q est 1 ; ou X est un atome d' azote et q est 2 ; et où chaque R²⁰ représente indépendamment un groupement C₁₋₆alkyle ; ou
Procédé d) la réaction d'un composé de formule **(VIII) :** 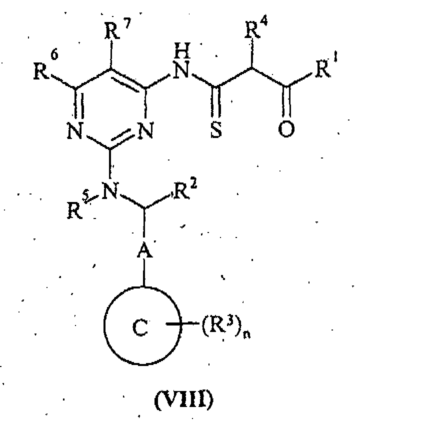 avec de l'hydrazine ;
et ensuite le cas échéant :
i) la transformation d'un composé de formule **(I)** en un autre composé de formule **(I) ;**
ii) l'élimination de tous groupements protecteurs ;
iii) la formation d'un sel pharmaceutiquement acceptable.

10. Composé de formule **(I),** ou un sel pharmaceutiquement acceptable de celui-ci, selon l'une quelconque des revendications 1 à 8, destiné à être utilisé comme médicament.

11. Utilisation d'un composé de formule **(I),** ou d'un sel pharmaceutiquement acceptable de celui-ci, selon l'une quelconque des revendications 1 à 8, dans la fabrication d'un médicament destiné à l'inhibition de l'activité Trk.

12. Utilisation d'un composé de formule **(I),** ou d'un sel pharmaceutiquement acceptable de celui-ci, selon l'une quelconque des revendications 1 à 8, dans la fabrication d'un médicament destiné au traitement ou à la prophylaxie du cancer.

13. Utilisation d'un composé de formule **(I),** ou d'un sel pharmaceutiquement acceptable de celui-ci, selon l'une quelconque des revendications 1 à 8, dans la fabrication d'un médicament destiné à la production d'un effet anti-prolifératif.

14. Composition pharmaceutique comprenant un composé de formule (I), ou un sel pharmaceutiquement acceptable de celui-ci, selon l'une quelconque des revendications 1 à 8, conjointement avec au moins un support, diluant ou excipient pharmaceutiquement acceptable.

15. Utilisation selon la revendication 12, **caractérisée en ce que** ledit cancer est choisi parmi le cancer de l'oesophage, le myélome, le cancer hépatocellulaire, pancréatique ou du col de l'utérus, la tumeur d'Ewing, le neuroblastome, le sarcome de Kaposi, le cancer des ovaires, le cancer du sein, le cancer colorectal, le cancer de la prostate, le cancer de la vessie, le mélanome, le cancer du poumon - le cancer du poumon non à petites cellules (NSCLC), le cancer du poumon à petites cellules (SCLC), le cancer gastrique, le cancer de la tête et du cou, le cancer du rein, le lymphome, la leucémie, les tumeurs du système nerveux central et périphérique, le mélanome, le fibrosarcome et l'ostéosarcome.
